(19) Europäisches Patentamt
European Patent Office
Office européen des brevets

(11) **EP 3 782 465 B1**

(12) **EUROPEAN PATENT SPECIFICATION**

(45) Date of publication and mention
of the grant of the patent:
**30.08.2023 Bulletin 2023/35**

(21) Application number: **19193235.9**

(22) Date of filing: **23.08.2019**

(51) International Patent Classification (IPC):
**A01K 67/027** (2006.01) **C07K 14/46** (2006.01)

(52) Cooperative Patent Classification (CPC):
**A01K 67/0275; C07K 14/461;** A01K 2217/052;
A01K 2217/056; A01K 2217/15; A01K 2217/203;
A01K 2217/206; A01K 2227/40; A01K 2267/0375

(54) **TRANSGENIC ZEBRAFISH MODEL FOR STROKE**

TRANSGENES ZEBRAFISCHMODELL FÜR SCHLAGANFALL

MODÈLE DE POISSON ZÈBRE TRANSGÉNIQUE POUR ACCIDENT VASCULAIRE CÉRÉBRAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**24.02.2021 Bulletin 2021/08**

(73) Proprietor: **Universität Potsdam**
**14469 Potsdam (DE)**

(72) Inventors:
• **SEYFRIED, Salim**
**13127 Berlin (DE)**
• **RÖDEL, Claudia Jasmin**
**14469 Potsdam (DE)**

(74) Representative: **Hertin und Partner**
**Rechts- und Patentanwälte PartG mbB**
**Kurfürstendamm 54/55**
**10707 Berlin (DE)**

(56) References cited:
**CN-A- 106 226 531**

• **STEFAN DONAT ET AL: "Heg1 and Ccm1/2 proteins control endocardial mechanosensitivity during zebrafish valvulogenesis", ELIFE, vol. 7, 24 January 2018 (2018-01-24), XP055658204, DOI: 10.7554/eLife.28939**

• **AUBREY C. CHAN ET AL: "Recent insights into cerebral cavernous malformations: animal models of CCM and the human phenotype : CCM animal models", FEBS JOURNAL, vol. 277, no. 5, March 2010 (2010-03), pages 1076-1083, XP55658276, GB ISSN: 1742-464X, DOI: 10.1111/j.1742-4658.2009.07536.x**

• **D. A. MCDONALD ET AL: "A novel mouse model of cerebral cavernous malformations based on the two-hit mutation hypothesis recapitulates the human disease", HUMAN MOLECULAR GENETICS, vol. 20, no. 2, 15 January 2011 (2011-01-15), pages 211-222, XP055658093, ISSN: 0964-6906, DOI: 10.1093/hmg/ddq433**

• **G. BOULDAY ET AL: "Tissue-specific conditional CCM2 knockout mice establish the essential role of endothelial CCM2 in angiogenesis: implications for human cerebral cavernous malformations", DISEASE MODELS & MECHANISMS, vol. 2, no. 3-4, March 2009 (2009-03), pages 168-177, XP55466448, GB ISSN: 1754-8403, DOI: 10.1242/dmm.001263**

Remarks:
The complete document including Reference Table(s) and the Sequence Listing(s) can be downloaded from the EPO website

**Description**

The invention relates to a transgenic zebrafish lacking at least one endogenous functional protein required for a functional CCM complex, comprising an exogenous nucleic acid encoding a functional version of said at least one protein under the transcriptional control of an endocardium-specific genetic system, wherein said at least one protein required for a functional CCM complex is selected from the group comprising CCM1 (KRIT1), CCM2, CCM3, and Heg1. In the context of the invention the transgenic zebrafish comprises a cardiovascular system with a heart that produces blood flow, and develops vascular lesions in cerebral blood vessels, in particular in lowly perfused cerebral blood vessels. The invention also relates to a method for measuring a pharmacological effect of a drug, comprising contacting a transgenic fish according to any one of the claims with a drug, and measuring a pharmacological effect of the drug, wherein the effect of the drug may be an effect on the formation of vascular lesions, the development of cerebral cavernous malformation and/or the development of stroke. Furthermore, the invention comprises various uses of the transgenic fish of the invention.

**BACKGROUND**

Stroke is a major unresolved medical problem. Therefore, assays that can contribute to the elucidation of the mechanisms and enable the testing of active substances, for example, are urgently required. Since strokes are usually caused by multiple factors, the production of such animal models is very difficult.

Cerebral cavernous malformation (CCM) is a vascular disease with a prevalence of 0.4 to 0.5 percent of the total population. The familiar form of the disease is caused by a two-hit mechanism (Knudson, 1971) in which heterozygous carriers with a heterozygous germline mutation in one of the genes CCM1/KRIT1 (Duboysky et al., 1995), CCM2/MGC4607 or CCM3/PDCD10 (Craig et al., 1998) acquire another somatic mutation in the other allele (Akers et al., 2009). The CCM defects occur mainly within the cerebral capillary vasculature and are characterized by a mulberry-like appearance. Clinical symptoms range from neuronal defects such as seizures and headaches to hemorrhagic strokes (Spiegler et al., 2018). Symptomatic cavernomas can be surgically resected, but this is not always possible due to the location and accessibility of the lesion. Other therapeutic measures are not available.

Hemodynamic forces play an important role in the development of the entire circulatory system. The biomechanical effect of the blood flow plays a central role here. Blood flow gradually decreases with distance to the heart. Thus, endothelial cells of different vascular beds are exposed to different graduations of biomechanical stress induced by blood flow. Endothelial cells therefore respond to different biomechanical demands and activate signal transduction pathways which have effects on cell orientation, migration and proliferation (Baeyens et al., 2016a). Pathological changes in biomechanical signal transduction pathways and abnormal patterns of blood flow have already been associated with etiologies of various blood vessel diseases (Baeyens et al., 2016b), including cerebral cavernous malformations (CCM) (Macek Jilkova et al., 2014; Mleynek et al., 2014; Renz et al., 2015).

Several indications suggest a central role of blood flow in the etiology of CCMs: i) CCM lesions mostly occur in venous vessels that are predominantly weakly supplied with blood (Chohan et al., 2018). ii) The loss of CCM protein function causes upregulation of blood flow sensitive factors KLF2 and KLF4, which act as mediators of biomechanical signals in endothelial cells (Renz et al., 2015;). iii) Switching off the Klf2a/b expression causes a rescue of cardiovascular defects in zebrafish CCM mutants (Renz et al., 2015). iv.) In conditional endothelial CCM/ or CCM2 knock-out mice, the formation of cerebrovascular lesions is suppressed by KLF2/4 knock-outs (Zhou et al., 2015; Cuttano et al., 2016; Zhou et al., 2016). v.) The blood pressure regulating substance propanolol reduces CCM lesion exposure in patients (Reinhard et al., 2016; Zabramski et al., 2016) and suppresses the *ccm2* mutant embryonic phenotype in zebrafish (Offen et al., 2018).

In the zebrafish animal model of cerebral cavernomas, a complete loss of CCM protein function causes developmental defects in the entire cardiovascular system (Mably et al., 2006; Kleaveland et al., 2009; Renz et al., 2015). These defects lead, among other things, to the loss of cardiac function and thus the blood flow is missing as an essential stimulus for normal development of the cardiovascular system. According to this, it has not been possible to reproduce blood flow-dependent hemorrhagic conditions in zebrafish as they occur in CCM patients. However, this is an essential prerequisite for a comprehensive investigation of CCM protein-deficient endothelial cells and their defects and the causes of CCM-related cerebral hemorrhages.

In the CCM animal model of the mouse, conditionally induced knock-outs can be used to generate post-natal lesions in the cerebral vasculature. However, due to the high workload and costs involved, the mouse is not a suitable model for systematically screening for new active substances for the cure or treatment of CCM. In comparison the Zebrafish is clearly more advantageous. In zebrafish, large quantities of experimental embryos can be created and examined in parallel at relatively low cost.

Accordingly, there remains a strong need in the art for the provision of a suitable animal model that can be used for investigating the pathological mechanisms leading to stroke and related diseases, such as the formation of vascular lesions and the development of cerebral cavernous malformation, as well as for testing or measuring the effect of drug candidates for the treatment of stroke or related diseases.

## SUMMARY OF THE INVENTION

In light of the prior art the technical problem underlying the present invention is the provision of an improved animal model for studying stroke and related diseases including vascular malformations. This problem is solved by the features of the independent claims. Preferred embodiments of the present invention are provided by the dependent claims.
The invention is set out in the appended set of claims.

*Embodiments relating to a transgenic fish of the invention*

The invention therefore relates to a transgenic fish lacking at least one endogenous functional protein required for a functional CCM complex, comprising an exogenous nucleic acid encoding a functional version of said at least one protein under the transcriptional control of an endocardium-specific genetic system.
In the context of the invention, the transgenic fish is a zebrafish.
The transgenic fish of the invention can be used as a model system for studying stroke and other diseases involving vascular lesions. For example, the transgenic fish can be used as a tool in the search for drugs or active substances to be used in stroke patients, for example for treatment of prevention of stroke. The invention represents a genetic animal model, for example in the zebrafish embryos, in which pathological changes and hemorrhagic bleeding occurs in the vasculature and serves as a model for stroke and other diseases associated with vascular lesions, such as CCM. Due to its small size of only a few millimeters and its extrauterine development, the zebrafish embryo is excellently suited for pharmacological drug discovery. This animal model can be used for the targeted investigation and/or testing of drugs that have an effect on the formation of vascular lesions and potentially suppress hemorrhagic stroke.
The invention is based on the entirely surprising finding that the expression of a functional protein required for the formation of a functional CCM complex, such as a CCM protein (such as CCM1, CCM2, CCM3 or Heg1) in the endocardium of a mutant that lacks the respective endogenous functional protein required for a functional CCM complex, restores heart development and leads to establishment of apparently normal blood flow. However, in such transgenic fish and embryos bleeding in the cerebral blood vessels, which are deficient in CCM protein, can be detected. The phenotype occurring in the transgenic fish of the present invention with cerebral hemorrhage resembles to the phenotype of hemorrhagic stroke and other disease associated with vascular lesions, in particular cerebral vascular lesions.
In the transgenic fish described herein, pharmacological or genetic investigations on the development of strokes as well as possibilities for the prevention and treatment of hemorrhagic stroke can be carried out.
In addition, the transgenic fish described herein, can be used to study the formation of vascular malformations and to study the interactions between endothelial cells with neighboring cells including cells of the immune system, pericytes, and vascular smooth muscle cells. These interactions can also be subjected to pharmacological or genetic investigations to elucidate potential roles of cell-cell communication in the development of strokes as well as to assess possibilities for the prevention and treatment of hemorrhagic stroke or vascular malformations.
In the context of the invention, the at least one protein required for a functional CCM complex is selected from the group comprising CCM1 (KRIT1), CCM2, CCM3, and Heg1.
The term "protein required for a functional CCM complex" relates to any protein that needs to be present and functional for the formation of a functional CCM complex. Such proteins have been described in the art, as for example by Kleaveland et al. (Nat Med. 2009 Feb;15(2):169-76. doi: 10.1038/nm.1918. Epub 2009 Jan 18), among others, and is therefore a clearly defined term as understood by a skilled person, as will be explained in the detailed description below.
Furthermore, in the context of the invention the at least one protein required for a functional CCM complex is CCM1. Additionally, the fish lacking at least one endogenous functional protein required for a functional CCM complex may be a zebrafish carrying a loss-of-function allele of *krit1,* such as *a krit1*$^{ty219c}$ allele, preferably a zebrafish homozygously carrying a loss-of-function allele of *krit1,* such as a *krit1*$^{ty219c}$ allele.
Loss-of-function alleles of *krit1* that can be used in the context of the invention comprise ty219c, m775, s234,s610,t26458 and sa44926.
In embodiments of the transgenic fish, lack of the least one endogenous functional protein required for a functional CCM complex is due to a knock down of expression by an antisense oligonucleotide, preferably an antisense oligonucleotide Morpholino, or another method of inactivating the gene function known to the person skilled in the art.
In the context of the invention, the endocardium-specific genetic system can comprise a Gal4-UAS system, wherein the Gal4-UAS system comprises a Gal4 gene, preferably Gal4ff, under the control of an endocardium-specific transcriptional regulator, such as an endocardium-specific promoter, and a nucleic acid sequence encoding said functional protein required for a functional CCM complex under the control of a UAS region. The use of the Gal4-UAS system is particularly advantageous, since established Gal4-lines can be combined with newly developed lines expressing the gene for transgenic expression under the control of a UAS element. Such lines can be crossed to the Gal4-line and lead to specific conditional gene expression.

As used herein, the use of the term "endocardium-specific" in the context of a genetic system, a transcriptional regulator, a promoter, an enhancer, a promoter/enhancer combination or the like relates to a genetic element that drives or activates gene expression in endocardial cells, whereas it is not activated and does not drive gene expression in (other) endothelial cells or other cells of the cardiovascular system. Endocardial cells as used herein can be regarded as a specific subtype of endothelial cells , that differ from endothelial cells by expression of certain endocardial-specific genes.

In embodiments, the endocardium-specific genetic system can comprise or consist of a endocardium-specific promoter or a genetic element that directly drives expression of said functional protein required for a functional CCM complex.

For example, in embodiments comprising a Gal4-UAS system the endocardium-specific expression of the Gal4 gene can be driven by an nfatc1 promoter. Furthermore, in the context of the invention endocardium-specific expression of the said functional protein required for a functional CCM complex can be driven directly by an *nfatc1* promoter. Further promoters that can be used in the context of the invention for endocardium-specific expression comprise the *spp1*-promoter and/or genetic elements controlling *spp1* in a fish, such as a zebrafish.

In embodiments of the invention, the functional version of said at least one protein is a fusion protein of the at least one functional protein and a reporter protein, such as a fluorescent reporter protein, such as for example mCherry, EGFP or any other fluorescent or chemiluminescent or otherwise traceable protein suitable for fusion to said protein required for a functional CCM complex.

Accordingly, the nucleic acid sequence encoding the functional version of said at least one protein can be fused to a nucleic acid sequence of a reporter gene encoding a reporter protein.

The resulting expression of a fusion protein of the at least one functional protein and a reporter protein may represent a function version of protein required for a functional CCM complex.

The transgenic fish of the invention is characterized in that it comprises a cardiovascular system with a heart that produces blood flow, and wherein said fish develops vascular lesions in cerebral blood vessels, in particular in lowly perfused cerebral blood vessels.

In embodiments, the transgenic fish comprises a functional cardiovascular system with a heart that produces blood flow within an endothelium, functional endothelial cells of the lateral dorsal aorta and/or lumenized intersegmental vessels, and wherein said fish develops vascular lesions in cerebral blood vessels, in particular in lowly perfused cerebral blood vessels.

In the context of the invention, the vascular lesions developed by the transgenic fish can be visualized. Preferably, the transgenic lesions can be visualized by a traceable reagent injected into the cardiovascular system of said transgenic fish, such as a traceable dye or labeled nanobeads, such as a fluorescent dyes or fluorescent nanobeads, for example by microangiography.

Furthermore, the lesions may be visualized by using a transgenic fish additionally expressing fluorescently-labelled blood proteins or blood cells. For example transgenes expressing fluorescently-labelled vitamin D-binding protein (VDBP), such as a green fluorescent protein (GFP)-tagged VDBP. Furthermore, the transgenic zebrafish line Tg(gata1 a:DsRed) or similar lines expressing fluorescently labelled blood cells may be used and can be selected by a skilled person. Further methods of visualizing vascular lesions in a transgenic fish are known to a person skilled in the art.

In the context of the invention, the transgenic fish can be at a stage of development selected from the group consisting of an embryo stage, a larva stage, and an adult stage.

*Embodiments relating to use of a transgenic fish of the invention and methods employing a transgenic fish of the invention*

The invention further relates to a method for measuring a pharmacological effect of a drug, comprising

- contacting a transgenic fish of the present invention with a drug, and

- measuring a pharmacological effect of the drug.

In the context of the method for measuring (or testing) a pharmacological effect of a drug as disclosed herein, the pharmacological effect of the drug is an effect on the formation of vascular lesions, the development of cerebral cavernous malformation and/or the development of stroke.

In this context, the pharmacological effect of the drug can be measured by comparing the formation of vascular lesions, the development of cerebral cavernous malformation and/or the development of stroke in the transgenic fish contacted with the drug to the formation of vascular lesions, the development of cerebral cavernous malformation and/or the development of stroke in a transgenic fish of the invention not contacted with said drug.

As used herein, the term "drug" relates to any compound, such as a biological or chemical molecule, such as a small molecule, a protein, a nucleic acid or any other compound that can be envisioned to be used as a drug in the treatment of a patient.

In the context of the invention, "contacting" a transgenic fish with a drug or compound comprises incubation of the fish in a suitable medium comprising said compound, but also any other form of administration of said compound to the transgenic fish comprising feeding/oral administration and injection, such as injection into a body cavity or into the blood stream or into a tissue. Suitable routes of administration are known to a skilled person.

The invention further relates to use of a transgenic fish of the present invention as a model for the treatment and/or prevention of vascular lesions, cerebral cavernous malformation and/or stroke. Furthermore, the invention comprises the use of a transgenic fish of the invention in a method of screening a compound for an effect on the formation of vascular lesions, the development of cerebral cavernous malformation and/or the development of stroke, the method comprising

- contacting said transgenic fish to a compound and
- measuring the formation of vascular lesions, the development of cerebral cavernous malformation and/or the development of stroke.

In the method of screening a compound for an effect on the formation of vascular lesions, the development of cerebral cavernous malformation and/or the development of stroke the measuring of the formation of vascular lesions, the development of cerebral cavernous malformation and/or the development of stroke may comprise comparing the formation of vascular lesions, the development of cerebral cavernous malformation and/or the development of stroke in the transgenic fish contacted with the compound to the formation of vascular lesions, the development of cerebral cavernous malformation and/or the development of stroke in a transgenic fish of the invention not contacted with said compound.

Further uses of the transgenic fish of the invention can be envisioned by the person skilled in the art. This includes use of the transgenic fish for various research purposes such as studying the mechanisms leading to the development of vascular lesions.

The various embodiments and features of the transgenic fish of the invention disclosed herein also apply to the various embodiments of the methods and uses employing such a transgenic fish presented herein, and vice versa.

## DETAILED DESCRIPTION OF THE INVENTION

The present invention relates to a transgenic fish, methods involving said transgenic fish and the use of said transgenic fish of the invention.

As used herein, "transgenic fish" refers to fish, or progeny of a fish, into which an exogenous nucleic acid (which may also be referred to as an exogenous construct in the context of the invention) has been introduced. A fish into which a construct has been introduced includes fish which have developed from embryonic cells into which the construct has been introduced. As used herein, an exogenous construct can be a nucleic acid that is artificially introduced, or was originally artificially introduced, into an animal. The term artificial introduction is intended to exclude introduction of a construct through normal reproduction or genetic crosses. That is, the original introduction of a gene or trait into a line or strain of animal by cross breeding is intended to be excluded. However, fish produced by transfer, through normal breeding, of an exogenous construct (that is, a construct that was originally artificially introduced) from a fish containing the construct are considered to contain an exogenous construct. Such fish are progeny of fish into which the exogenous construct has been introduced. As used herein, progeny of a fish are any fish which are descended from the fish by sexual reproduction or cloning, and from which genetic material has been inherited. In this context, cloning refers to production of a genetically identical fish from DNA, a cell, or cells of the fish. The fish from which another fish is descended is referred to as a progenitor fish. As used herein, development of a fish from a cell or cells (embryonic cells, for example), or development of a cell or cells into a fish, refers to the developmental process by which fertilized egg cells or embryonic cells (and their progeny) grow, divide, and differentiate to form an adult fish.

The invention relates to fish suitable for transgenic manipulation and introduction of exogenous nucleic acids. As used herein, fish refers to zebrafish. It is preferred that fish belonging to species and varieties of zebrafish of commercial or scientific interest are used.

The fish for use with the disclosed constructs and methods is zebrafish, *Danio rerio*. Zebrafish are a popular experimental animal since they have many of the advantages of popular invertebrate experimental organisms, and include the additional advantage that they are vertebrates. Another significant advantage of zebrafish for the study of development and cell lineages is that, like *Caenorhabditis,* they are largely transparent (Kimmel, Trends Genet 5:283-8 (1989)). The generation of thousands of zebrafish mutants (Driever et al., Development 123:37-46 (1996); Haffter et al.,

Development 123:1-36 (1996)) provides abundant raw material for transgenic study of these animals. General zebrafish care and maintenance is described by Streisinger, Natl. Cancer Inst. Monogr. 65:53-58 (1984).

Zebrafish embryos are easily accessible and nearly transparent. Given these characteristics, a transgenic zebrafish embryo, carrying a construct encoding a reporter protein and tissue-specific expression sequences, can provide a rapid real time *in vivo* system for analyzing spatial and temporal expression patterns of developmentally regulated genes. In addition, embryonic development of the zebrafish is extremely rapid. In 24 hours an embryo develops rudiments of all the major organs, including a functional heart and circulating blood cells (Kimmel, Trends Genet 5:283-8 (1989)).

As used herein, "nucleic acid" shall mean any nucleic acid molecule, including, without limitation, DNA, RNA and hybrids or modified variants thereof. An "exogenous nucleic acid" or "exogenous genetic element" or "exogenous construct" relates to any nucleic acid introduced into the cell, which is not a component of the cells "original" or "natural" genome or transcriptome. Exogenous nucleic acids may be integrated or non-integrated in the genetic material of the target cell of the fish of the invention, or relate to stably transduced nucleic acids.

Any given suitable delivery method for providing exogenous nucleic acids to cells of fish of the invention is encompassed by the invention. The transgenic fish of the invention can be produced by introducing a transgenic construct into cells of a fish, such as a zebrafish, preferably embryonic cells, and most preferably in a single cell embryo, essentially as described in Meng et al. (1998). The transgenic construct is preferably integrated into the genome of the zebrafish, however, the construct can also be constructed as an artificial chromosome.

The exogenous nucleic acids, which may be recombinant constructs, can be introduced into fish, preferably embryonic fish cells, using any suitable technique known in the art or later developed for the introduction of transgenic constructs. For example, microinjection, electroporation, liposomal delivery and particle gun bombardment can all be utilized to effect transgenic construct delivery to embryonic cells as well as other methods standard in the art for delivery of nucleic acids to zebrafish embryos or embryonic cells. Further included are microinjection (described by, for example, Culp et al. (1991) Proc Natl Acad Sci USA 88, 7953-7957), electroporation (described by, for example, Inoue et al.(1990), Cell. Differ. Develop. 29, 123-128; Muller et al. (1993), FEBS Lett. 324, 27-32; Murakami et al. (1994), J. Biotechnol. 34, 35-42; Muller et al. (1992), Mol. Mar. Biol. Biotechnol. 1, 276-281; and Symonds et al.(1994), Aquaculture 119, 313-327), particle gun bombardment (Zelenin et al. (1991), FEBS Lett. 287, 118-120), retroviral vectors (Lu et al (1997). Mol Mar Biol Biotechnol 6, 289-95), and the use of liposomes (Szelei et al. (1994), Transgenic Res. 3,116-119).

Zebrafish embryos can be obtained by mating adult zebrafish in specially designed mating tanks. Eggs are usually laid in the morning and are collected immediately so that they can be microinjected at the one cell stage. Embryonic cells can be obtained from zebrafish as described by Fan et al. (2004). Zebrafish containing a transgene can be identified by numerous methods such as probing the genome of the zebrafish for the presence of the transgene construct by Northern or Southern blotting. Polymerase chain reaction techniques can also be employed to detect the presence of the transgene.

In one embodiment, a transgenic fish of the present invention is one whose somatic and germ cells contain at least one genomically integrated copy of a recombinant construct of the invention. In another embodiment, the exogenous construct (exogenous nucleic acid) may not be integrated into the genome of the fish. The invention further provides a transgenic fish gamete, including a transgenic fish egg or sperm cell, a transgenic fish embryo, and any other type of transgenic fish cell or cluster of cells, whether haploid, diploid, triploid or other zygosity, having at least one copy of an exogenous nucleic acid of the present invention, which may be genomically integrated.

The "Tol2 transposon system" to generate transgenic zebrafish is known in the art (Kawakami, K. Genome Biol.; 8(Suppl 1): S7 (2007)). The medaka fish Tol2 element is an autonomous transposon that encodes a fully functional transposase. The transposase protein can catalyze transposition of a transposon construct that has 200 and 150 base pairs of DNA from the left and right ends of the Tol2 sequence, respectively. These sequences contain essential terminal inverted repeats and subterminal sequences. DNA inserts of fairly large sizes (as large as 11 kilobases) can be cloned between these sequences without reducing transpositional activity.

As used herein, the term "fish" comprises the embryo stage, larva stage and adult stage of the fish. The term "embryo" includes a single cell fertilized egg (i.e., a zygote) stage of the organism.

In particular, the recombinant construct is integrated into the fish's somatic and germ cells such that it is stable and inheritable (is stably transmitted through the germ line). The transgenic fish or fish cell preferably contains a multiplicity exogenous nucleic acids or constructs, which may be genomically integrated.

Progeny of the transgenic fish of the invention, and transgenic fish derived from a transgenic fish egg, sperm, embryo or other fish cell of the present invention, are also described. A fish is "derived from" a transgenic fish egg, sperm cell, embryo or other cell if the transgenic fish egg, sperm cell, embryo or other cell contributes DNA to the fish's genomic DNA. For example, a transgenic embryo of the present invention can develop into a transgenic fish of the present invention; a transgenic egg can be fertilized to create a transgenic embryo of the present invention that develops into a transgenic fish of the present invention; a transgenic sperm cell can be used to fertilize an egg to create a transgenic embryo of the present invention that develops into a transgenic fish of the present invention; and a transgenic cell can be used to clone a transgenic fish of the present invention. In some embodiments of the present invention, the transgenic fish is sterile. Further disclosed is a cell line derived from a transgenic fish embryo or other transgenic fish cell of the present invention, which contains at least one copy of a recombinant construct of the present invention. Methods of isolating such cells and propagating them are conventional.

In one embodiment, the disclosed transgenic fish of the present invention are produced by introducing a recombinant construct of the present invention into cells of a fish, particularly into embryonic cells, and more particularly into a single cell embryo. Where the transgene construct is introduced into embryonic cells, the transgenic fish is obtained by allowing the embryo to develop into a fish. Introduction of constructs into embryonic cells of fish, and subsequent development of the fish, are simplified by the fact that embryos develop outside of the parent fish.

Embryos or embryonic cells can generally be obtained by collecting eggs immediately after they are laid. It is generally preferred that the eggs be fertilized prior to or at the time of collection. This is particularly accomplished by placing a male and female fish together in a tank that allows egg collection under conditions that stimulate mating. After collecting eggs, one technique is that the embryo be exposed for introduction of genetic material by removing the chorion. This can be done manually or, particularly, by using a protease such as pronase. A fertilized egg cell prior to the first cell division is considered a one-cell embryo, and the fertilized egg cell is thus considered an embryonic cell.

After introduction of the transgene construct, the embryo is allowed to develop into a fish. This generally need involve no more than incubating the embryos under the same conditions used for incubation of eggs. However, the embryonic cells can also be incubated briefly in an isotonic buffer. If appropriate, expression of an introduced transgene construct can be observed during development of the embryo.

Fish harboring a transgene can be identified by any suitable means. For example, the genome of potential transgenic fish can be probed for the presence of construct sequences. To identify transgenic fish actually expressing the transgene, the presence of an expression product can be assayed. Several techniques for such identification are known and used for transgenic animals and most can be applied to transgenic fish. Probing of potential or actual transgenic fish for nucleic acid sequences present in or characteristic of a transgene construct can be accomplished by Southern or Northern blotting. Also, detection can be achieved using polymerase chain reaction (PCR) or other sequence-specific nucleic acid amplification techniques. Furthermore, the transgenic constructs/exogenous nucleic acids introduced into the fish of the invention may comprise reporter genes, such as fluorescent reporter genes, which are expressed in visible locations of the fish, as for example the eye. Examples of such tissue specific reporter genes for identification of transgenic fish are known to the person skilled in the art, such as for example cryaa:EGFP, where the fluorescent reporter protein EGFP is expressed under the control of the lens specific promoter of the cryaa gene of zebrafish.

After "founder" transgenic fish are identified, one can mate them to wild type fish to identify those fish which comprise the transgene in their germ cells. Transgenic fish of the present invention can be either male or female. A transgenic fish of the present invention can be hemizygous for the transgene, which is a particular state for maintenance of transgenic fish lines. Alternatively, hemizygous fish can be crossed with each other to produce homozygous fish or fish lines.

The transgenic fish of the invention lack at least one endogenous functional protein required for a functional CCM complex.

Cerebral cavernous malformations (CCMs) are a common vascular malformation with a prevalence of 0.1%-0.5% in the human population. CCMs arise primarily in the brain as thin-walled, dilated blood vessels that cause seizures, headaches and stroke in midlife, often in association with focal hemorrhage. Familial CCM shows an autosomal dominant pattern of inheritance and is caused by loss-of-function mutations in three genes: KRIT1 (also known as CCM1), CCM2 (also known as malcalvernin and osm) and PDCD10 (also known as CCM3). The CCM proteins are putative adaptor proteins that interact biochemically leading to the formation of a CCM complex and participate in a signaling pathway that is not yet fully characterized. A clue to the role of CCM protein signaling in the cardiovascular system comes from genetic studies in zebrafish, which reveal that loss of krit1, ccm2 or heg1 (encoding the type I transmembrane receptor heart of glass) results in a dilated heart phenotype early in development. This phenotype is characterized by heart failure associated with enlarged cardiac chambers in which the endocardium is covered by a thin layer of myocardial cells. Heg1 has been shown to also interact with the CCM complex comprising CCM1, CCM2 and/or CCM3 and functional Heg1 is required for a functional CCM complex. Expression of heg1, krit1 and ccm2 mRNA has been detected in the endocardium, but not the myocardium, of zebrafish embryos, suggesting that these proteins operate in an endothelial cell-autonomous signaling pathway. The involvement of CCM1-3 and Heg1 in the formation of a CCM phenotype and the formation of a functional CCM complex has been describe in detail in the literature, for example by Kleaveland et al. (Nature Medicine, VOLUME 15, NUMBER 2, FEBRUARY 2009, pp. 169-176) and Spiegeler et al. (Mol Syndromol 2018;9:60-69).

Signaling through the HEG1 receptor and the CCM proteins of the CCM complex is required to regulate endothelial cell-cell association during formation of the cardiovascular system and for its integrity thereafter. Accordingly, loss of function of at least one of Heg1, CCM1, CCM2 and CCM3 leads to a malfunction of the CCM complex and to malformation of the components of the cardiovascular system, such as failure of nascent endothelial cells, defects in the heart, the aorta, lymphatic vessels, abnormal endothelial cell junctions, dilated heart phenotype early in development, heart failure associated with enlarged cardiac chambers in which the endocardium is covered by a thin layer of myocardial cells. In the context of the present invention, a protein required for a functional CCM complex includes Heg1, CCM1, CCM2 and CCM3 and any other protein known by the skilled person or that will be identified as being required for a functional CCM1 complex.

CCM3 is encoded by two paralog genes *ccm3a* (*pdcd10a*) and *ccm3b* (*pdcd10b*) encoding the corresponding protein versions CCM3a and CCM3b. The paralog genes *ccm3a* and *ccm3b* have 83 % sequence homology and protein function of CCM3a and CCM3b are redundant. Accordingly, in case of a lack of a functional endogenous CCM3, both versions of the protein have are lacking, for example due to inactivation of both genes *ccm3a* and *ccm3b.*

In this context, a "functional" version of a protein is understood as any version of a protein that can fulfill a specific function. Accordingly, a functional protein or a functional version of a protein required for a functional CCM complex, such as for example a functional CCM1, relates to any version of a CCM1 protein that can support the function of the CCM complex. This may include mutated or truncated versions of the respective protein, for example CCM1, or version of the protein that have been fused to other proteinaceous components, such as a reporter protein.

In cases where the functional protein required for a functional CCM complex is a fusion protein of for example a function CCM1 protein and a reporter protein, a suitable linker sequence can be used. A skilled person is aware of such linkers and can design linker sequences that enable flexibility between the components of the fusion protein and do not interfere with the function of the components.

In the context of the invention, an "endogenous" functional protein is a protein that is encoded by an endogenous nucleic acid of the cell of the transgenic fish. Accordingly, an endogenous protein is not encoded by an exogenous nucleic acid of the transgenic fish, but can be encoded and expressed from nucleic acids occurring naturally in the fish, irrespective of the introduced genetic modification, such as from a genomic DNA sequence or a mRNA sequence generated from genomic DNA. A fish that lacks an endogenous functional protein can comprise a functional version of said protein that is encoded and expressed from an exogenous nucleic acid.

A fish lacking a functional endogenous protein required for a functional CCM complex comprises a modification, such as a genetic mutation, preventing the expression of a functional protein required a functional CCM complex, such as a function CCM1, CCM2, CCM3 or Heg1, from an endogenous nucleic acid sequence. Such modification, for example genetic mutations of the corresponding endogenous gene, are known to the person skilled in the art and can also be introduced into the genome of a fish, such as a zebrafish, by the skilled person.

For example, in the case of CCM1, also known as Krit1, many mutations of the endogenous *krit1* gene leading to lack of a functional endogenous Krit1 protein are known, such as for example the following *krit1*-alleles: ty219c, ty219c, m775, s234,s610,t26458, sa44926. Further loss of function alleles of *krit1* leading to malfunction of the CCM complex are known or can be designed. Similarly, suitable alleles or modification of other proteins required for a functional CCM complex such as Heg1, CCM2 and CCM3 can be identified or designed/developed by a skilled person.

Furthermore, the lack of at least one endogenous functional protein required for a functional CCM complex, such as CCM1 (KRIT1), CCM2, CCM3, and Heg1, can be induced by other means than alteration of the genomic DNA, such as knock down of mRNA by use of antisense RNA molecules, such as antisense oligonucleotide Morpholino mediated knockdown or any other method known to the skilled person for inactivating gene function. Alternatively, it may be possible to use specific inhibitors of the respective at least one endogenous protein, while using a transgenic version that is not inhibited by said inhibitor. Further suitable approaches leading to a lack of an endogenous functional protein can be developed or identified by a skilled person.

These methods can involve a tissue-specific knock-out through the targeted insertion of recombination elements (e.g. Cre/Lox-Recombination system), for example via CRISPR/Cas9-mediated genome engineering. Another more tedious method of generating such a disease model is cell transplantation of krit1 mutant cells into the wild-type host embryos. These mutant cells will, in some instances, generate mutant clones in regions of the vasculature susceptible to the formation of vascular lesions or vascular malformations.

Furthermore, the transgenic fish of the invention comprise an exogenous nucleic acid encoding a functional version of said at least one lacking endogenous protein required for a functional CCM complex. Said function version of the protein is under the control of a endocardium-specific genetic system, leading to expression of the functional protein from the exogenous nucleic acid in cells where the endocardium-specific genetic system gets activated. Accordingly, in such cells the lack or loss of the endogenous functional protein can be complemented by expression of the functional version of said protein form the exogenous nucleic acid.

The following *Table* 1 contains preferred protein sequence of functional proteins required for a functional CCM complex to be comprised by the transgenic fish of the invention. *Table* 2 contains preferred nucleic acid sequences that can be comprised by the exogenous nucleic acid of the transgenic fish of the invention. The amino acid sequence of *Table* 1 correspond to the wild-type sequences of CCM1, CCM2, CCM3 (paralog versions a and b as encoded by the corresponding paralog genes) and Heg1. The corresponding nucleic acid sequences encoding these proteins are listed in *Table 2.* Furthermore, *Table* 2 includes further preferred nucleic acid sequence that can be used in the context of the present invention.

*Table 1. Preferred amino acid sequences of the present invention.*

| SEQ ID NO. | Amino Acid Sequence | Description |
|---|---|---|
| SEQ ID NO 1 | MMGNQELEEVFVAVIRPKNTTSLNSKEYRAKSYEILLIEV PLEGKEKKRKKVLLGTKIHADSDRTKSILEFVDETTKPISN NQGIIGKRVVHMRKFLLDGDSGGKEASLFIVPINVKDNSK SVHHPGSPSFYCLQDIMRVCSETSAHFSSSTSKMLLALD KWLAEQHTVPHAIPALFRPAPVDRVKTNVSNPAYAVEKQ TDGTLHMGYTALEIKSKLMSLEKADLCIQNPLYGSDLQYT NRVDKVIINPYFGLGAPDYSKIQIPKRDKWQHSMTSVTED KERQWVDDFPLHRSACEGDTELLSKLLDGGFSVKQLDS DHWAPIHYACWHGKVEATKLLLEKGNCNPNLLNGQLSS PLHFAAIGGHAEIVQLLLQHPEIDRHIEDQQKRSPLQVCE ENKQNNWEETVNLLQQASNKPYEKVRIYRMDGSYRSVE LKHGNNTTVQQIMEGMRLSQETQQYFTIWICSENLSLQL KPYHKPLQHLRMWSEIVTDLTALDPQRESPQLFLRRDVR LPLEVEKKVEDPLSILILFDEARHCLLKGFLSTSDNKLITLA SLLLQIIYGNYDSKKHKQGFLNEENLKSIVPISKVKSKAHH WTNRILHEYKSLSTSEGVSKEMHHLQRLFLQNCWDIPTY GAAFFTGQVFTKASSSTHKVIRVYVGVNTKGLHLMNMET KVLHLSLEYGTFMWQLGQADQYVQIHSLENKKNFVVHTK QAGLIVKLLMKLSGQIAPNDRAVSDKYAYG | protein sequence of CCM1 (KRIT1) |
| SEQ ID NO 2 | MEEDVKKVKKPGIVSPFKRVFLKGEKGRDKKALEKSTER RALHTFSLSLPDHRIDPDILLNDYIEKEVKYLGQLTSVPGY LNPSSRTEVLQLIDNARKSHQLAGQLTSEQDAVVSLSAY NVKLVWRDGEDIILRVPIHDIAAVSYIRDDSLHLVVLKTAQ EPGGSPCHSTEMSKSPTLSSLSESGAVLVEVCCLLVLAV DNKAAAEELCLLLSQVFQIVYTESTIDFLDRAIFDGATTPT RHLSIYSEDSSSKVDVKDVFEAEASTFSFQSSLEAGHSS SPSPTSAPASPQTKTASESELSTTAAELLQDYMTTLRTKL SSKEIQQFATLLHEYRNGASIHEFCINLRQLYGDSRKFLLL GLRPFIPEKDSQHFENFLETIGVKDGRGIITDSFGRYKRTT SSASDSTTNGNGAAGGSDEGTATSEGDEWDRMISDISN DIEALGSSMDQDGVPS | protein sequence of CCM2 |
| SEQ ID NO 3 | MTMEEMKNEADATSMVSMTLYAVMYPVFNELESVNLSA AQTLRAAFKKAEKENPGLTQDIIMKILEKKNVEINFTESLL RMAADDVEEYMIDRPEREFQDLNERARALKQILSKIPDEI NDRVRFLQTIKDIASAIKELLDTVNNVFKKYQYQNRRALE HQKKEFVKHSKSFSDTLKTYFKDGKAINVFASANRLIHQT NLILQTFKTVA | protein sequence of Pdcd10a (Ccm3a) |

(continued)

| SEQ ID NO. | Amino Acid Sequence | Description |
|---|---|---|
| SEQ ID NO 4 | MTMEEMKNEAEPNSIVSMTLYAVMYPVFNELGRINPSAA QTLRAAFVKAEKENPGLTQDIIMKILEKKNVEINFTESLLR MAADDVEEYMIKRPEQEFQDLNEKARALKHILSKIPDEIN DRVRFLQTIKDIASAIKELLDTVNNVFRKYQYQNRRALEH QKKEFVKYSKSFSDTLKTYFKDGKAINVFISANRLIHQTNL ILQTFKTVA | protein sequence of Pdcd10b (Ccm3b) |
| SEQ ID NO 5 | MMETCARRVLFTAALLVLSTVIAETFSTDSDTDNPLSTET FYSRTSGLKQTSSWPGREATATAVDLSSGLGEMTEIPAS VSITAAREGHSPKPLQTSTNAADWKTSMTSDETTEHLQS DTELTHNATAQWESPSSASHSITSHHPVTETRTVRDVTD LIDMDTTDSVSHTDSTYISTTNRVGERTLLSVISNSTFAYT QNSSISDAESQTSPWEEKTSGATQVNEETEETVSTVSEQ TDPTFEDRNITSATLETGRSTLFQGTESQTGQPSVTGQT AKEVTDIDNPNSTPPLTVTSRDVEETDATSVSSETSYTQT SSDSASSILPFTSSERNVTSTSQESHNSTLIYSTNTGGST EFSTGSVSSTAHEETERSSTQIVDETTLHDVTSAPPVLED VATTIDDSLSKFPSGQSPTIPKTDDQTNTQVVPTSTHRPQ VTDEATDEVSTVYSSTTTLTTTTPSVTTRQLQPHHTTVET QTQHTTIVTTDIIQVLRTTPSTAHHVPTSTTSGPQAPSTAD SSDVTTLHLETSTATPGNTTAHGGRATTPFSKSSPSRTT VVVTTGHLTDKSTTETGSATTQMPLRTSASPGHVCGPKT CANGGHCVRSAEGSYYCQCLSAWTGPFCTEDVDECVN SPCPQGSVCVNTGGSFSCECDLGFDLEDGRSCTQVKTF LGTFTVNNSLHLRNLGLHELHREIQQLLNASLSIFHGYRR FTLGKRDGQGVQIPVVSMFSLSSNVTSADVFNSIQMSLN NCSRTYSHCPIKLQHQLSYHVESLCMAQKTKCDVQYSD CSDISGIPNCQCLPGYFKRNPEDMTCRDCGDGLKLVNG KCVECMFGFGGFNCNNFYKLIAVVVSPAGGALLLIVVIALI VTCCKKDKNDINKIIFKSGELQMSPYAEFPKSNRVSMEW GRETIEMQENGSTKNLLQMTDIYYSPALRNSDLERNGLY PFSGLPGSRHSCIYPAQWNPSFLSDDSRRRDYF | protein sequence of Heg1 |

*Table 2.* Nucleic acid sequence of the present invention.

| SEQ ID NO. | Nucleic Acid Sequence | Description |
|---|---|---|
| SEQ ID NO 6 | ATGATGGGAAACCAAGAGCTAGAGGAGGTGTTTGTTG CTGTTATCCGACCAAAAAACACAACCAGCCTCAACTCC AAAGAATACCGTGCTAAATCCTATGAGATTTTACTTATC GAAGTTCCCCTGGAGGGAAAAGAGAAAAAGCGGAAGA AAGTGCTGCTTGGGACTAAAATCCACGCCGACAGTGA CCGGACAAAGTCAATTCTGGAGTTTGTGGATGAAACCA CCAAGCCCATATCCAATAACCAGGGCATAATAGGGAA GCGTGTCGTGCACATGAGGAAGTTCCTGTTAGATGGA GACAGTGGAGGAAAAGAGGCCTCGTTATTTATTGTGC CAATTAATGTCAAAGACAACAGCAAGTCCGTGCATCAC CCTGGGAGCCCCAGTTTCTACTGCCTTCAGGACATCAT GCGTGTGTGTAGTGAAACCAGTGCTCATTTCTCCTCCA GCACCTCAAAGATGCTGCTCGCTCTCGACAAGTGGTT AGCGGAGCAGCACACTGTGCCTCACGCTATTCCTGCT CTGTTCCGGCCGGCTCCGGTGGACCGTGTGAAAACTA ACGTGAGTAACCCGGCGTACGCAGTGGAGAAGCAGA CAGACGGGACGCTGCACATGGGCTACACGGCTCTGG AGATCAAGAGTAAACTGATGTCTCTGGAGAAAGCAGAT CTGTGCATCCAGAACCCGCTGTACGGCTCAGATCTGC AGTACACCAACAGAGTGGACAAAGTCATCATCAACCCT TACTTTGGCTTGGGAGCTCCAGATTACTCCAAAATCCA | Coding nucleotide sequence of ccm1 (krit1) |

(continued)

| SEQ ID NO. | Nucleic Acid Sequence | Description |
|---|---|---|
| | GATTCCAAAGAGAGACAAATGGCAGCACAGCATGACC AGCGTCACCGAAGACAAAGAGCGCCAGTGGGTGGAT GATTTCCCTCTGCACCGAAGCGCCTGTGAGGGAGACA CTGAGCTTTTGTCTAAATTGCTGGATGGCGGATTCTCA GTCAAACAGCTGGACAGCGACCATTGGGCACCCATTC ATTATGCATGCTGGCATGGGAAGGTGGAGGCCACTAA ACTGCTGCTGGAGAAAGGCAACTGTAACCCCAACCTG CTGAACGGCCAGCTGAGCTCTCCTTTACACTTCGCAG CTATCGGCGGTCATGCAGAAATCGTGCAGCTCCTCCT GCAGCACCCAGAGATCGACAGACACATCGAGGACCAG CAGAAACGATCGCCGCTTCAAGTATGTGAAGAAAACAA GCAGAACAACTGGGAGGAAACCGTGAATCTCCTTCAA CAAGCCAGCAATAAACCATACGAGAAGGTGAGGATTT ACCGTATGGATGGATCGTACCGCTCTGTGGAGCTGAA GCATGGGAACAACACCACCGTGCAGCAGATCATGGAG GGCATGAGATTATCACAGGAGACCCAGCAGTATTTTAC CATCTGGATCTGCTCCGAAAACCTTAGTCTGCAGCTGA AGCCGTACCACAAGCCCTTGCAGCACTTACGGATGTG GAGCGAGATCGTGACGGATCTCACTGCTCTGGATCCA CAGAGAGAAAGTCCACAGCTCTTCCTGCGCAGAGACG TTCGGCTGCCTTTAGAGGTCGAGAAAAAGGTTGAAGA TCCGCTGTCCATCCTCATCCTATTTGATGAAGCTCGTC ACTGCCTCCTTAAAGGTTTCCTCTCGACGTCAGATAAC AAGCTAATCACGCTCGCAAGCCTCCTCCTGCAGATCAT CTACGGAAACTACGACAGCAAGAAGCACAAACAGGGC TTTCTAAATGAGGAAAACCTGAAATCCATAGTTCCCATT TCAAAGGTCAAGAGCAAAGCACATCACTGGACTAACA GGATACTTCACGAGTACAAGAGTCTGAGCACTAGTGA GGGTGTGAGTAAAGAAATGCATCACCTCCAGAGGCTC TTCCTGCAGAACTGCTGGGATATTCCTACTTATGGTGC AGCGTTTTTCACAGGACAGGTCTTCACTAAAGCCAGCT CCAGCACACACAAGGTGATCCGCGTGTATGTGGGCGT CAACACCAAAGGCCTGCATCTGATGAACATGGAGACA AAAGTGCTTCACCTCAGTCTGGAGTATGGGACATTTAT GTGGCAGCTGGGTCAGGCTGATCAATACGTGCAAATC CACAGTCTGGAGAACAAGAAGAACTTTGTCGTTCACAC CAAACAGGCTGGTCTTATCGTGAAGCTGCTGATGAAG CTGAGTGGACAAATTGCACCAAACGACAGAGCTGTGT CTGATAAATATGCGTATGGGTAA | |

(continued)

| SEQ ID NO. | Nucleic Acid Sequence | Description |
|---|---|---|
| SEQ ID NO 7 | ATGGAGGAGGATGTAAAGAAAGTCAAAAAGCCAGGTA TCGTGTCTCCGTTCAAGCGGGTGTTTCTGAAAGGGGA GAAGGGTAGGGATAAGAAGGCTCTGGAGAAGTCCACT GAACGCAGGGCCCTCCACACCTTCTCTCTCTCTCC CAGACCACCGTATCGACCCAGATATTCTGCTCAATGAC TACATCGAGAAGGAAGTTAAGTATCTGGGACAGCTGA CCTCAGTTCCTGGATACCTGAATCCCTCCAGCCGCACT GAAGTTCTGCAGCTCATTGACAATGCCAGGAAGTCTCA TCAGTTGGCTGGGCAGCTGACCTCAGAGCAGGATGCT GTTGTCAGTTTATCAGCGTACAATGTGAAGCTTGTGTG GCGTGATGGAGAGGACATCATTCTGCGCGTACCCATC CACGACATTGCAGCTGTGTCCTACATCAGAGACGACT CGCTTCATCTAGTGGTGCTAAAAACAGCTCAGGAGCC CGGAGGTTCCCCATGTCATAGTACAGAGATGTCGAAA TCTCCCACTCTGAGCTCTCTATCTGAGAGTGGGGCTGT GCTGGTGGAGGTCTGCTGTCTGCTTGTGCTGGCTGTT GACAACAAGGCTGCAGCAGAGGAGCTCTGTCTTCTAC TCAGTCAAGTCTTCCAGATCGTCTATACCGAGTCCACC ATCGACTTCCTAGACAGGGCCATCTTTGATGGAGCCA CAACACCCACCAGGCATCTCTCAATCTATAGTGAAGAC TCCTCAAGTAAAGTTGATGTCAAAGATGTGTTTGAGGC CGAGGCAAGCACATTCTCGTTCCAGAGTTCTCTAGAG GCGGGACATTCCTCTAGCCCCTCCCCCACCTCGGCCC CGGCCTCGCCACAAACCAAAACAGCCAGTGAGAGCGA ACTGAGCACAACAGCTGCTGAACTGCTGCAAGACTAC ATGACCACATTGAGGACAAAGCTTTCGTCCAAAGAGAT CCAGCAGTTTGCCACACTGTTACATGAATACAGAAACG GCGCCTCCATCCATGAGTTCTGTATCAACCTGCGGCA GCTTTATGGAGACAGCAGGAAGTTCCTTCTCCTCGGA CTGCGGCCGTTCATCCCCGAGAAGGACAGCCAGCATT TTGAGAACTTTCTGGAGACCATCGGCGTGAAGGATGG CCGCGGAATCATCACAGACAGTTTCGGCCGTTATAAG CGCACGACGAGCTCTGCGTCTGACTCCACCACGAACG GAAACGGTGCTGCGGGCGGATCAGATGAGGGAACAG CCACCTCTGAGGGCGACGAATGGGACCGCATGATTTC AGACATAAGCAATGACATTGAAGCCTTAGGAAGCAGC ATGGATCAAGACGGCGTGCCATCTTGA | Coding nucleotide sequence of ccm2 |

(continued)

| SEQ ID NO. | Nucleic Acid Sequence | Description |
|---|---|---|
| SEQ ID NO 8 | ATGACAATGGAAGAGATGAAGAATGAAGCGGACGCGA CCTCCATGGTATCCATGACCCTCTACGCCGTGATGTAC CCGGTGTTCAATGAGCTGGAGAGTGTAAATCTGTCTG CGGCACAGACTCTGCGAGCTGCTTTCAAAAAGGCAGA GAAGGAAAACCCAGGACTGACTCAGGACATTATCATG AAAATACTGGAGAAGAAGAACGTGGAGATCAACTTCAC CGAGTCTTTATTACGCATGGCTGCTGATGATGTTGAGG AGTACATGATTGACAGGCCCGAGCGGGAGTTCCAGGA CCTGAATGAAAGAGCTCGCGCGCTGAAACAGATCCTC AGCAAAATCCCAGACGAAATTAATGACCGAGTCCGATT CCTGCAGACAATCAAAGATATAGCCAGTGCCATAAAAG AGCTGCTGGACACAGTGAACAACGTCTTCAAGAAATAT CAGTACCAGAACAGAAGGGCACTTGAGCATCAGAAGA AAGAGTTTGTGAAACATTCGAAAAGCTTCAGCGACACT CTGAAAACATACTTCAAAGACGGAAAGGCAATAAACGT GTTTGCCAGTGCCAACCGGCTCATCCACCAAACCAAC TTGATACTGCAGACCTTCAAAACTGTCGCATAG | Coding nucleotide sequence of pdcd10a (ccm3a) |
| SEQ ID NO 9 | ATGACAATGGAAGAGATGAAGAATGAAGCTGAGCCAA ATTCAATAGTCTCTATGACATTGTACGCAGTCATGTATC CAGTCTTCAATGAGTTGGGGAGGATAAACCCGTCAGC AGCACAGACATTGCGGGCAGCATTTGTCAAAGCAGAG AAGGAGAACCCAGGGCTCACTCAAGATATCATTATGAA GATACTGGAGAAGAAGAATGTGGAGATCAACTTCACA GAGTCACTACTGCGCATGGCTGCAGATGATGTGGAGG AGTACATGATTAAGAGACCTGAACAGGAGTTCCAGGA CCTGAATGAGAAAGCACGAGCTCTAAAACATATCCTGA GTAAAATCCCAGATGAGATCAACGACAGAGTGCGCTT CCTACAGACTATCAAAGACATCGCCAGTGCTATAAAGG AGCTGCTGGACACAGTCAACAATGTTTTTAGGAAGTAT CAGTACCAGAATCGCAGGGCACTTGAACACCAAAAGA AGGAGTTTGTGAAGTACTCCAAAAAGCTTCAGTGATACG CTGAAAACATACTTCAAAGACGGAAAGGCAATAAACGT CTTTATCAGTGCCAACAGGCTTATCCACCAAACTAACT TGATACTGCAGACCTTCAAAACCGTCGCCTAG | Coding nucleotide sequence of pdcd10b (ccm3b) |

(continued)

| SEQ ID NO. | Nucleic Acid Sequence | Description |
|---|---|---|
| SEQ ID NO 10 | ATGATGGAAACGTGCGCTCGCCGTGTGCTTTTCACGG CCGCTCTGCTCGTCCTCAGTACTGTGATAGCGGAAAC TTTCTCCACAGACTCGGACACCGATAATCCACTGAGTA CAGAAACTTTTTATAGCCGAACAAGTGGCTTGAAACAA ACCTCCTCCTGGCCAGGGAGAGAGGCCACTGCCACA GCCGTGGATCTATCGAGCGGACTGGGAGAGATGACG GAGATTCCCGCCAGTGTATCCATCACTGCAGCCCGAG AGGGACATTCACCAAAACCTTTACAAACCTCTACAAAT GCAGCGGATTGGAAGACCAGTATGACCTCAGATGAGA CAACAGAACATCTTCAATCTGACACTGAGCTTACCCAT AATGCGACCGCCCAATGGGAGAGTCCATCATCAGCAT CTCACAGCATTACCAGCCACCATCCAGTAACAGAGAC ACGAACCGTGCGAGATGTAACAGATCTGATAGACATG GACACCACAGACTCAGTCTCCCACACTGATAGCACCT ACATTTCCACAACCAACCGAGTTGGAGAACGCACACT GCTCTCAGTGATCTCCAACAGCACCTTTGCGTACACCC AGAACTCAAGCATCTCTGATGCAGAGTCTCAAACGTCC CCATGGGAGGAGAAGACATCAGGAGCCACCCAAGTCA ATGAGGAAACTGAAGAAACTGTGTCAACAGTGTCCGA ACAGACTGATCCCACTTTTGAAGACCGCAATATCACCA GTGCAACTCTGGAGACTGGGCGGTCAACATTATTCCA AGGCACTGAGTCACAGACAGGACAACCTAGTGTCACA GGACAGACCGCTAAGGAGGTGACTGATATCGACAATC CAAATTCAACACCGCCTCTCACAGTAACCAGTAGGGAT GTTGAGGAAACAGATGCCACATCAGTGAGCAGTGAGA CATCTTATACACAAACCAGCAGTGACTCTGCCTCCTCA ATCTTGCCTTTTACTTCTAGTGAACGCAATGTCACTAG CACGTCCCAAGAGAGCCATAATTCCACACTCATTTATT CCACAAATACTGGTGGTTCCACTGAGTTTTCGACTGGA | Coding nucleotide sequence of heg1 |

(continued)

| SEQ ID NO. | Nucleic Acid Sequence | Description |
|---|---|---|
| | TCAGTGAGCTCTACCGCCCATGAAGAAACTGAACGGT CTTCAACTCAAATAGTAGATGAGACAACCCTTCATGAT GTTACTTCTGCACCCCCAGTGCTTGAAGATGTAGCCAC AACTATCGATGACTCGCTTTCCAAGTTCCCTTCCGGCC AATCGCCTACCATCCCTAAAACCGATGACCAAACCAAC ACACAAGTGGTGCCAACATCAACTCATAGGCCACAGG TTACAGATGAAGCCACCGATGAGGTGTCTACAGTTTAC AGTTCTACCACTACTTTAACTACCACAACTCCTTCTGTC ACCACTAGACAACTCCAACCACACCACACCACAGTGG AAACCCAAACACAACACACTACCATTGTTACCACCGAT ATCATTCAGGTACTGCGAACGACACCTTCTACAGCCCA TCATGTGCCTACATCGACTACCAGTGGACCACAGGCT CCAAGTACAGCTGATTCTTCTGACGTCACCACATTGCA CTTGGAAACCAGCACAGCCACGCCGGGGAACACTACG GCGCACGGTGGACGTGCAACAACGCCTTTTAGCAAGA GCAGCCCGAGTAGAACAACTGTGGTAGTGACCACTGG ACATCTCACTGACAAAAGCACTACAGAGACAGGAAGC GCAACCACGCAGATGCCTCTTAGAACATCAGCATCAC CAGGTCATGTATGTGGACCTAAAACCTGTGCAAATGGA GGTCATTGTGTTAGATCAGCTGAAGGAAGTTACTACTG TCAGTGTCTCTCCGCATGGACCGGACCCTTCTGCACT GAAGATGTGGACGAGTGTGTGAACAGTCCATGTCCTC AGGGTTCAGTGTGTGTCAACACAGGTGGTTCTTTCAGC TGTGAATGTGACCTGGGCTTTGACCTGGAGGATGGCC GCAGTTGTACACAAGTCAAGACATTTTTGGGCACTTTC ACAGTCAACAACTCTCTTCATCTCAGAAATTTAGGTCT GCACGAGCTACACAGAGAGATCCAACAGCTGCTCAAT GCTTCTCTCTCCATCTTCCATGGTTATAGACGCTTTAC CCTGGGTAAAAGAGATGGACAAGGTGTGCAAATCCCA GTGGTGAGCATGTTTTCACTCTCCTCCAATGTGACCAG CGCGGATGTTTTCAACAGCATCCAGATGTCCCTTAACA ACTGCAGCCGGACATACTCGCACTGTCCCATTAAACTT CAGCACCAGCTCTCCTATCACGTGGAGAGCCTGTGCA TGGCCCAGAAGACCAAGTGTGATGTGCAGTACTCAGA TTGCTCGGATATTAGCGGGATTCCCAACTGTCAGTGCC TTCCTGGGTACTTTAAAAGGAACCCAGAAGACATGACC TGCAGAGACTGTGGAGATGGACTCAAGCTTGTTAATG GCAAATGTGTCGAATGCATGTTTGGATTTGGAGGTTTC AACTGCAATAATTTCTATAAGCTGATAGCCGTGGTGGT CTCGCCTGCTGGAGGAGCTCTTCTGTTGATTGTGGTC ATTGCTCTTATTGTCACCTGCTGCAAAAAAGACAAAAA CGACATCAACAAGATCATCTTCAAAAGTGGAGAGCTTC AGATGTCGCCATACGCAGAGTTTCCTAAGAGCAATCG CGTGTCCATGGAGTGGGGTAGAGAGACTATCGAGATG CAGGAGAACGGCAGTACAAAAAAACCTCCTGCAAATGA CTGACATTTATTACTCGCCTGCACTGAGAAACTCCGAC CTGGAGCGTAATGGTCTGTATCCATTCTCGGGCCTTCC TGGTTCAAGGCATTCCTGCATCTATCCGGCTCAGTGGA | |

(continued)

| SEQ ID NO. | Nucleic Acid Sequence | Description |
|---|---|---|
| | ACCCTTCCTTCTTAAGCGACGATTCACGCCGAAGAGA CTACTTTTGA | |

(continued)

| SEQ ID NO. | Nucleic Acid Sequence | Description |
|---|---|---|
| SEQ ID NO 11 | TACCACCTAAATTGTAAGCGTTAATATTTTGTTAAAATT CGCGTTAAATTTTTGTTAAATCAGCTCATTTTTTAACCA ATAGGCCGAAATCGGCAAAATCCCTTATAAATCAAAG AATAGACCGAGATAGGGTTGAGTGTTGTTCCAGTTTGG AACAAGAGTCCACTATTAAAGAACGTGGACTCCAACGT CAAAGGGCGAAAAACCGTCTATCAGGGCGATGGCCCA CTACGTGAACCATCACCCTAATCAAGTTTTTTGGGGTC GAGGTGCCGTAAAGCACTAAATCGGAACCCTAAAGGG AGCCCCGATTTAGAGCTTGACGGGGAAAGCCGGCG AACGTGGCGAGAAAGGAAGGGAAGAAAGCGAAAGGA GCGGGCGCTAGGGCGCTGGCAAGTGTAGCGGTCACG CTGCGCGTAACCACCACACCCGCCGCGCTTAATGCGC CGCTACAGGGCGCGTCCCATTCGCCATTCAGGCTGCG CAACTGTTGGGAAGGGCGATCGGTGCGGGCCTCTTCG CTATTACGCCAGCTGGCGAAAGGGGGATGTGCTGCAA GGCGATTAAGTTGGGTAACGCCAGGGTTTTCCCAGTC ACGACGTTGTAAAACGACGGCCAGTGAGCGCGCGTAA TACGACTCACTATAGGGCGAATTGGGTACCAAATAGTA GGAATTACCCACCTGTACAAGTGCTGAAAACTTGGATG AATAAGCCCGTTTGCCATTTTCTACTGCTATTTTAAATC TTTTCTGTATTTGTCTGCATTTGTCTTTACCCTTGAATA AATGTTTTAGTTGTTTTTTTTCAATTATGACTGTGTTTAA CAGACATTATTACAATAATATGTAATAGTAGTACACACT ATTATTATGTAATAGTACTTGTTGACTGTATTTGAGAAC TGGACCGAGTGAGTGTTACGTCACCCATTCAAAATGAC TTACTTCTGGCTCCAACAAAATGAAGTTAATTCAGTTG CCATTTTTCACTGTATGGACATCGCCGTGTTGGAGCTA GACGTCGCCAATAAGCAAGAAAGAGCCGAGATGCGTC GAGTCTGAGTCACCGTTCCTATGGCAACCCCTCTAACC AATCAGAAGTAAGCTTGTTGGAAGTCCACAGCCTACCA CTTGAAAGCGGGCTGCACAAAATCTGTCAAACGTTTTG AACGTTGGATGTGAGAGCACATACTTTTATTAAGGCAT CTGGTTGGTCAGTTTATAATATCAACAACTTGGGCTAC AGAAAAGAAAGTTATTACAGAAATTATGATTAACAAGT ACATGTTAAATAAAGATTTTAATATGAATGCCACCACTG GAGCATTCATGCCATTTGGAGCTTCTTCCTGTTTGGAT CACTAGAAGGAGGAGGTCACTCATTACAGTTCTCATAT ACAGTCGTTGGTTGGTTGGTTGGTTGGTTGGTTGGTA GATTGATTGATTGATTGATTGATTGATTGATTGATTGAT TGATTGATTGATTGATTGATTGATTGATTGATTGATTGA TTGATTGATTGATTGATTGATTGATTGGTAGTCAAAATA AGAAATAATTTCCACAGATTCATTACAGAAATGATTAAA TGCATACATAAAAACTGGGGGGGGGGGGGATACAACA ACACACTTAAGTCACATTTGCCTACGTAAAGAAAAGTA | Plasmid Sequenz of pDestTol2_UAS: mChe rry-Krit1,alpha-cry:EGFP used in the examples of the invention |

(continued)

| SEQ ID NO. | Nucleic Acid Sequence | Description |
|---|---|---|
|  | AAGAAAATCAATAGCTATATTTTACATCTCCTTTTTTTG CTGTCTTTTAATTAGCCTTGTTTTGCTGTTATCTTGATT GAAACTGTAACTTCTTCACCTGCTTCTTTTCTTTGTAGG TTTTGCCAGCCCAGAAACAGGCATGGCTGTGCAAGGC CAGAGCACCATGCACAATGCGCTGCATGTCTTCATGA ACGGCTCAATGTCCTCAGTCCAGGGCTCAGCCAACGA CCCCATCTTCCTCCTTCACCATGCTTTCATTGACAGGT AACAAACACGTCATGACATTAGACTGCACAGTTTTTGA CAAAGTTCATACAATCTGTTGTTTATAGCTGCTACAATT AGTGAAGTTTGTGAATGTACTTGGATGAGCAGCGAAA GATCAATTGAGATCAATTGTTAGAGTTTGGTTGCCCTG CAGAGCAAAGAACAAAAAATAATCTGGTGGCTTTACTG CGTGAGGTTATTATTGGTGGAATAGAAACACAAAACAT AATTGCATTTATTTGTTTAATTTTTTATCTTATCTTAACTT TCATCTTGCATATTTGTTTCTTACATCATTTCTAGCATCT TTGAGCGCTGGCTAAGAACTCATCAGCCTCCCCGGTC CATCTACCCACGTACCAATGCACCAATTGGCCACAATG ACGGCTACTACATGGTGCCATTCCTTCCTCTTTATAGG AATGGAGACTACCTCCTGTCCAACAAGGCTCTTGGATA CGAGTACGCCTACCTGTTGGACCCAGGTCATTGCACA ACACCAGAAATGCCCTCTGATCTGCAAAAGACGTGAAT ATCTGTTCAGACACCCATATCCACTCTGTTCCACACAG GTCAGAGGTTTGTCCAGGAGTTCTTGACAGAGGTGTA AAAAGTACTCAAAAATTTTACTCAAGTGAAAGTACAAGT ACTTAGGGAAAATTTTACTCAATTAAAAGTAAAAGTATC TGGCTAGAATCTTACTTGAGTAAAAGTAAAAAAGTACT CCATTAAAATTGTACTTGAGTATTAAGGAAGTAAAAGTA AAAGCAAGAAAGAAAACTAGAGATTCTTGTTTAAGCTT TTAATCTCAAAAAACATTAAATGAAATGCATACAAGGTT TTATCCTGCTTTAGAACTGTTTGTATTTAATTATCAAACT ATAAGACAGACAATCTAATGCCAGTACACGCTACTCAA AGTTGTAAAACCTCAGATTTAACTTCAGTAGAAGCTGA TTCTCAAAATTGTTAGTGTCAAGCCTAGCTCTTTTGGG GCTGAAAAGCAATCCTGCAGTGCTGAAAAGCCTCTCA CAGGCAGCCGATGCGGGAAGAGGTGTATTAGTCTTGA TAGAGAGGCTGCAAATAGCAGGAAACGTGAGCAGAGA CTCCCTGGTGTCTGAAACACAGGCCAGATGGGCCCTC GAGCAGGAAACAGCTATGACCATGATTACGCCAAGCT ATCAACTTTGTATAGAAAAGTTGGTAATACGACTCACTA TAGGGCGAATTGGGTACCGGGCCCCCCCTCGAGGTC GACGGTATCGATAAGCTTAGGCCTCCAAGGCGGAGTA CTGTCCTCCGGGCTGGCGGAGTACTGTCCTCCGGCAA GGTCGGAGTACTGTCCTCCGACACTAGAGGTCGGAGT ACTGTCCTCCGACGCAAGGCGGAGTACTGTCCTCCGG GCTGGCGGAGTACTGTCCTCCGGCAAGGTCGGAGTA CTGTCCTCCGACACTAGAGGTCGGAGTACTGTCCTCC GACGCAAGGCGGAGTACTGTCCTCCGGGCTGGCGGA GTACTGTCCTCCGGCAAGGGTCGACTCTAGAGGGTAT |  |

(continued)

| SEQ ID NO. | Nucleic Acid Sequence | Description |
|---|---|---|
| | ATAATGGATCCCATCGCGTCTCAGCCTCACTTTGAGCT CCTCCACACGAATTCCTGCAGCCCGGGGGATCCACTA GTTCTAGAGCGGCCGCCACCGCGGTGGAGCTCCAGC TTTTGTTCCCTTTAGTGAGGGTTAATTCAAGTTTGTACA AAAAAGCAGGCTCATGGCATGGTGAGCAAGGGCGAG GAcGACAACATGGCCATCATCAAGGAGTTCATGCGCTT CAAGGTGCACATGGAGGGCTCCGTGAACGGCCACGA GTTCGAGATCGAGGGCGAGGGCGAGGGCCGCCCCTA CGAGGGCACCCAGACCGCCAAGCTGAAGGTGACCAA GGGCGGCCCCCTGCCCTTCGCCTGGGACATCCTGTC CCCTCAGTTCATGTACGGCTCCAAGGCCTACGTGAAG CACCCCGCCGACATCCCCGACTACTTGAAGCTGTCCT TCCCCGAGGGCTTCAAGTGGGAGCGCGTGATGAACTT CGAGGACGGCGGCGTGGTGACCGTGACCCAGGACTC CTCCCTGCAGGACGGCGAGTTCATCTACAAGGTGAAG CTGCGCGGCACCAACTTCCCCTCCGACGGCCCCGTAA TGCAGAAGAAGACCATGGGCTGGGAGGCCTCCTCCG AGCGGATGTACCCCGAGGACGGCGCCCTGAAGGGGCG AGATCAAGCAGAGGCTGAAGCTGAAGGACGGCGGCC ACTACGACGCCGAGGTCAAGACCACCTACAAGGCCAA GAAGCCCGTGCAGCTGCCCGGCGCCTACAACGTCAA CATCAAGCTGGACATCACCTCCCACAACGAGGACTAC ACCATCGTGGAACAGTACGAGCGCGCCGAGGGCCGC CACTCCACCGGCGGCATGGACGAGCTGTACAAGGGA GGCGCTGCAGGAGGCTCTAGAATGGGAAACCAAGAG CTAGAGGAGGTGTTTGTTGCTGTTATCCGACCAAAAAA CACAACCAGCCTCAACTCCAAAGAATACCGTGCTAAAT CCTATGAGATTTTACTTATCGAAGTTCCCCTGGAGGGA AAAGAGAAAAAGCGGAAGAAAGTGCTGCTTGGGACTA AAATtCACGCCGACAGTGACCGGACAAAGTCAATTCTG GAGTTTGTGGATGAAACCACCAAGCCCATATCCAATAA CCAGGGCATAATAGGGAAGCGTGTtGTGCACATGAGG AAGTTCCTGTTAGATGGAGACAGTGGAGGAAAAGAGG CCTCGTTATTTATTGTGCCAATTAATGTCAAAGACAACA GCAAGTCCGTGCATCACCCTGGGAGCCCCAGTTTCTA CTGCCTTCAGGACATCATGCGTGTGTGTAGTGAAACC AGTGCTCATTTCTCCTCCAGCACCTCAAAGATGCTGCT CGCTCTCGACAAGTGGTTAGCGGAGCAGCACACTGTG CCTCACGCTATTCCTGCTCTGTTCCGGCCGGCTCCGG TGGACCGTGTGAAAACTAACGTGAGTAACCCGGCGTA CGCAGTGGAGAAGCAGACAGACGGGACGCTGCACAT GGGCTACACGGCTCTGGAGATCAAGAGTAAACTGATG TCTCTGGAGAAAGCAGATCTGTGCATCCAGAACCCGC TGTACGGCTCAGATCTGCAGTACACCAACAGAGTGGA CAAAGTCATCATCAACCCTTACTTTGGCTTGGGAGCTC CAGATTACTCCAAAATCCAGATTCCAAAGAGAGACAAA TGGCAGCACAGCATGACCAGCGTCACCGAAGACAAAG AGCGCCAGTGGGTGGATGATTTCCCTCTGCACCGAAG | |

(continued)

| SEQ ID NO. | Nucleic Acid Sequence | Description |
|---|---|---|
| | CGCCTGTGAGGGAGACACTGAGCTTTTGTCTAAATTGC TGGATGGCGGATTCTCAGTCAAACAGCTGGACAGCGA CCATTGGGCACCCATTCATTATGCATGCTGGCATGGG AAGGTGGAGGCCACTAAACTGCTGCTGGAGAAAGGCA ACTGTAACCCCAACCTGCTGAACGGCCAGCTGAGCTC TCCTTTACACTTCGCAGCTATCGGCGGTCATGCAGAAA TCGTGCAGCTCCTCCTGCAGCACCCAGAGATCGACAG ACACATCGAGGACCAGCAGAAACGATCGCCGCTTCAA GTATGTGAAGAAACAAGCAGAACAACTGGGAGGAAA CCGTGAATCTCCTTCAACAAGCCAGCAATAAACCATAC GAGAAGGTGAGGATTTACCGTATGGATGGATCGTACC GCTCTGTGGAGCTGAAGCATGGGAACAACACCACCGT GCAGCAGATCATGGAGGGCATGAGATTATCACAGGAG ACCCAGCAGTATTTTACCATCTGGATCTGCTCCGAAAA CCTTAGTCTGCAGCTGAAGCCGTACCACAAGCCCTTG CAGCACTTACGGATGTGGAGCGAGATCGTGACGGATC TCACTGCTCTGGATCCACAGAGAGAAAGTCCACAGCT CTTCCTGCGCAGAGACGTTCGGCTGCCTTTAGAGGTC GAGAAAAAGGTTGAAGATCCGCTGTCCATCCTCATCCT ATTTGATGAAGCTCGTCACTGCCTCCTTAAAGGTTTCC TCTCGACGTCAGATAACAAGCTAATCACGCTCGCAAG CCTCCTCCTGCAGATCATCTACGGAAACTACGACAGC AAGAAGCACAAACAGGGCTTTCTAAATGAGGAAAACCT GAAATCCATAGTTCCCATTTCAAAGGTCAAGAGCAAAG CACATCACTGGACTAACAGGATACTTCACGAGTACAAG AGTCTGAGCACTAGTGAGGGTGTGAGTAAAGAAATGC ATCACCTCCAGAGGCTCTTCCTGCAGAACTGCTGGGA TATTCCTACTTATGGTGCAGCGTTTTTCACAGGACAGG TCTTCACTAAAGCCAGCTCCAGCACACACAAGGTGATC CGCGTGTATGTGGGCGTCAACACCAAAGGCCTGCATC TGATGAACATGGAGACAAAAGTGCTTCACCTCAGTCTG GAGTATGGGACATTTATGTGGCAGCTGGGTCAGGCTG ATCAATACGTGCAAATCCACAGTCTGGAGAACAAGAAG AACTTTGTCGTTCACACCAAACAGGCTGGTCTTATCGT GAAGCTGCTGATGAAGCTGAGTGGACAAATTGCACCA AACGACAGAGCTGTGTCTGATAAATATGCGTATGGGTA AACCCAGCTTTCTTGTACAAAGTGGAATTCACTAGTGA TTTAATAGTGTGCATTCAGTGCAGGATGTTATGTATTTA TTTTCTGTAGTGCAAACTTTTTCAAAGAATTTTCCAGTC TAAGCTTTTCAGTCAAGAAAAAAAAGGCGTCTGATTA TCTGTGATATTTTAAATAATTAGCTCTTAAATCTGGTGA ACCAAGTGGCCACAACACATTAAATACTGACGTTCTAC AGCCATGTAGCTTCATCTGGTCTGGTTTGTTTTGGCAG GCACTTATTTTCCGGTGATAGTTGGCCACAAGAGTATT GTCTGAGCCATTCAGTGCTAGACTGTCATTCTCAGGTC AGAATCCACCCCACTGATTCTGCTGACAACACTGTTCC CACCATGAGATAATGCCATTCCAGAGAGATCCATTTGT AAGCCCCTCTTTCTGCAGCACAGGTATATAACCAGGG | |

(continued)

| SEQ ID NO. | Nucleic Acid Sequence | Description |
|---|---|---|
| | CTCTGCCTCCACTAAGGCCGGCACACATCATTTGGGG<br>ATCTTTGTACTGTACTAGCCTCTATTAGCCTCCTACTTG<br>CGTTCAGTTCACATCTTTTGTTCATCAGGTGCAATAAGT<br>TACAGTAAGGAGTTACCAGGTCTGACATTAATCGAATT<br>CCACCATGGTGAGCAAGGGCGAGGAGCTGTTCACCG<br>GGGTGGTGCCCATCCTGGTCGAGCTGGACGGCGACG<br>TAAACGGCCACAAGTTCAGCGTGTCCGGCGAGGGCG<br>AGGGCGATGCCACCTACGGCAAGCTGACCCTGAAGTT<br>CATCTGCACCACCGGCAAGCTGCCCGTGCCCTGGCCC<br>ACCCTCGTGACCACCCTGACCTACGGCGTGCAGTGCT<br>TCAGCCGCTACCCCGACCACATGAAGCAGCACGACTT<br>CTTCAAGTCCGCCATGCCCGAAGGCTACGTCCAGGAG<br>CGCACCATCTTCTTCAAGGACGACGGCAACTACAAGA<br>CCCGCGCCGAGGTGAAGTTCGAGGGCGACACCCTGG<br>TGAACCGCATCGAGCTGAAGGGCATCGACTTCAAGGA<br>GGACGGCAACATCCTGGGGCACAAGCTGGAGTACAAC<br>TACAACAGCCACAACGTCTATATCATGGCCGACAAGCA<br>GAAGAACGGCATCAAGGTGAACTTCAAGATCCGCCAC<br>AACATCGAGGACGGCAGCGTGCAGCTCGCCGACCAC<br>TACCAGCAGAACACCCCCATCGGCGACGGCCCCGTG<br>CTGCTGCCCGACAACCACTACCTGAGCACCCAGTCCG<br>CCCTGAGCAAAGACCCCAACGAGAAGCGCGATCACAT<br>GGTCCTGCTGGAGTTCGTGACCGCCGCCGGGATCACT<br>CTCGGCATGGACGAGCTGTACAAGGGCGGTGGAAGA<br>TCTGGGAATTCAAGGCCTCTCGAGCCTCTAGATTCTGC<br>AGCCCTATAGTGAGTCGTATTACGTAGATCCAGACATG<br>ATAAGATACATTGATGAGTTTGGACAAACCACAACTAG<br>AATGCAGTGAAAAAAATGCTTTATTTGTGAAATTTGTGA<br>TGCTATTGCTTTATTTGTAACCATTATAAGCTGCAATAA<br>ACAAGTTAACAACAACAATTGCATTCATTTTATGTTTCA<br>GGTTCAGGGGGAGGTGTGGGAGGTTTTTTCCAACTTT<br>ATTATACATAGTTGATAATTCACTGGCCGTCGTTTTACA<br>TCGATGATGATCCAGACATGATAAGATACATTGATGAG<br>TTTGGACAAACCACAACTAGAATGCAGTGAAAAAAATG<br>CTTTATTTGTGAAATTTGTGATGCTATTGCTTTATTTGTA<br>ACCATTATAAGCTGCAATAAACAAGTTAACAACAACAAT<br>TGCATTCATTTTATGTTTCAGGTTCAGGGGGAGGTGTG<br>GGAGGTTTTTTAAAGCAAGTAAAACCTCTACAAATGTG<br>GTATGGCTGATTATGATCCTCTAGATCAGATCTGCGAA<br>GATACGGCCACGGGTGCTCTTGATCCTGTGGCTGATT<br>TTGGACTGTGCTGCTCGCAGCTGCTGATGAATCACATA<br>CTTCCTCCATTTTCTTCCACTGATTGACTGTTATAATTT<br>CCCTAATTTCCAGGTCAAGGTGCTGTGCATTGTGGTAA<br>TAGATGTGACATGACGTCACTTCCAAAGGACCAATGAA<br>CATGTCTGACCAATTTCATATAATGTGAAAACGATTTTC<br>ATAGGCAGAATAAATAACATTTAAATTAAACTGGGCAT<br>CAGCGCAATTCAATTGGTTTGGTAATAGCAAGGGAAAA<br>TAGAATGAAGTGATCTCCAAAAAATAAGTACTTTTTGAC | |

(continued)

| SEQ ID NO. | Nucleic Acid Sequence | Description |
|---|---|---|
| | TGTAAATAAAATTGTAAGGAGTAAAAAGTACTTTTTTTT<br>CTAAAAAAATGTAATTAAGTAAAAGTAAAAGTATTGATT<br>TTTAATTGTACTCAAGTAAAGTAAAAATCCCCAAAAATA<br>ATACTTAAGTACAGTAATCAAGTAAAATTACTCAAGTAC<br>TTTACACCTCTGGTTCTTGACCCCCTACCTTCAGCAAG<br>CCCAGCAGATCCACTAGTTCTAGAGCGGCCGCCACCG<br>CGGTGGAGCTCCAGCTTTTGTTCCCTTTAGTGAGGGTT<br>AATTGCGCGCTTGGCGTAATCATGGTCATAGCTGTTTC<br>CTGTGTGAAATTGTTATCCGCTCACAATTCCACACAAC<br>ATACGAGCCGGAAGCATAAAGTGTAAAGCCTGGGGTG<br>CCTAATGAGTGAGCTAACTCACATTAATTGCGTTGCGC<br>TCACTGCCCGCTTTCCAGTCGGGAAACCTGTCGTGCC<br>AGCTGCATTAATGAATCGGCCAACGCGCGGGGAGAG<br>GCGGTTTGCGTATTGGGCGCTCTTCCGCTTCCTCGCT<br>CACTGACTCGCTGCGCTCGGTCGTTCGGCTGCGGCGA<br>GCGGTATCAGCTCACTCAAAGGCGGTAATACGGTTAT<br>CCACAGAATCAGGGGATAACGCAGGAAAGAACATGTG<br>AGCAAAAGGCCAGCAAAAGGCCAGGAACCGTAAAAAG<br>GCCGCGTTGCTGGCGTTTTTCCATAGGCTCCGCCCCC<br>CTGACGAGCATCACAAAAATCGACGCTCAAGTCAGAG<br>GTGGCGAAACCCGACAGGACTATAAAGATACCAGGCG<br>TTTCCCCCTGGAAGCTCCCTCGTGCGCTCTCCTGTTCC<br>GACCCTGCCGCTTACCGGATACCTGTCCGCCTTTCTC<br>CCTTCGGGAAGCGTGGCGCTTTCTCATAGCTCACGCT<br>GTAGGTATCTCAGTTCGGTGTAGGTCGTTCGCTCCAA<br>GCTGGGCTGTGTGCACGAACCCCCCGTTCAGCCCGAC<br>CGCTGCGCCTTATCCGGTAACTATCGTCTTGAGTCCAA<br>CCCGGTAAGACACGACTTATCGCCACTGGCAGCAGCC<br>ACTGGTAACAGGATTAGCAGAGCGAGGTATGTAGGCG<br>GTGCTACAGAGTTCTTGAAGTGGTGGCCTAACTACGG<br>CTACACTAGAAGGACAGTATTTGGTATCTGCGCTCTGC<br>TGAAGCCAGTTACCTTCGGAAAAAGAGTTGGTAGCTCT<br>TGATCCGGCAAACAAACCACCGCTGGTAGCGGTGGTT<br>TTTTTGTTTGCAAGCAGCAGATTACGCGCAGAAAAAAA<br>GGATCTCAAGAAGATCCTTTGATCTTTTCTACGGGGTC<br>TGACGCTCAGTGGAACGAAAACTCACGTTAAGGGATTT<br>TGGTCATGAGATTATCAAAAAGGATCTTCACCTAGATC<br>CTTTTAAATTAAAAATGAAGTTTTAAATCAATCTAAAGTA<br>TATATGAGTAAACTTGGTCTGACAGTTACCAATGCTTA<br>ATCAGTGAGGCACCTATCTCAGCGATCTGTCTATTTCG<br>TTCATCCATAGTTGCCTGACTCCCCGTCGTGTAGATAA<br>CTACGATACGGGAGGGCTTACCATCTGGCCCCAGTGC<br>TGCAATGATACCGCGAGACCCACGCTCACCGGCTCCA<br>GATTTATCAGCAATAAACCAGCCAGCCGGAAGGGCCG<br>AGCGCAGAAGTGGTCCTGCAACTTTATCCGCCTCCAT<br>CCAGTCTATTAATTGTTGCCGGGAAGCTAGAGTAAGTA<br>GTTCGCCAGTTAATAGTTTGCGCAACGTTGTTGCCATT<br>GCTACAGGCATCGTGGTGTCACGCTCGTCGTTTGGTA | |

(continued)

| SEQ ID NO. | Nucleic Acid Sequence | Description |
|---|---|---|
| | TGGCTTCATTCAGCTCCGGTTCCCAACGATCAAGGCG AGTTACATGATCCCCCATGTTGTGCAAAAAAGCGGTTA GCTCCTTCGGTCCTCCGATCGTTGTCAGAAGTAAGTTG GCCGCAGTGTTATCACTCATGGTTATGGCAGCACTGC ATAATTCTCTTACTGTCATGCCATCCGTAAGATGCTTTT CTGTGACTGGTGAGTACTCAACCAAGTCATTCTGAGAA TAGTGTATGCGGCGACCGAGTTGCTCTTGCCCGGCGT CAATACGGGATAATACCGCGCCACATAGCAGAACTTTA AAAGTGCTCATCATTGGAAAACGTTCTTCGGGGCGAAA ACTCTCAAGGATCTTACCGCTGTTGAGATCCAGTTCGA TGTAACCCACTCGTGCACCCAACTGATCTTCAGCATCT TTTACTTTCACCAGCGTTTCTGGGTGAGCAAAAACAGG AAGGCAAAATGCCGCAAAAAAGGGAATAAGGGCGACA CGGAAATGTTGAATACTCATACTCTTCCTTTTTCAATAT TATTGAAGCATTTATCAGGGTTATTGTCTCATGAGCGG ATACATATTTGAATGTATTTAGAAAAATAAACAAATAGG GGTTCCGCGCACATTTCCCCGAAAAGTG | |

(continued)

| SEQ ID NO. | Nucleic Acid Sequence | Description |
|---|---|---|
| SEQ ID NO 12 | TACCACCTAAATTGTAAGCGTTAATATTTTGTTAAAATT CGCGTTAAATTTTTGTTAAATCAGCTCATTTTTTAACCA ATAGGCCGAAATCGGCAAAATCCCTTATAAATCAAAAG AATAGACCGAGATAGGGTTGAGTGTTGTTCCAGTTTGG AACAAGAGTCCACTATTAAAGAACGTGGACTCCAACGT CAAAGGGCGAAAAACCGTCTATCAGGGCGATGGCCCA CTACGTGAACCATCACCCTAATCAAGTTTTTTGGGGTC GAGGTGCCGTAAAGCACTAAATCGGAACCCTAAAGGG AGCCCCCGATTTAGAGCTTGACGGGGAAAGCCGGCG AACGTGGCGAGAAAGGAAGGGAAGAAAGCGAAAGGA GCGGGCGCTAGGGCGCTGGCAAGTGTAGCGGTCACG CTGCGCGTAACCACCACACCCGCCGCGCTTAATGCGC CGCTACAGGGCGCGTCCCATTCGCCATTCAGGCTGCG CAACTGTTGGGAAGGGCGATCGGTGCGGGCCTCTTCG CTATTACGCCAGCTGGCGAAAGGGGGATGTGCTGCAA GGCGATTAAGTTGGGTAACGCCAGGGTTTTCCCAGTC ACGACGTTGTAAAACGACGGCCAGTGAGCGCGCGTAA TACGACTCACTATAGGGCGAATTGGGTACCAAATAGTA GGAATTACCCACCTGTACAAGTGCTGAAAACTTGGATG AATAAGCCCGTTTGCCATTTTCTACTGCTATTTTAAATC TTTTCTGTATTTGTCTGCATTTGTCTTTACCCTTGAATA AATGTTTTAGTTGTTTTTTTTTCAATTATGACTGTGTTTAA CAGACATTATTACAATAATATGTAATAGTAGTACACACT ATTATTATGTAATAGTACTTGTTGACTGTATTTGAGAAC TGGACCGAGTGAGTGTTACGTCACCCATTCAAAATGAC TTACTTCTGGCTCCAACAAAATGAAGTTAATTCAGTTG CCATTTTTCACTGTATGGACATCGCCGTGTTGGAGCTA GACGTCGCCAATAAGCAAGAAAGAGCCGAGATGCGTC GAGTCTGAGTCACCGTTCCTATGGCAACCCCTCTAACC | Plasmid Sequenz of pDestTol2_UAS: EGFP -Krit1,alpha-cry:EGFP used in the examples of the invention |

(continued)

| SEQ ID NO. | Nucleic Acid Sequence | Description |
|---|---|---|
| | AATCAGAAGTAAGCTTGTTGGAAGTCCACAGCCTACCA<br>CTTGAAAGCGGGCTGCACAAAATCTGTCAAACGTTTTG<br>AACGTTGGATGTGAGAGCACATACTTTTATTAAGGCAT<br>CTGGTTGGTCAGTTTATAATATCAACAACTTGGGCTAC<br>AGAAAAGAAAAGTTATTACAGAAATTATGATTAACAAGT<br>ACATGTTAAATAAAGATTTTAATATGAATGCCACCACTG<br>GAGCATTCATGCCATTTGGAGCTTCTTCCTGTTTGGAT<br>CACTAGAAGGAGGAGGTCACTCATTACAGTTCTCATAT<br>ACAGTCGTTGGTTGGTTGGTTGGTTGGTTGGTTGGTA<br>GATTGATTGATTGATTGATTGATTGATTGATTGATTGAT<br>TGATTGATTGATTGATTGATTGATTGATTGATTGATTGA<br>TTGATTGATTGATTGATTGATTGGTAGTCAAAATA<br>AGAAATAATTTCCACAGATTCATTACAGAAATGATTAAA<br>TGCATACATAAAAAACTGGGGGGGGGGGGGGATACAACA<br>ACACACTTAAGTCACATTTGCCTACGTAAAGAAAAGTA<br>AAGAAAATCAATAGCTATATTTTACATCTCCTTTTTTTG<br>CTGTCTTTTAATTAGCCTTGTTTTGCTGTTATCTTGATT<br>GAAACTGTAACTTCTTCACCTGCTTCTTTTCTTTGTAGG<br>TTTTGCCAGCCCAGAAACAGGCATGGCTGTGCAAGGC<br>CAGAGCACCATGCACAATGCGCTGCATGTCTTCATGA<br>ACGGCTCAATGTCCTCAGTCCAGGGCTCAGCCAACGA<br>CCCCATCTTCCTCCTTCACCATGCTTTCATTGACAGGT<br>AACAAACACGTCATGACATTAGACTGCACAGTTTTTGA<br>CAAAGTTCATACAATCTGTTGTTTATAGCTGCTACAATT<br>AGTGAAGTTTGTGAATGTACTTGGATGAGCAGCGAAA<br>GATCAATTGAGATCAATTGTTAGAGTTTGGTTGCCCTG<br>CAGAGCAAAGAACAAAAAATAATCTGGTGGCTTTACTG<br>CGTGAGGTTATTATTGGTGGAATAGAAACACAAAACAT<br>AATTGCATTTATTTGTTTAATTTTTTATCTTATCTTAACTT<br>TCATCTTGCATATTTGTTTCTTACATCATTTCTAGCATCT<br>TTGAGCGCTGGCTAAGAACTCATCAGCCTCCCCGGTC<br>CATCTACCCACGTACCAATGCACCAATTGGCCACAATG<br>ACGGCTACTACATGGTGCCATTCCTTCCTCTTTATAGG<br>AATGGAGACTACCTCCTGTCCAACAAGGCTCTTGGATA<br>CGAGTACGCCTACCTGTTGGACCCAGGTCATTGCACA<br>ACACCAGAAATGCCCTCTGATCTGCAAAAGACGTGAAT<br>ATCTGTTCAGACACCCATATCCACTCTGTTCCACACAG<br>GTCAGAGGTTTGTCCAGGAGTTCTTGACAGAGGTGTA<br>AAAAGTACTCAAAAATTTTACTCAAGTGAAAGTACAAGT<br>ACTTAGGGAAAATTTTACTCAATTAAAAGTAAAAGTATC<br>TGGCTAGAATCTTACTTGAGTAAAAGTAAAAAAGTACT<br>CCATTAAAATTGTACTTGAGTATTAAGGAAGTAAAAGTA<br>AAAGCAAGAAAGAAAACTAGAGATTCTTGTTTAAGCTT<br>TTAATCTCAAAAAAACATTAAATGAAATGCATACAAGGTT<br>TTATCCTGCTTTAGAACTGTTTGTATTTAATTATCAAACT<br>ATAAGACAGACAATCTAATGCCAGTACACGCTACTCAA<br>AGTTGTAAAACCTCAGATTTAACTTCAGTAGAAGCTGA<br>TTCTCAAAATTGTTAGTGTCAAGCCTAGCTCTTTTGGG | |

(continued)

| SEQ ID NO. | Nucleic Acid Sequence | Description |
|---|---|---|
| | GCTGAAAAGCAATCCTGCAGTGCTGAAAAGCCTCTCA CAGGCAGCCGATGCGGGAAGAGGTGTATTAGTCTTGA TAGAGAGGCTGCAAATAGCAGGAAACGTGAGCAGAGA CTCCCTGGTGTCTGAAACACAGGCCAGATGGGCCCTC GAGCAGGAAACAGCTATGACCATGATTACGCCAAGCT ATCAACTTTGTATAGAAAAGTTGGTAATACGACTCACTA TAGGGCGAATTGGGTACCGGGCCCCCCCTCGAGGTC GACGGTATCGATAAGCTTAGGCCTCCAAGGCGGAGTA CTGTCCTCCGGGCTGGCGGAGTACTGTCCTCCGGCAA GGTCGGAGTACTGTCCTCCGACACTAGAGGTCGGAGT ACTGTCCTCCGACGCAAGGCGGAGTACTGTCCTCCGG GCTGGCGGAGTACTGTCCTCCGGCAAGGTCGGAGTA CTGTCCTCCGACACTAGAGGTCGGAGTACTGTCCTCC GACGCAAGGCGGAGTACTGTCCTCCGGGCTGGCGGA GTACTGTCCTCCGGCAAGGGTCGACTCTAGAGGGTAT ATAATGGATCCCATCGCGTCTCAGCCTCACTTTGAGCT CCTCCACACGAATTCCTGCAGCCCGGGGGATCCACTA GTTCTAGAGCGGCCGCCACCGCGGTGGAGCTCCAGC TTTTGTTCCCTTTAGTGAGGGTTAATTCAAGTTTGTACA AAAAAGCAGGCTATGGTGAGCAAGGGCGAGGAGCTGT TCACCGGGGTGGTGCCCATCCTGGTCGAGCTGGACG GCGACGTAAACGGCCACAAGTTCAGCGTGTCCGGCGA GGGCGAGGGCGATGCCACCTACGGCAAGCTGACCCT GAAGTTCATCTGCACCACCGGCAAGCTGCCCGTGCCC TGGCCCACCCTCGTGACCACCCTGACCTACGGCGTGC AGTGCTTCAGCCGCTACCCCGACCACATGAAGCAGCA CGACTTCTTCAAGTCCGCCATGCCCGAAGGCTACGTC CAGGAGCGCACCATCTTCTTCAAGGACGACGGCAACT ACAAGACCCGCGCCGAGGTGAAGTTCGAGGGCGACA CCCTGGTGAACCGCATCGAGCTGAAGGGCATCGACTT CAAGGAGGACGGCAACATCCTGGGGCACAAGCTGGA GTACAACTACAACAGCCACAACGTCTATATCATGGCCG ACAAGCAGAAGAACGGCATCAAGGTGAACTTCAAGAT CCGCCACAACATCGAGGACGGCAGCGTGCAGCTCGC CGACCACTACCAGCAGAACACCCCCATCGGCGACGG CCCCGTGCTGCTGCCCGACAACCACTACCTGAGCACC CAGTCCGCCCTGAGCAAAGACCCCAACGAGAAGCGC GATCACATGGTCCTGCTGGAGTTCGTGACCGCCGCCG GGATCACTCTCGGCATGGACGAGCTGTACAAGGGAGG CGCTGCAGGAGGCTCTAGAATGGGAAACCAAGAGCTA GAGGAGGTGTTTGTTGCTGTTATCCGACCAAAAAACAC AACCAGCCTCAACTCCAAAGAATACCGTGCTAAATCCT ATGAGATTTTACTTATCGAAGTTCCCCTGGAGGGAAAA GAGAAAAAGCGGAAGAAAGTGCTGCTTGGGACTAAAA TtCACGCCGACAGTGACCGGACAAAGTCAATTCTGGAG TTTGTGGATGAAACCACCAAGCCCATATCCAATAACCA GGGGCATAATAGGGAAGCGTGTtGTGCACATGAGGAAG TTCCTGTTAGATGGAGACAGTGGAGGAAAAGAGGCCT | |

(continued)

| SEQ ID NO. | Nucleic Acid Sequence | Description |
|---|---|---|
| | CGTTATTTATTGTGCCAATTAATGTCAAAGACAACAGC AAGTCCGTGCATCACCCTGGGAGCCCCAGTTTCTACT GCCTTCAGGACATCATGCGTGTGTGTAGTGAAACCAG TGCTCATTTCTCCTCCAGCACCTCAAAGATGCTGCTCG CTCTCGACAAGTGGTTAGCGGAGCAGCACACTGTGCC TCACGCTATTCCTGCTCTGTTCCGGCCGGCTCCGGTG GACCGTGTGAAAACTAACGTGAGTAACCCGGCGTACG CAGTGGAGAAGCAGACAGACGGGACGCTGCACATGG GCTACACGGCTCTGGAGATCAAGAGTAAACTGATGTC TCTGGAGAAAGCAGATCTGTGCATCCAGAACCCGCTG TACGGCTCAGATCTGCAGTACACCAACAGAGTGGACA AAGTCATCATCAACCCTTACTTTGGCTTGGGAGCTCCA GATTACTCCAAAATCCAGATTCCAAAGAGAGACAAATG GCAGCACAGCATGACCAGCGTCACCGAAGACAAAGAG CGCCAGTGGGTGGATGATTTCCCTCTGCACCGAAGCG CCTGTGAGGGAGACACTGAGCTTTTGTCTAAATTGCTG GATGGCGGATTCTCAGTCAAACAGCTGGACAGCGACC ATTGGGCACCCATTCATTATGCATGCTGGCATGGGAA GGTGGAGGCCACTAAACTGCTGCTGGAGAAAGGCAAC TGTAACCCCAACCTGCTGAACGGCCAGCTGAGCTCTC CTTTACACTTCGCAGCTATCGGCGGTCATGCAGAAATC GTGCAGCTCCTCCTGCAGCACCCAGAGATCGACAGAC ACATCGAGGACCAGCAGAAACGATCGCCGCTTCAAGT ATGTGAAGAAAACAAGCAGAACAACTGGGAGGAAACC GTGAATCTCCTTCAACAAGCCAGCAATAAACCATACGA GAAGGTGAGGATTTACCGTATGGATGGATCGTACCGC TCTGTGGAGCTGAAGCATGGGAACAACACCACCGTGC AGCAGATCATGGAGGGCATGAGATTATCACAGGAGAC CCAGCAGTATTTTACCATCTGGATCTGCTCCGAAAACC TTAGTCTGCAGCTGAAGCCGTACCACAAGCCCTTGCA GCACTTACGGATGTGGAGCGAGATCGTGACGGATCTC ACTGCTCTGGATCCACAGAGAGAAAGTCCACAGCTCT TCCTGCGCAGAGACGTTCGGCTGCCTTTAGAGGTCGA GAAAAAGGTTGAAGATCCGCTGTCCATCCTCATCCTAT TTGATGAAGCTCGTCACTGCCTCCTTAAAGGTTTCCTC TCGACGTCAGATAACAAGCTAATCACGCTCGCAAGCC TCCTCCTGCAGATCATCTACGGAAACTACGACAGCAA GAAGCACAAACAGGGCTTTCTAAATGAGGAAAACCTG AAATCCATAGTTCCCATTTCAAAGGTCAAGAGCAAAGC ACATCACTGGACTAACAGGATACTTCACGAGTACAAGA GTCTGAGCACTAGTGAGGGTGTGAGTAAAGAAATGCA TCACCTCCAGAGGCTCTTCCTGCAGAACTGCTGGGAT ATTCCTACTTATGGTGCAGCGTTTTTCACAGGACAGGT CTTCACTAAAGCCAGCTCCAGCACACACAAGGTGATC CGCGTGTATGTGGGCGTCAACACCAAAGGCCTGCATC TGATGAACATGGAGACAAAAGTGCTTCACCTCAGTCTG GAGTATGGGACATTTATGTGGCAGCTGGGTCAGGCTG ATCAATACGTGCAAATCCACAGTCTGGAGAACAAGAAG | |

| SEQ ID NO. | Nucleic Acid Sequence | Description |
|---|---|---|
| | AACTTTGTCGTTCACACCAAACAGGCTGGTCTTATCGT GAAGCTGCTGATGAAGCTGAGTGGACAAATTGCACCA AACGACAGAGCTGTGTCTGATAAATATGCGTATGGGTA AACCCAGCTTTCTTGTACAAAGTGGAATTCACTAGTGA TTTAATAGTGTGCATTCAGTGCAGGATGTTATGTATTTA TTTTCTGTAGTGCAAACTTTTTCAAAGAATTTTCCAGTC TAAGCTTTTCAGTCAAGAAAAAAAAGGCGTCTGATTA TCTGTGATATTTTAAATAATTAGCTCTTAAATCTGGTGA ACCAAGTGGCCACAACACATTAAATACTGACGTTCTAC AGCCATGTAGCTTCATCTGGTCTGGTTTGTTTTGGCAG GCACTTATTTTCCGGTGATAGTTGGCCACAAGAGTATT GTCTGAGCCATTCAGTGCTAGACTGTCATTCTCAGGTC AGAATCCACCCCACTGATTCTGCTGACAACACTGTTCC CACCATGAGATAATGCCATTCCAGAGAGATCCATTTGT AAGCCCCTCTTTCTGCAGCACAGGTATATAACCAGGG CTCTGCCTCCACTAAGGCCGGCACACATCATTTGGGG ATCTTTGTACTGTACTAGCCTCTATTAGCCTCCTACTTG CGTTCAGTTCACATCTTTTGTTCATCAGGTGCAATAAGT TACAGTAAGGAGTTACCAGGTCTGACATTAATCGAATT CCACCATGGTGAGCAAGGGCGAGGAGCTGTTCACCG GGGTGGTGCCCATCCTGGTCGAGCTGGACGGCGACG TAAACGGCCACAAGTTCAGCGTGTCCGGCGAGGGCG AGGGCGATGCCACCTACGGCAAGCTGACCCTGAAGTT CATCTGCACCACCGGCAAGCTGCCCGTGCCCTGGCCC ACCCTCGTGACCACCCTGACCTACGGCGTGCAGTGCT TCAGCCGCTACCCCGACCACATGAAGCAGCACGACTT CTTCAAGTCCGCCATGCCCGAAGGCTACGTCCAGGAG CGCACCATCTTCTTCAAGGACGACGGCAACTACAAGA CCCGCGCCGAGGTGAAGTTCGAGGGCGACACCCTGG TGAACCGCATCGAGCTGAAGGGCATCGACTTCAAGGA GGACGGCAACATCCTGGGGCACAAGCTGGAGTACAAC TACAACAGCCACAACGTCTATATCATGGCCGACAAGCA GAAGAACGGCATCAAGGTGAACTTCAAGATCCGCCAC AACATCGAGGACGGCAGCGTGCAGCTCGCCGACCAC TACCAGCAGAACACCCCCATCGGCGACGGCCCCGTG CTGCTGCCCGACAACCACTACCTGAGCACCCAGTCCG CCCTGAGCAAAGACCCCAACGAGAAGCGCGATCACAT GGTCCTGCTGGAGTTCGTGACCGCCGCCGGGATCACT CTCGGCATGGACGAGCTGTACAAGGGCGGTGGAAGA TCTGGGAATTCAAGGCCTCTCGAGCCTCTAGATTCTGC AGCCCTATAGTGAGTCGTATTACGTAGATCCAGACATG ATAAGATACATTGATGAGTTTGGACAAACCACAACTAG AATGCAGTGAAAAAAATGCTTTATTTGTGAAATTTGTGA TGCTATTGCTTTATTTGTAACCATTATAAGCTGCAATAA ACAAGTTAACAACAACAATTGCATTCATTTTATGTTTCA GGTTCAGGGGGAGGTGTGGGAGGTTTTTTCCAACTTT ATTATACATAGTTGATAATTCACTGGCCGTCGTTTTACA TCGATGATGATCCAGACATGATAAGATACATTGATGAG | |

(continued)

| SEQ ID NO. | Nucleic Acid Sequence | Description |
|---|---|---|
| | TTTGGACAAACCACAACTAGAATGCAGTGAAAAAAATG<br>CTTTATTTGTGAAATTTGTGATGCTATTGCTTTATTTGTA<br>ACCATTATAAGCTGCAATAAACAAGTTAACAACAACAAT<br>TGCATTCATTTTATGTTTCAGGTTCAGGGGGAGGTGTG<br>GGAGGTTTTTTAAAGCAAGTAAAACCTCTACAAATGTG<br>GTATGGCTGATTATGATCCTCTAGATCAGATCTGCGAA<br>GATACGGCCACGGGTGCTCTTGATCCTGTGGCTGATT<br>TTGGACTGTGCTGCTCGCAGCTGCTGATGAATCACATA<br>CTTCCTCCATTTTCTTCCACTGATTGACTGTTATAATTT<br>CCCTAATTTCCAGGTCAAGGTGCTGTGCATTGTGGTAA<br>TAGATGTGACATGACGTCACTTCCAAAGGACCAATGAA<br>CATGTCTGACCAATTTCATATAATGTGAAAACGATTTTC<br>ATAGGCAGAATAAATAACATTTAAATTAAACTGGGCAT<br>CAGCGCAATTCAATTGGTTTGGTAATAGCAAGGGAAAA<br>TAGAATGAAGTGATCTCCAAAAAATAAGTACTTTTTGAC<br>TGTAAATAAAATTGTAAGGAGTAAAAAGTACTTTTTTTT<br>CTAAAAAAATGTAATTAAGTAAAAGTAAAAGTATTGATT<br>TTTAATTGTACTCAAGTAAAGTAAAAATCCCCAAAAATA<br>ATACTTAAGTACAGTAATCAAGTAAAATTACTCAAGTAC<br>TTTACACCTCTGGTTCTTGACCCCCTACCTTCAGCAAG<br>CCCAGCAGATCCACTAGTTCTAGAGCGGCCGCCACCG<br>CGGTGGAGCTCCAGCTTTTGTTCCCTTTAGTGAGGGTT<br>AATTGCGCGCTTGGCGTAATCATGGTCATAGCTGTTTC<br>CTGTGTGAAATTGTTATCCGCTCACAATTCCACACAAC<br>ATACGAGCCGGAAGCATAAAGTGTAAAGCCTGGGGTG<br>CCTAATGAGTGAGCTAACTCACATTAATTGCGTTGCGC<br>TCACTGCCCGCTTTCCAGTCGGGAAACCTGTCGTGCC<br>AGCTGCATTAATGAATCGGCCAACGCGCGGGGAGAG<br>GCGGTTTGCGTATTGGGCGCTCTTCCGCTTCCTCGCT<br>CACTGACTCGCTGCGCTCGGTCGTTCGGCTGCGGCGA<br>GCGGTATCAGCTCACTCAAAGGCGGTAATACGGTTAT<br>CCACAGAATCAGGGGATAACGCAGGAAAGAACATGTG<br>AGCAAAAGGCCAGCAAAAGGCCAGGAACCGTAAAAAG<br>GCCGCGTTGCTGGCGTTTTTCCATAGGCTCCGCCCCC<br>CTGACGAGCATCACAAAAATCGACGCTCAAGTCAGAG<br>GTGGCGAAACCCGACAGGACTATAAAGATACCAGGCG<br>TTTCCCCCTGGAAGCTCCCTCGTGCGCTCTCCTGTTCC<br>GACCCTGCCGCTTACCGGATACCTGTCCGCCTTTCTC<br>CCTTCGGGAAGCGTGGCGCTTTCTCATAGCTCACGCT<br>GTAGGTATCTCAGTTCGGTGTAGGTCGTTCGCTCCAA<br>GCTGGGCTGTGTGCACGAACCCCCCGTTCAGCCCGAC<br>CGCTGCGCCTTATCCGGTAACTATCGTCTTGAGTCCAA<br>CCCGGTAAGACACGACTTATCGCCACTGGCAGCAGCC<br>ACTGGTAACAGGATTAGCAGAGCGAGGTATGTAGGCG<br>GTGCTACAGAGTTCTTGAAGTGGTGGCCTAACTACGG<br>CTACACTAGAAGGACAGTATTTGGTATCTGCGCTCTGC<br>TGAAGCCAGTTACCTTCGGAAAAAGAGTTGGTAGCTCT<br>TGATCCGGCAAACAAACCACCGCTGGTAGCGGTGGTT | |

(continued)

| SEQ ID NO. | Nucleic Acid Sequence | Description |
|---|---|---|
| | TTTTTGTTTGCAAGCAGCAGATTACGCGCAGAAAAAAA GGATCTCAAGAAGATCCTTTGATCTTTTCTACGGGGTC TGACGCTCAGTGGAACGAAAACTCACGTTAAGGGATTT TGGTCATGAGATTATCAAAAAGGATCTTCACCTAGATC CTTTTAAATTAAAAATGAAGTTTTAAATCAATCTAAAGTA TATATGAGTAAACTTGGTCTGACAGTTACCAATGCTTA ATCAGTGAGGCACCTATCTCAGCGATCTGTCTATTTCG TTCATCCATAGTTGCCTGACTCCCCGTCGTGTAGATAA CTACGATACGGGAGGGCTTACCATCTGGCCCCAGTGC TGCAATGATACCGCGAGACCCACGCTCACCGGCTCCA GATTTATCAGCAATAAACCAGCCAGCCGGAAGGGCCG AGCGCAGAAGTGGTCCTGCAACTTTATCCGCCTCCAT CCAGTCTATTAATTGTTGCCGGGAAGCTAGAGTAAGTA GTTCGCCAGTTAATAGTTTGCGCAACGTTGTTGCCATT GCTACAGGCATCGTGGTGTCACGCTCGTCGTTTGGTA TGGCTTCATTCAGCTCCGGTTCCCAACGATCAAGGCG AGTTACATGATCCCCCATGTTGTGCAAAAAAGCGGTTA GCTCCTTCGGTCCTCCGATCGTTGTCAGAAGTAAGTTG GCCGCAGTGTTATCACTCATGGTTATGGCAGCACTGC ATAATTCTCTTACTGTCATGCCATCCGTAAGATGCTTTT CTGTGACTGGTGAGTACTCAACCAAGTCATTCTGAGAA TAGTGTATGCGGCGACCGAGTTGCTCTTGCCCGGCGT CAATACGGGATAATACCGCGCCACATAGCAGAACTTTA AAAGTGCTCATCATTGGAAAACGTTCTTCGGGGCGAAA ACTCTCAAGGATCTTACCGCTGTTGAGATCCAGTTCGA TGTAACCCACTCGTGCACCCAACTGATCTTCAGCATCT TTTACTTTCACCAGCGTTTCTGGGTGAGCAAAAACAGG AAGGCAAAATGCCGCAAAAAAGGGAATAAGGGCGACA CGGAAATGTTGAATACTCATACTCTTCCTTTTTCAATAT TATTGAAGCATTTATCAGGGTTATTGTCTCATGAGCGG ATACATATTTGAATGTATTTAGAAAAATAAACAAATAGG GGTTCCGCGCACATTTCCCCGAAAAGTG | |

(continued)

| SEQ ID NO. | Nucleic Acid Sequence | Description |
|---|---|---|
| SEQ ID NO 13 | GTAATACGACTCACTATAGGGCGAATTGGGTACCGGG CCCCCCCTCGAGGTCGACGGTATCGATAAGCTTAGGC CTCCAAGGCGGAGTACTGTCCTCCGGGCTGGCGGAG TACTGTCCTCCGGCAAGGTCGGAGTACTGTCCTCCGA CACTAGAGGTCGGAGTACTGTCCTCCGACGCAAGGCG GAGTACTGTCCTCCGGGCTGGCGGAGTACTGTCCTCC GGCAAGGTCGGAGTACTGTCCTCCGACACTAGAGGTC GGAGTACTGTCCTCCGACGCAAGGCGGAGTACTGTCC TCCGGGCTGGCGGAGTACTGTCCTCCGGCAAGGGTC GACTCTAGAGGGTATATAATGGATCCCATCGCGTCTCA GCCTCACTTTGAGCTCCTCCACACGAATTCCTGCAGCC CGGGGGATCCACTAGTTCTAGAGCGGCCGCCACCGC GGTGGAGCTCCAGCTTTTGTTCCCTTTAGTGAGGGTTA ATT | UAS promoter sequence, which can be used in a endocardium-specific genetic system of the invention |

(continued)

| SEQ ID NO. | Nucleic Acid Sequence | Description |
|---|---|---|
| SEQ ID NO 14 | ATGGTGAGCAAGGGCGAGGAcGACAACATGGCCATCA TCAAGGAGTTCATGCGCTTCAAGGTGCACATGGAGGG CTCCGTGAACGGCCACGAGTTCGAGATCGAGGGCGA GGGCGAGGGCCGCCCCTACGAGGGCACCCAGACCGC CAAGCTGAAGGTGACCAAGGGCGGCCCCCTGCCCTT CGCCTGGGACATCCTGTCCCCTCAGTTCATGTACGGC TCCAAGGCCTACGTGAAGCACCCCGCCGACATCCCCG ACTACTTGAAGCTGTCCTTCCCCGAGGGCTTCAAGTG GGAGCGCGTGATGAACTTCGAGGACGGCGGCGTGGT GACCGTGACCCAGGACTCCTCCCTGCAGGACGGCGA GTTCATCTACAAGGTGAAGCTGCGCGGCACCAACTTC CCCTCCGACGGCCCCGTAATGCAGAAGAAGACCATGG GCTGGGAGGCCTCCTCCGAGCGGATGTACCCCGAGG ACGGCGCCCTGAAGGGCGAGATCAAGCAGAGGCTGA AGCTGAAGGACGGCGGCCACTACGACGCCGAGGTCA AGACCACCTACAAGGCCAAGAAGCCCGTGCAGCTGCC CGGCGCCTACAACGTCAACATCAAGCTGGACATCACC TCCCACAACGAGGACTACACCATCGTGGAACAGTACG AGCGCGCCGAGGGCCGCCACTCCACCGGCGGCATGG ACGAGCTGTACAAGGGAGGCGCTGCAGGAGGCTCTA GAATGGGAAACCAAGAGCTAGAGGAGGTGTTTGTTGC TGTTATCCGACCAAAAAACACAACCAGCCTCAACTCCA AAGAATACCGTGCTAAATCCTATGAGATTTTACTTATCG AAGTTCCCCTGGAGGGAAAAGAGAAAAAGCGGAAGAA AGTGCTGCTTGGGACTAAAAATtCACGCCGACAGTGACC GGACAAAGTCAATTCTGGAGTTTGTGGATGAAACCACC AAGCCCATATCCAATAACCAGGGCATAATAGGGAAGC GTGTtGTGCACATGAGGAAGTTCCTGTTAGATGGAGAC AGTGGAGGAAAAGAGGCCTCGTTATTTATTGTGCCAAT TAATGTCAAAGACAACAGCAAGTCCGTGCATCACCCTG GGAGCCCCAGTTTCTACTGCCTTCAGGACATCATGCG TGTGTGTAGTGAAACCAGTGCTCATTTCTCCTCCAGCA CCTCAAAGATGCTGCTCGCTCTCGACAAGTGGTTAGC GGAGCAGCACACTGTGCCTCACGCTATTCCTGCTCTG TTCCGGCCGGCTCCGGTGGACCGTGTGAAAACTAACG TGAGTAACCCGGCGTACGCAGTGGAGAAGCAGACAGA CGGGACGCTGCACATGGGCTACACGGCTCTGGAGAT CAAGAGTAAACTGATGTCTCTGGAGAAAGCAGATCTGT GCATCCAGAACCCGCTGTACGGCTCAGATCTGCAGTA CACCAACAGAGTGGACAAAGTCATCATCAACCCTTACT TTGGCTTGGGAGCTCCAGATTACTCCAAAATCCAGATT CCAAAGAGAGACAAATGGCAGCACAGCATGACCAGCG TCACCGAAGACAAAGAGCGCCAGTGGGTGGATGATTT CCCTCTGCACCGAAGCGCCTGTGAGGGAGACACTGA GCTTTTGTCTAAATTGCTGGATGGCGGATTCTCAGTCA AACAGCTGGACAGCGACCATTGGGCACCCATTCATTA TGCATGCTGGCATGGGAAGGTGGAGGCCACTAAACTG | Coding nucleotide sequence of an mCherry-KRIT1 fusion protein including a linker sequence, which can be used as a functional Krit1/CCM1 protein in the context of the invention. |

(continued)

| SEQ ID NO. | Nucleic Acid Sequence | Description |
|---|---|---|
| | CTGCTGGAGAAAGGCAACTGTAACCCCAACCTGCTGA ACGGCCAGCTGAGCTCTCCTTTACACTTCGCAGCTATC GGCGGTCATGCAGAAATCGTGCAGCTCCTCCTGCAGC ACCCAGAGATCGACAGACACATCGAGGACCAGCAGAA ACGATCGCCGCTTCAAGTATGTGAAGAAAACAAGCAG AACAACTGGGAGGAAACCGTGAATCTCCTTCAACAAG CCAGCAATAAACCATCGAGAAGGTGAGGATTTACCG TATGGATGGATCGTACCGCTCTGTGGAGCTGAAGCAT GGGAACAACACCACCGTGCAGCAGATCATGGAGGGC ATGAGATTATCACAGGAGACCCAGCAGTATTTTACCAT CTGGATCTGCTCCGAAAACCTTAGTCTGCAGCTGAAG CCGTACCACAAGCCCTTGCAGCACTTACGGATGTGGA GCGAGATCGTGACGGATCTCACTGCTCTGGATCCACA GAGAGAAAGTCCACAGCTCTTCCTGCGCAGAGACGTT CGGCTGCCTTTAGAGGTCGAGAAAAAGGTTGAAGATC CGCTGTCCATCCTCATCCTATTTGATGAAGCTCGTCAC TGCCTCCTTAAAGGTTTCCTCTCGACGTCAGATAACAA GCTAATCACGCTCGCAAGCCTCCTCCTGCAGATCATCT ACGGAAACTACGACAGCAAGAAGCACAAACAGGGCTT TCTAAATGAGGAAAACCTGAAATCCATAGTTCCCATTT CAAAGGTCAAGAGCAAAGCACATCACTGGACTAACAG GATACTTCACGAGTACAAGAGTCTGAGCACTAGTGAG GGTGTGAGTAAAGAAATGCATCACCTCCAGAGGCTCT TCCTGCAGAACTGCTGGGATATTCCTACTTATGGTGCA GCGTTTTTCACAGGACAGGTCTTCACTAAAGCCAGCTC CAGCACACACAAGGTGATCCGCGTGTATGTGGGCGTC AACACCAAAGGCCTGCATCTGATGAACATGGAGACAA AAGTGCTTCACCTCAGTCTGGAGTATGGGACATTTATG TGGCAGCTGGGTCAGGCTGATCAATACGTGCAAATCC ACAGTCTGGAGAACAAGAAGAACTTTGTCGTTCACACC AAACAGGCTGGTCTTATCGTGAAGCTGCTGATGAAGC TGAGTGGACAAATTGCACCAAACGACAGAGCTGTGTC TGATAAATATGCGTATGGGTAA | |

34

(continued)

| SEQ ID NO. | Nucleic Acid Sequence | Description |
|---|---|---|
| SEQ ID NO 15 | ATGGTGAGCAAGGGCGAGGAcGACAACATGGCCATCA TCAAGGAGTTCATGCGCTTCAAGGTGCACATGGAGGG CTCCGTGAACGGCCACGAGTTCGAGATCGAGGGCGA GGGCGAGGGCCGCCCCTACGAGGGCACCCAGACCGC CAAGCTGAAGGTGACCAAGGGCGGCCCCCTGCCCTT CGCCTGGGACATCCTGTCCCCTCAGTTCATGTACGGC TCCAAGGCCTACGTGAAGCACCCCGCCGACATCCCCG ACTACTTGAAGCTGTCCTTCCCCGAGGGCTTCAAGTG GGAGCGCGTGATGAACTTCGAGGACGGCGGCGTGGT GACCGTGACCCAGGACTCCTCCCTGCAGGACGGCGA GTTCATCTACAAGGTGAAGCTGCGCGGCACCAACTTC CCCTCCGACGGCCCCGTAATGCAGAAGAAGACCATGG GCTGGGAGGCCTCCTCCGAGCGGATGTACCCCGAGG ACGGCGCCCTGAAGGGCGAGATCAAGCAGAGGCTGA AGCTGAAGGACGGCGGCCACTACGACGCCGAGGTCA AGACCACCTACAAGGCCAAGAAGCCCGTGCAGCTGCC CGGCGCCTACAACGTCAACATCAAGCTGGACATCACC TCCCACAACGAGGACTACACCATCGTGGAACAGTACG AGCGCGCCGAGGGCCGCCACTCCACCGGCGGCATGG ACGAGCTGTACAAG | Coding nucleotide sequence of an mCherry that can be used for encoding a fusion protein of the invention. |
| SEQ ID NO 16 | GGAGGCGCTGCAGGAGGCTCTAGA | Coding nucleotide sequence of a linker that can be used for encoding a linker of a fusion protein of the invention. |
| SEQ ID NO 17 | AATTCACTAGTGATTTAATAGTGTGCATTCAGTGCAGG ATGTTATGTATTTATTTTCTGTAGTGCAAACTTTTTCAAA GAATTTTCCAGTCTAAGCTTTTCAGTCAAGAAAAAAAA GGCGTCTGATTATCTGTGATATTTTAAATAATTAGCTCT TAAATCTGGTGAACCAAGTGGCCACAACACATTAAATA CTGACGTTCTACAGCCATGTAGCTTCATCTGGTCTGGT TTGTTTTGGCAGGCACTTATTTTCCGGTGATAGTTGGC CACAAGAGTATTGTCTGAGCCATTCAGTGCTAGACTGT CATTCTCAGGTCAGAATCCACCCCACTGATTCTGCTGA CAACACTGTTCCCACCATGAGATAATGCCATTCCAGAG AGATCCATTTGTAAGCCCCTCTTTCTGCAGCACAGGTA TATAACCAGGGCTCTGCCTCCACTAAGGCCGGCACAC ATCATTTGGGGATCTTTGTACTGTACTAGCCTCTATTAG CCTCCTACTTGCGTTCAGTTCACATCTTTTGTTCATCAG GTGCAATAAGTTACAGTAAGGAGTTACCAGGTCTGACA TTAATCGAATT | Nucleotide sequence of an alpha-crystallin promoter that can be used for an exogenous nucleic acid construct of the invention. |

(continued)

| SEQ ID NO. | Nucleic Acid Sequence | Description |
|---|---|---|
| SEQ ID NO 18 | ATGGTGAGCAAGGGCGAGGAGCTGTTCACCGGGGTG GTGCCCATCCTGGTCGAGCTGGACGGCGACGTAAAC GGCCACAAGTTCAGCGTGTCCGGCGAGGGCGAGGGC GATGCCACCTACGGCAAGCTGACCCTGAAGTTCATCT GCACCACCGGCAAGCTGCCCGTGCCCTGGCCCACCC TCGTGACCACCCTGACCTACGGCGTGCAGTGCTTCAG CCGCTACCCCGACCACATGAAGCAGCACGACTTCTTC AAGTCCGCCATGCCCGAAGGCTACGTCCAGGAGCGC ACCATCTTCTTCAAGGACGACGGCAACTACAAGACCC GCGCCGAGGTGAAGTTCGAGGGCGACACCCTGGTGA ACCGCATCGAGCTGAAGGGCATCGACTTCAAGGAGGA CGGCAACATCCTGGGGCACAAGCTGGAGTACAACTAC AACAGCCACAACGTCTATATCATGGCCGACAAGCAGA AGAACGGCATCAAGGTGAACTTCAAGATCCGCCACAA CATCGAGGACGGCAGCGTGCAGCTCGCCGACCACTA CCAGCAGAACACCCCCATCGGCGACGGCCCCGTGCT GCTGCCCGACAACCACTACCTGAGCACCCAGTCCGCC CTGAGCAAAGACCCCAACGAGAAGCGCGATCACATGG TCCTGCTGGAGTTCGTGACCGCCGCCGGGATCACTCT CGGCATGGACGAGCTGTACAAG | Coding nucleotide sequence of an EGFP that can be used for encoding a fusion protein of the invention or as a marker of the presence of a transgenic construct. |

(continued)

| SEQ ID NO. | Nucleic Acid Sequence | Description |
|---|---|---|
| SEQ ID NO 19 | ATGGTGAGCAAGGGCGAGGAGCTGTTCACCGGGGTG GTGCCCATCCTGGTCGAGCTGGACGGCGACGTAAAC GGCCACAAGTTCAGCGTGTCCGGCGAGGGCGAGGGC GATGCCACCTACGGCAAGCTGACCCTGAAGTTCATCT GCACCACCGGCAAGCTGCCCGTGCCCTGGCCCACCC TCGTGACCACCCTGACCTACGGCGTGCAGTGCTTCAG CCGCTACCCCGACCACATGAAGCAGCACGACTTCTTC AAGTCCGCCATGCCCGAAGGCTACGTCCAGGAGCGC ACCATCTTCTTCAAGGACGACGGCAACTACAAGACCC GCGCCGAGGTGAAGTTCGAGGGCGACACCCTGGTGA ACCGCATCGAGCTGAAGGGCATCGACTTCAAGGAGGA CGGCAACATCCTGGGGCACAAGCTGGAGTACAACTAC AACAGCCACAACGTCTATATCATGGCCGACAAGCAGA AGAACGGCATCAAGGTGAACTTCAAGATCCGCCACAA CATCGAGGACGGCAGCGTGCAGCTCGCCGACCACTA CCAGCAGAACACCCCCATCGGCGACGGCCCCGTGCT GCTGCCCGACAACCACTACCTGAGCACCCAGTCCGCC CTGAGCAAAGACCCCAACGAGAAGCGCGATCACATGG TCCTGCTGGAGTTCGTGACCGCCGCCGGGATCACTCT CGGCATGGACGAGCTGTACAAGGGAGGCGCTGCAGG AGGCTCTAGAATGGGAAACCAAGAGCTAGAGGAGGTG TTTGTTGCTGTTATCCGACCAAAAAACACAACCAGCCT CAACTCCAAAGAATACCGTGCTAAATCCTATGAGATTT TACTTATCGAAGTTCCCCTGGAGGGAAAAGAGAAAAA GCGGAAGAAAGTGCTGCTTGGGACTAAAATtCACGCC GACAGTGACCGGACAAAGTCAATTCTGGAGTTTGTGG ATGAAACCACCAAGCCCATATCCAATAACCAGGGCATA ATAGGGAAGCGTGTtGTGCACATGAGGAAGTTCCTGTT AGATGGAGACAGTGGAGGAAAAGAGGCCTCGTTATTT ATTGTGCCAATTAATGTCAAAGACAACAGCAAGTCCGT GCATCACCCTGGGAGCCCCAGTTTCTACTGCCTTCAG GACATCATGCGTGTGTGTAGTGAAACCAGTGCTCATTT CTCCTCCAGCACCTCAAAGATGCTGCTCGCTCTCGAC AAGTGGTTAGCGGAGCAGCACACTGTGCCTCACGCTA TTCCTGCTCTGTTCCGGCCGGCTCCGGTGGACCGTGT GAAAACTAACGTGAGTAACCCGGCGTACGCAGTGGAG AAGCAGACAGACGGGACGCTGCACATGGGCTACACG GCTCTGGAGATCAAGAGTAAACTGATGTCTCTGGAGA AAGCAGATCTGTGCATCCAGAACCCGCTGTACGGCTC AGATCTGCAGTACACCAACAGAGTGGACAAAGTCATC ATCAACCCTTACTTTGGCTTGGGAGCTCCAGATTACTC | Coding nucleotide sequence of an EGFP-KRIT1 fusion protein including a linker sequence, which can be used as a functional Krit1/CCM1 protein in the context of the invention. |

(continued)

| SEQ ID NO. | Nucleic Acid Sequence | Description |
|---|---|---|
| | CAAAATCCAGATTCCAAAGAGAGACAAATGGCAGCAC AGCATGACCAGCGTCACCGAAGACAAAGAGCGCCAGT GGGTGGATGATTTCCCTCTGCACCGAAGCGCCTGTGA GGGAGACACTGAGCTTTTGTCTAAATTGCTGGATGGC GGATTCTCAGTCAAACAGCTGGACAGCGACCATTGGG CACCCATTCATTATGCATGCTGGCATGGGAAGGTGGA GGCCACTAAACTGCTGCTGGAGAAAGGCAACTGTAAC CCCAACCTGCTGAACGGCCAGCTGAGCTCTCCTTTAC ACTTCGCAGCTATCGGCGGTCATGCAGAAATCGTGCA GCTCCTCCTGCAGCACCCAGAGATCGACAGACACATC GAGGACCAGCAGAAACGATCGCCGCTTCAAGTATGTG AAGAAAACAAGCAGAACAACTGGGAGGAAACCGTGAA TCTCCTTCAACAAGCCAGCAATAAACCATACGAGAAGG TGAGGATTTACCGTATGGATGGATCGTACCGCTCTGTG GAGCTGAAGCATGGGAACAACACCACCGTGCAGCAGA TCATGGAGGGCATGAGATTATCACAGGAGACCCAGCA GTATTTTACCATCTGGATCTGCTCCGAAAACCTTAGTC TGCAGCTGAAGCCGTACCACAAGCCCTTGCAGCACTT ACGGATGTGGAGCGAGATCGTGACGGATCTCACTGCT CTGGATCCACAGAGAGAAAGTCCACAGCTCTTCCTGC GCAGAGACGTTCGGCTGCCTTTAGAGGTCGAGAAAAA GGTTGAAGATCCGCTGTCCATCCTCATCCTATTTGATG AAGCTCGTCACTGCCTCCTTAAAGGTTTCCTCTCGACG TCAGATAACAAGCTAATCACGCTCGCAAGCCTCCTCCT GCAGATCATCTACGGAAACTACGACAGCAAGAAGCAC AAACAGGGCTTTCTAAATGAGGAAAACCTGAAATCCAT AGTTCCCATTTCAAAGGTCAAGAGCAAAGCACATCACT GGACTAACAGGATACTTCACGAGTACAAGAGTCTGAG CACTAGTGAGGGTGTGAGTAAAGAAATGCATCACCTC CAGAGGCTCTTCCTGCAGAACTGCTGGGATATTCCTAC TTATGGTGCAGCGTTTTTCACAGGACAGGTCTTCACTA AAGCCAGCTCCAGCACACACAAGGTGATCCGCGTGTA TGTGGGCGTCAACACCAAAGGCCTGCATCTGATGAAC ATGGAGACAAAAGTGCTTCACCTCAGTCTGGAGTATG GGACATTTATGTGGCAGCTGGGTCAGGCTGATCAATA CGTGCAAATCCACAGTCTGGAGAACAAGAAGAACTTT GTCGTTCACACCAAACAGGCTGGTCTTATCGTGAAGCT GCTGATGAAGCTGAGTGGACAAATTGCACCAAACGAC AGAGCTGTGTCTGATAAATATGCGTATGGGTAA | |

The invention further relates to functionally analogous sequences of the respective functional versions of a protein required for a functional CCM complex. Protein modifications to the functional proteins required for a functional CCM complex of the present invention, which may occur through substitutions in amino acid sequence, and nucleic acid sequences encoding such molecules, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. In some embodiments this amendment will not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, Ile and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

As explained herein, in the context of the invention functional versions of a protein required for a functional CCM complex may be provided in the form of an exogenous nucleic acids encoding the respective functional versions of a protein required for a functional CCM complex, which may comprise more than one protein, for example in case of CCM3, namely CCM3a and CCM3b.

Nucleic acid sequences of the invention include the nucleic acid sequences encoding a functional version of a protein required for a functional CCM complex of the invention. Protein sequences according to *Table* 1 and functionally analogous sequences represent preferred sequences of functional proteins required for a functional CCM complex. Preferred nucleic acid sequence comprised by encoding an exogenous nucleic acid encoding a functional version of a protein required for a functional CCM complex are listed under *Table 2*.

The functional versions of a protein required for a functional CCM complex of the invention may include proteins tags that allow easy identification or binding of the provided protein through standard techniques, for example by using antibodies directed against the protein tag. A preferred protein-tag that can be encoded by a nucleic acid sequence of the invention is a V5-tag, myc-tag, HA-tag, HIS-tag or an antibody Fc-Fragment. Alternative tags, which are well known in the art and can be selected by a skilled person, may be used instead.

In another aspect, the invention encompasses functional versions of proteins required for a function CCM complex as disclosed herein as well as their use in the context of the methods disclosed herein. In particular, the invention also relates to nucleic acid molecules encoding a functional version of a protein required for a function CCM complex, and in particular one or more nucleic acid molecules encoding a functional version of a protein required for a function CCM complex or parts thereof, selected from the group comprising:

a) one or more nucleic acid molecules comprising a nucleotide sequence which encodes a functional version of a protein required for a function CCM complex;

b) one or more nucleic acid molecules which are complementary to the nucleotide sequences in accordance with a);

c) one or more nucleic acid molecules which undergo hybridization with the nucleotide sequences according to a) or b) under stringent conditions;

d) one or more nucleic acid molecules comprising a nucleotide sequence having sufficient sequence identity to be functionally analogous the nucleotide sequences according to a), b) or c);

e) one or more nucleic acid molecules which, as a consequence of the genetic code, are degenerated into nucleotide sequences according to a) through d); and

f) one or more nucleic acid molecules according the nucleotide sequences of a) through e) which are modified by deletions, additions, substitutions, translocations, inversions and/or insertions and functionally analogous to a nucleotide sequence according to a) through e)

Accordingly, the invention encompasses nucleic acid molecules with at least 60%, preferably 70%, more preferably 80%, especially preferably 90% sequence identity to the nucleic acid molecule encoding a functional version of a protein required for a function CCM complex.

Sequence variants of the claimed nucleic acids and/or proteins, for example defined by the provided % sequence identity, that maintain the said properties of the invention are also included in the scope of the invention. Such variants, which show alternative sequences, but maintain essentially the same properties, such as functionally complementing the CCM complex present in the respective cell of the transgenic fish of the invention. Sequence identity relates to the percentage of identical nucleotides or amino acids when carrying out a sequence alignment, for example using software such as BLAST.

It will be appreciated by those of ordinary skill in the art that, as a result of the degeneracy of the genetic code, there are many nucleotide sequences that encode a polypeptide as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

As used herein, a "reporter gene" may be a gene that encodes a "reporter protein" is any protein that can be specifically detected when expressed. Reporter proteins are useful for detecting or quantitating expression from expression sequences. For example, operatively linking nucleotide sequences encoding a reporter protein to a tissue specific expression sequence allows one to study lineage development, such as the development of the cardiovascular system or blood vessels. In such studies, the reporter protein serves as a marker for monitoring developmental processes, such as development of the heart, blood vessels and/or other aspects of the cardiovascular system. Many reporter proteins are known to one of skill in the art. These include, but are not limited to, beta-galactosidase, luciferase, and alkaline phosphatase that produce specific detectable products. Fluorescent reporter proteins can also be used, such as green fluorescent protein (GFP), cyan fluorescent protein (CFP), red fluorescent protein (RFP), yellow fluorescent protein (YFP). Other examples include the green fluorescent protein from Aequorea coerelescens (AcGFP), DsRedExpress, and red coral fluorescent proteins (for example, AmCyan, ZsGreen, Zs Yellow, AsRed2, DsRed2, and HcRedl). For example, by utilizing GFP, fluorescence is observed upon exposure to light at 489 nm without the addition of a substrate. The use of a reporter protein that, like GFP, is directly detectable without requiring the addition of exogenous factors are preferred for detecting or assessing gene expression during zebrafish embryonic development. Fluorescent proteins can be isolated from many different species, including but not limited to, Aequorea Victoria (Chalfie, et al., 1994), Zoanthus species (Matz, et al., 1999), Renilla reniformis (Ward and Cormier, 1979) and Aequorea coerelescens. Furthermore, the term reporter protein includes but is not limited to pigment proteins, fluorescent proteins, luminescent protein, enzymes and other detectable proteins. Specific examples include but are not limited to melanin (including eumalanin and pheomelanin), carotenoids, pteridines, cyan biocbromes, aequorin, luciferase, luciferin, blue fluorescent protein, red fluorescent protein, ds red fluorescent protein, cyan fluorescent protein, yellow fluorescent protein, and green fluorescent protein (including naturally occurring and mutant variants thereof). See, e.g., U.S. Pat. Nos. 7,034,141; 7,037,645; and 6,087,476. In some embodiments the visually detectable proteins such as the fluorescent or luminescent proteins are preferred. The foregoing is to be construed as inclusive of "enhanced" proteins, including but not limited to enhanced green fluorescent protein (eGFP), which are known in the art. A "reporter gene" is a nucleic acid encoding a reporter protein. "Fluorescent or luminescent" as used herein to describe an animal or fish phenotype means that at least a portion of the animal or fish is visibly fluorescent or luminescent to the ordinary human observer under usual conditions for observing fluorescence or luminescence (e.g., dimmed or reduced light, as in night-time or a darkened room, with or without the addition of supplemental illumination of the animal with an ultra-violet or "black" light). The phenotype may be exhibited as a pattern of fluorescence or luminescence on or through the skin of the animal, overall fluorescence or luminescence on or through the skin of the animal, and/or fluorescence or luminescence of the eyes of the animal, etc.

The transgenic fish of the invention comprise an exogenous nucleic acid encoding a functional version of a protein required for a functional CCM complex under the transcriptional control of an endocardium-specific genetic system.

In the context of the invention, the term "transcriptional control" is used in the sense of regulation of gene expression or gene regulation and includes a wide range of mechanisms that are used by cells to increase or decrease the production of specific gene products (protein or RNA). Sophisticated programs of gene expression are widely observed in biology, for example to trigger developmental pathways, respond to environmental stimuli, or adapt to new food sources. Such systems have been modified or adapted for use in biotechnological application and the control of transgenic genetic elements that may be introduced in cells and organisms, including transgenic fish. In molecular biology and genetics, transcriptional regulation is the means by which a cell regulates the conversion of DNA to RNA (transcription), thereby orchestrating gene activity. A single gene can be regulated in a range of ways, from altering the number of copies of RNA that are transcribed, to the temporal control of when the gene is transcribed. The regulation of transcription is a vital process in all living organisms. It is orchestrated by transcription factors and other proteins working in concert to finely tune the amount of RNA being produced through a variety of mechanisms. Important regulatory elements and aspects of transcriptional control that can form part of genetic systems controlling transcription of nucleic acid sequence include promoters, which are elements of DNA that may bind RNA polymerase and other proteins for the successful initiation of transcription directly upstream of the gene, the chromatin state, transcription factors, and enhancer (or cis-regulatory modules/elements (CRM/CRE)), which are on-coding DNA sequences containing multiple activator and repressor binding sites.

Expression sequences including promoters, enhancers, response elements and combinations thereof (sometimes referred to as "regulatory elements") useful for producing the disclosed zebrafish are known. See, e.g., U.S. Pat. Nos. 6,730,822; 6,380,458; and 5,932,780. The expression sequences may be constitutively active or inducible (e.g., tissue-specific). Examples include but are not limited to the CMV promoter, beta-actin promoter, the RSV promoter, crystalline promoters such as the alpha and delta crystalline promoters, the mylz2 promoter, the PGK promoter, the myosin heavy chain promoter, the myosin light chain promoter, the cardiac myosin promoter, and the keratin promoter. See, e.g., U.S. Pat. Nos. 6,949,242; 6,897,045; and 6,784,289. In some embodiments crystalline promoters and/or enhancers, such as the delta crystalline promoter and/or enhancers, are preferably included in the expression sequence.

A nucleic acid encoding the expression product (sometimes also referred to as a protein of interest) is operatively associated with the expression sequence to form what is sometimes referred to as an "expression cassette" in accordance with known techniques. If desired, insulators can be included upstream, downstream, or both upstream and downstream from the expression sequence and associated nucleic acid encoding the expression product, in accordance with known techniques. See, e.g., U.S. Pat. Nos. 6,395,549; 6,229,070; 6,100,448; and 5,610,053. Also if desired, scaffold attachment regions can be included upstream, downstream, or both upstream and downstream from the expression sequence and associated nucleic acid encoding the expression product, in accordance with known techniques. See, e.g., U.S. Pat. Nos. 6,239,328; 6,100,448; 5,773,695; and 5,773,689.

As used herein, an "endocardium-specific genetic system" relates to any kind of regulatory nucleic acid element of sequence or combination of elements or sequences that lead to endocardium-specific expression of a nucleic acid sequence that is under the control of the said genetic system. For example, an endocardium-specific genetic system can be a endocardium-specific promoter that selectively activates expression of the controlled coding sequence in endocardial cells. Enhancers or promoter/enhancer combination are further examples of elements that can be comprised or can constitute a genetic system in the sense of the invention.

In the context of the invention, the term "endocardium-specific" used in the context of a genetic system, a transcriptional regulator, a transcriptional regulator, a promoter, an enhancer, a promoter/enhancer combination or the like relates to a genetic element that drives or activates gene expression in endocardial cells, whereas it is not activated and does not drive gene expression in endothelial cells or other cells of the cardiovascular system. In that sense, the term "endocardium-specific" is not restricted to genetic elements that are exclusively expressed in endocardial cells, but to those who are capable of inducing expression in endocardial cells, while they are not active in other cells types, in particular not in endothelial cells. However, activity in other cell types, in particular in cell types that do not form part of the cardiovascular system and do not significantly influence development or function of the cardiovascular system, does not exclude a genetic system or element from falling under the term "endocardium-specific" as used herein.

Examples of endocardium-specific promoters include, as non-limiting examples, the nfatc1 promoter, the spp1 promoter and the npr3 promoter.

The endocard or endocardium is the innermost layer of tissue that lines the chambers of the heart. Its cells are embryologically and biologically similar to the endothelial cells that line blood vessels and can be regarded as a specialized subset of endothelial cells. The endocardium also provides protection to the valves and heart chambers. The endocardium underlies the much more voluminous myocardium, the muscular tissue responsible for the contraction of the heart. The endocardium controls certain aspects of myocardial function and therefore fulfills specialized functions that differentiate them from "normal" or "other" endothelial cells.

In embodiments of the invention, the term endocardium can include the endothelial cells of the myocardial capillaries and blood vessels, which are in closed contact to the cardiomyocytes (heart muscle cells), is involved in this modulatory role. In other embodiments of the invention, the term endocardium does not include the endothelial cells of the myocardial capillaries and blood vessels.

Endocardial cells are different from endothelial cells. The term "endothelium" refers to cells that line the interior surface of blood vessels and lymphatic vessels, forming an interface between circulating blood or lymph in the lumen and the rest of the vessel wall. It is a thin layer of simple, or single-layered, squamous cells called endothelial cells. Endothelial cells in direct contact with blood are called vascular endothelial cells, whereas those in direct contact with lymph are known as lymphatic endothelial cells.

Vascular endothelial cells line the entire circulatory system, from the heart to the smallest capillaries. These cells have unique functions in vascular biology. These functions include fluid filtration, such as in the glomerulus of the kidney, blood vessel tone, hemostasis, neutrophil recruitment, and hormone trafficking.

The endothelium of the interior surfaces of the heart chambers is called endocardium. As explained above, endocardial cells have specific functions which are due to their specific location in the heart and therefore have to be differentiated from endothelial cells that are not located in the heart and that do not line the heart chambers.

As used herein the term "endocardium-specific" therefore relates to all genes or genetic elements or genetic systems that are specifically activated in endocardial cells but not in endothelial cells of other organs than the heart or endothelial cells that do not line the heart chambers or endothelial cells that are not located in the heart.

In the context of the present invention, the GAL4-UAS system is a preferred genetic system which can be used for endocardium-specific expression of the functional version of the protein required for a functional CCM complex from the exogenous nucleic acid.

The GAL4-UAS system is generally used to study gene expression and function in organisms such as the fruit fly or the zebra fish. It has also been adapted to study receptor chemical-binding functions in vitro in cell culture. The system has two parts: the Gal4 gene, encoding the yeast transcription activator protein Gal4, and the UAS (Upstream Activation Sequence), an enhancer to which GAL4 specifically binds to activate gene transcription.

For the different model organisms, in which the GAL4-UAS system is used, such as the zebrafish, several so-called GAL4 lines have been developed, each of which expresses GAL4 in some subset of the zebrafish's tissues or cell type. For example, some lines might express GAL4 only or predominantly in muscle cells, nerves, or endocardial cells. For zebrafish, there are many such lines, which express GAL4 in only a very specific subset of cells in the fish. The presence of GAL4, by itself, in these cells has little or no effect, since GAL4's main effect is to bind to a UAS region, and zebrafish cells have no functional UAS regions. Since Gal4 by itself is not visible, and has little effect on cells, the other necessary part of this system are the "reporter lines". These are strains of zebrafish with the special UAS region next to a desired gene. These genetic instructions occur in every cell of the animal, but in most cells nothing happens since that cell is not producing GAL4. In the cells that are producing GAL4, however, the UAS is activated, the gene next to it is turned on, and it starts producing its resulting protein. This may report to the investigator which cells are expressing GAL4, hence the term "reporter line", but genes intended to manipulate the cell behavior are often used as well.

Gal4 is a modular protein consisting broadly of a DNA-binding domain and an activation domain. The UAS to which GAL4 binds is CGG-N11-CCG, where N can be any base. Although GAL4 is a yeast protein not normally present in other organisms it has been shown to work as a transcription activator in a variety of organisms such as Drosophila, zebrafish and human cells.

For study in zebrafish, the GAL4 gene can be placed under the control of a native gene promoter, or driver gene, such as an endocardium-specific promoter or genetic element or promoter/enhancer combination or the like, while the UAS controls expression of a target gene. GAL4 is then only expressed in cells where the driver gene is usually active. In turn, GAL4 should only activate gene transcription where a UAS has been introduced. For example, by fusing a gene encoding a visible marker like GFP (Green Fluorescent Protein) the expression pattern of the driver genes can be determined. GAL4 and the UAS are very useful for studying gene expression in zebrafish as they are not normally present and their expression does not interfere with other processes in the cell.

For technical reasons, variants of Gal4 and the Gal4-UAS system have been developed for application in zebrafish and other organisms. For example, the Gal4-VP16 fusion gene was used in zebrafish to overcome the weak gene expression activity of the full-length Gal4. An even improved version of Gal4-VP16 is represented by Gal4FF, which consists of the DNA binding domain from Gal4 and two transcription activation modules from VP16, which consists of 13 aa containing a critical phenylalanine.

The upstream activating sequence or upstream activation sequence (UAS) is a cis-acting regulatory sequence. It is distinct from the promoter and increases or activates the expression of a neighboring gene. Due to its essential role in activating transcription, the upstream activating sequence is often considered to be analogous to the function of the enhancer in multicellular eukaryotes. Upstream activation sequences are a crucial part of induction, enhancing the expression of the protein of interest through increased transcriptional activity. The upstream activation sequence is found adjacently upstream to a minimal promoter (TATA box) and serves as a binding site for transactivators.

The invention relates to a transgenic fish comprising a cardiovascular system with a heart that produces blood flow, and wherein said fish develops vascular lesions in cerebral blood vessels, in particular in lowly perfused cerebral blood vessels.

As used herein the term "cardiovascular system" refers to the organ system that permits blood to circulate and transport nutrients (such as amino acids and electrolytes), oxygen, carbon dioxide, hormones, and blood cells to and from the cells in the body to provide nourishment and help in fighting diseases, stabilize temperature and pH, and maintain homeostasis. The terms circulatory system or vascular system may be used in the same sense.

The cardiovascular system includes the lymphatic system, which circulates lymph. The passage of lymph for example takes much longer than that of blood. Blood is a fluid consisting of plasma, red blood cells, white blood cells, and platelets that is circulated by the heart through the vertebrate vascular system, carrying oxygen and nutrients to and waste materials away from all body tissues. Lymph is essentially recycled excess blood plasma after it has been filtered from the interstitial fluid (between cells) and returned to the lymphatic system. The cardiovascular system comprises the blood, heart, and blood vessels. The lymph, lymph nodes, and lymph vessels form the lymphatic system, which returns filtered blood plasma from the interstitial fluid (between cells) as lymph.

Zebrafish lacking a functional CCM complex have been shown to form massively dilated hearts that fail to produce blood flow and are marked by increased endocardial cell numbers as compared to wild-type. Furthermore, such fish are characterized by collapsed intersegmental vessels, vascular anomalies of the lateral dorsal aorta, dilated and fused caudal venous plexus and oversprouting cerebrovasculature.

As used herein, the term vascular lesion can relate to vascular diseases and vascular malformation, which is a blood vessel abnormality. In general, the term "vascular lesion" describes any kind of unphysiological vascular formation or phenotype.

Vascular lesions include CCM lesions that consist of grossly dilated, capillary-like structures lined by endothelium and lacking mature vascular wall structure. Lesions can be found in ~0.5% of the general population and affected individuals have a lifetime risk of focal neurological deficits and hemorrhagic stroke. Sporadic lesions are typically solitary, whereas familial CCM is characterized by multifocal cerebral lesions in the setting of mutations in one of three genes encoding KRIT1 (CCM1), CCM2 and PDCD10 (CCM3) or other genes such as the gene encoding Heg1.

Vascular disease is a class of diseases of the blood vessels - the arteries and veins of the circulatory system of the body. It is a subgroup of cardiovascular disease. Disorders in this vast network of blood vessels, can cause a range of health problems which can be severe or prove fatal. There are several types of vascular disease, which is a subgroup of cardiovascular disease, and the signs and symptoms depend on which type, among them are: Erythromelalgia is a rare peripheral vascular disease where syndromes includes burning pain, increased temperature, erythema and swelling, of mainly the hands and feet are affected. Peripheral artery disease happens when atheromatous plaques build up in the arteries that supply blood to the arms and legs, plaque causes the arteries to narrow or become blocked. Renal artery stenosis is the narrowing of renal arteries that carry blood to the kidneys from the aorta. Buerger's disease is due to small blood vessels that inflame and swell, vessels then narrow or are blocked by blood clots. Raynaud's disease is a rare peripheral vascular disorder of constriction of the peripheral blood vessels, in the fingers and toes when the person is cold. Disseminated intravascular coagulation is a widespread activation of clotting in the smaller blood vessels. Cerebrovascular disease is a group of vascular diseases that affect brain function.

In the context of the invention, vascular diseases comprise also vascular diseases with a clonal nature of the disease such as in CCM, HHT, Tie2, RASA1, or Marfan syndrome.

As used herein, the term vascular lesion comprises cerebrovascular disease, which includes a variety of medical conditions that affect the blood vessels of the brain and the cerebral circulation and in particular stroke. Arteries supplying oxygen and nutrients to the brain are often damaged or deformed in these disorders. The most common presentation of cerebrovascular disease is an ischemic stroke or mini-stroke and sometimes a hemorrhagic stroke. Hypertension (high blood pressure) is the most important contributing risk factor for stroke and cerebrovascular diseases as it can change the structure of blood vessels and result in atherosclerosis. Atherosclerosis narrows blood vessels in the brain, resulting in decreased cerebral perfusion. Other risk factors that contribute to stroke include smoking and diabetes. Narrowed cerebral arteries can lead to ischemic stroke, but continually elevated blood pressure can also cause tearing of vessels, leading to a hemorrhagic stroke. A stroke usually presents with an abrupt onset of a neurologic deficit - such as hemiplegia (one-sided weakness), numbness, aphasia (language impairment), or ataxia (loss of coordination) - attributable to a focal vascular lesion. The neurologic symptoms manifest within seconds because neurons need a continual supply of nutrients, including glucose and oxygen, that are provided by the blood. Therefore if blood supply to the brain is impeded, injury and energy failure is rapid. Besides hypertension, there are also many less common causes of cerebrovascular disease, including those that are congenital or idiopathic and include CADASIL (cerebral autosomal dominant arteriopathy with subcortical infarcts and leukoencephalopathy), aneurysms, amyloid angiopathy, arteriovenous malformations, fistulas, and arterial dissections. Many of these diseases can be asymptomatic until an acute event, such as a stroke, occurs. Cerebrovascular diseases can also present less commonly with headache or seizures. Any of these diseases can result in vascular dementia due to ischemic damage to the brain.

A stroke is a medical condition in which poor blood flow to the brain results in cell death. There are two main types of stroke: ischemic, due to lack of blood flow, and hemorrhagic, due to bleeding. Both result in parts of the brain not functioning properly. Signs and symptoms of a stroke may include an inability to move or feel on one side of the body, problems understanding or speaking, dizziness, or loss of vision to one side. Signs and symptoms often appear soon after the stroke has occurred. If symptoms last less than one or two hours it is known as a transient ischemic attack (TIA) or mini-stroke. A hemorrhagic stroke may also be associated with a severe headache. The symptoms of a stroke can be permanent. Long-term complications may include pneumonia or loss of bladder control. The main risk factor for stroke is high blood pressure. Other risk factors include tobacco smoking, obesity, high blood cholesterol, diabetes mellitus, a previous TIA, and atrial fibrillation. An ischemic stroke is typically caused by blockage of a blood vessel, though there are also less common causes. A hemorrhagic stroke is caused by either bleeding directly into the brain or into the space between the brain's membranes. Bleeding may occur due to a ruptured brain aneurysm. Diagnosis is typically based on a physical exam and supported by medical imaging such as a CT scan or MRI scan. A CT scan can rule out bleeding, but may not necessarily rule out ischemia, which early on typically does not show up on a CT scan. Other tests such as an electrocardiogram (ECG) and blood tests are done to determine risk factors and rule out other possible causes. Low blood sugar may cause similar symptoms.

The most common blood vessel abnormality/vascular malformation is arteriovenous malformation. Arteriovenous malformation is an abnormal connection between arteries and veins, bypassing the capillary system. This vascular anomaly is widely known because of its occurrence in the central nervous system (usually cerebral AVM), but can appear in any location. Although many AVMs are asymptomatic, they can cause intense pain or bleeding or lead to other serious medical problems. AVMs are usually congenital and belong to the RASopathies.

The term "cerebellar blood vessels" relates to blood vessels of the cerebellum.

In the context of the invention, the term "lowly perfused blood vessel" refers to blood vessels, with a low blood flow, meaning that the rate of blood flowing through these vessels is low, in particular compared to strongly perfused main blood vessels, such as the aorta. In lowly perfused blood vessels, hemodynamic forces are less pronounced and can lead to endothelial-to-mesenchymal transition (endMT) within lowly-perfuse blood vessels, which is a process common to many vascular pathologies.

Vascular lesions can be identified by visualizing the hollow or lumenized spaces of the cardiovascular system, which are the blood-filled spaces of the cardiovascular system. These can be visualized by various techniques known to a skilled person, for example by microangiography.

In a transgenic fish, components of the blood such as blood cells or proteins present in the blood can be labeled, for example by using reporter proteins that are expressed in blood cells or are fused to proteins present in blood cells or in the extracellular space of the blood, for example serum proteins or plasma proteins. Reporter proteins include, without limitation, photoproteins, such as aequorin; obelin; Aequorea fluorescent proteins, such, e.g., green fluorescent proteins (GFP, EGFP, AcGFP1), cyan fluorescent proteins (CFP, ECFP), blue fluorescent proteins (BFP, EBFP), red fluorescent proteins (RFP), yellow fluorescent proteins (YFP, EYFP), ultraviolet fluorescent protein (GFPuv), their fluorescence-enhancement variants, their peptide destabilization variants, and the like; coral reef fluorescent proteins, such, e.g., Discosoma red fluorescent proteins (DsRed, DsRed1, DsRed2, and DsRed-Express), Anemonia red fluorescent proteins (AsRed and AsRed2), Heteractis far-red fluorescent proteins (HcRed, HcRed1), Anemonia cyan fluorescent proteins (AmCyan, AmCyan1), Zoanthus green fluorescent proteins (ZsGreen, ZsGreen1), Zoanthus yellow fluorescent proteins (ZsYellow, ZsYellow1), their fluorescence-enhancement variants, their peptide destabilization variants, and the like; Renilla reniformis green fluorescent protein (Vitality hrGFP), its fluorescence-enhancement variants, its peptide destabilization variants, and the like; and Great Star Coral fluorescent proteins, such, e.g., Montastrea cavernosa fluorescent protein (Monster Green® Fluorescent Protein), its fluorescence-enhancement variants, its peptide destabilization variants, and the like. One skilled in the art understands that these and a variety of other fluorescent proteins can be useful as a fluorescent protein in aspects of the invention, see, e.g., Jennifer Lippincott-Schwartz & George H. Patterson, Development and Use of Fluorescent Protein Markers in Living Cells, 300(5616) Science 87-91 (2003); and Jin Zhang et al., 3(12) Nat. Rev. Mol. Cell. Biol. 906-918 (2002). One skilled in the art understands that these and many other fluorescent proteins, including species orthologs and paralogs of the above described naturally occurring fluorescent proteins as well as engineered fluorescent proteins can be useful as a fluorescent protein disclosed in aspects of the present specification.

Furthermore, traceable reagent can be injected into the cardiovascular system or the blood stream of the fish. For example, fluorescently labeled dextran can be injected into the blood stream of the fish, for example into the common cardinal vein (CCV). Alternatively, labeled nanobeads/nanoparticals can be injected into the blood stream of the fish. Suitable fluorescent labels are known to the skilled person and include, without limitation, rhodamine and derivatives, such as Texas Red, fluorescein and derivatives, such as 5-bromomethyl fluorescein, Lucifer Yellow, IAEDANS, 7-Me2N-coumarin-4-acetate, 7-OH-4-CH3-coumarin-3-acetate, 7-NH2-4CH3-coumarin-3-acetate (AMCA), monobromobimane, pyrene trisulfonates, such as Cascade Blue, and monobromotrimethyl-ammoniobimane, FAM, TET, CAL Fluor Gold 540, HEX, JOE, VIC, CAL Fluor Orange 560, Cy3, NED, Quasar 570, Oyster 556, TMR, CAL Fluor Red 590, ROX, LC red 610, CAL Fluor Red 610, Texas red, LC red 610, CAL Fluor Red 610, LC red 640, CAL Fluor Red 635, Cy5, LC red 670, Quasar 670, Oyster 645, LC red 705, Cy5.5, BODIPY FL, Oregon Green 488, Rhodamine Green, Oregon Green 514, Cal Gold, BODIPY R6Gj, Yakima Yellow, JOE, HEX, Cal Orange, BODIPY TMR-X, Quasar-570 /Cy3, TAMRA, Rhodamine Red-X, Rhodamine B, Rhodamine 6G, Redmond Red, BODIPY 581/591, Cy3.5, Cal Red/Texas Red, BODIPY TR-X, BODIPY 630/665-X, Pulsar-650, Quasar-670/Cy5.

Further traceable dyes or reagents are known a person skilled in the art.

Furthermore, the invention relates to a method for measuring or testing a pharmacological effect of a drug or for testing a pharmacological effect of a drug or compound, comprising

- contacting a transgenic fish according to any one of the claims with a drug, and

- measuring a pharmacological effect of the drug.

As used herein, the term "drug" relates to any compound, such as a biological or chemical molecule, such as a small molecule, a protein, a nucleic acid, chemicals, inorganic molecules, organic molecules, cDNA encoding a protein, a secreted protein, a large molecule, an antibody, a morpholino, a triple helix molecule, a peptide, siRNA, shRNA, miRNA, antisense RNA, ribozyme or any other compound that can be envisioned to be used as a drug in the treatment of a patient.

In the context of the invention, "contacting" a transgenic fish with a drug or compound comprises incubation of the fish in a suitable medium comprising said compound, but also any other form of administration of said compound to the transgenic fish comprising feeding, oral administration and injection, such as injection into a body cavity or into the blood stream or into a tissue. Contacting also comprises implantation of pellets that release substances into the body or blood flow. Suitable routes of administration are known to a skilled person.

In the context of the invention, the term "pharmacological effect" relates to a biochemical or physiological effect of a drug or compound on a cell, tissue or organ of the transgenic fish or on the whole organism, namely the transgenic fish. Such a pharmacological effect can be measured by determining the differences that occur in presence of the drug or compound as compared to its absence in a certain setup, such as an experimental setup.

As the present invention is in particular directed to methods for measuring an effect of a drug on the formation of vascular lesions, the development of cerebral cavernous malformation and/or the development of stroke, the setup for measuring the pharmacological effect of the drug should be suitable for detecting vascular lesions, the development of cerebral cavernous malformation and/or the development of stroke, for example by microscopic means, preferably involving microangiography. Such effects on can be quantified by various means, for example by measuring the number an the extend of the occurring lesions in the transgenic fish. Example of such measurements and quantifications can be found in the examples below.

Microangiography is a type of angiography that consists of the imaging of small blood or lymphatic vessels of an organ. While most other types of angiography cannot produce images of vessels smaller than 200 $\mu$m in diameter, microangiography does just that. A microangiographic image is the result of injection of a contrast medium or traceable medium or dye into either the blood or the lymphatic system. Thus, an image is obtained in which there is contrast between vessel and surrounding tissue. It is often used in order to detect microvascular lesions in organs. Microangiography includes, without limitation, fluorescent, silicone rubber, and synchrotron radiation microangiography.

The transgenic fish of the invention can be used as a model for the treatment and/or prevention of vascular lesions, cerebral cavernous malformation and/or stroke. Since the fish is an easily accessible model organism that is transparent and can be easily applied to high throughput analysis, for example parallel analysis of a large number of drug candidates, the fish of the invention represent a great model system for the analysis of the effect of a compound or drug on the formation of vascular lesions. For example, screening of compounds of a compound library for their effect on vascular lesions, the development of cerebral cavernous malformation and/or the development of stroke using the transgenic fish as a experimental model system is possible.

Unless defined otherwise, all technical and scientific terms used herein have the same meaning as commonly understood by one of ordinary skill in the art to which this invention belongs. The practice of the present invention will employ, unless otherwise indicated, conventional techniques of chemistry, molecular biology, microbiology, recombinant DNA, and immunology, which are within the capabilities of a person of ordinary skill in the art. Such techniques are explained in the literature. *See, e.g.,* J. Sambrook, E. F. Fritsch, and T. Maniatis, 1989, Molecular Cloning: A Laboratory Manual, Second Edition, Books 1-3, Cold Spring Harbor Laboratory Press; Ausubel, F. M. et al. (1995 and periodic supplements) Current Protocols in Molecular Biology, ch. 9, 13, and 16, John Wiley & Sons, New York, N.Y.); B. Roe, J. Crabtree, and A. Kahn, 1996, DNA Isolation and Sequencing: Essential Techniques, John Wiley & Sons J. M. Polak and James O'D. McGee, 1990, In Situ Hybridization: Principles and Practice; Oxford University Press; M. J. Gait (Editor), 1984, Oligonucleotide Synthesis: A Practical Approach , Irl Press; D. M. J. Lilley and J. E. Dahlberg, 1992, Methods of Enzymology: DNA Structure Part A: Synthesis and Physical Analysis of DNA Methods in Enzymology, Academic Press; Using Antibodies: A Laboratory Manual: Portable Protocol NO. I by Edward Harlow, David Lane, Ed Harlow (1999, Cold Spring Harbor Laboratory Press, ISBN 0-87969-544-7); Antibodies: A Laboratory Manual by Ed Harlow (Editor), David Lane (Editor) (1988, Cold Spring Harbor Laboratory Press, ISBN 0-87969-3,4-2), 1855. Handbook of Drug Screening, edited by Ramakrishna Seethala, Prabhavathi B. Fernandes (2001, New York, N.Y., Marcel Dekker, ISBN 0-8247-0562-9); and Lab Ref: A Handbook of Recipes, Reagents, and Other Reference Tools for Use at the Bench, Edited Jane Roskams and Linda Rodgers, 2002, Cold Spring Harbor Laboratory, ISBN 0-87969-630-3. Also, all publications, patent applications, patents and other references mentioned herein are incorporated by reference in their entirety.

**FIGURES**

The invention is further described by the following figures. These are not intended to limit the scope of the invention, but represent preferred embodiments of aspects of the invention provided for greater illustration of the invention described herein.

**Description of the figures:**

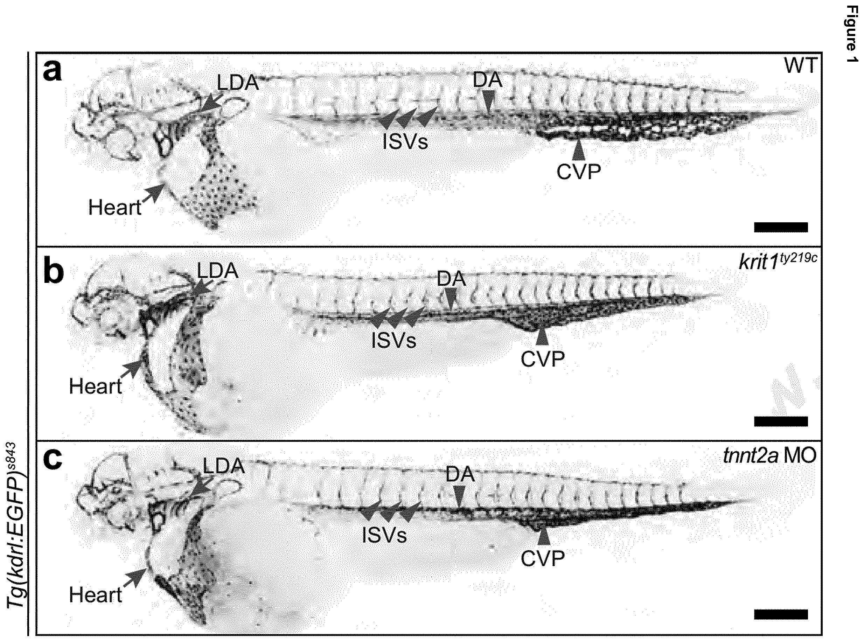

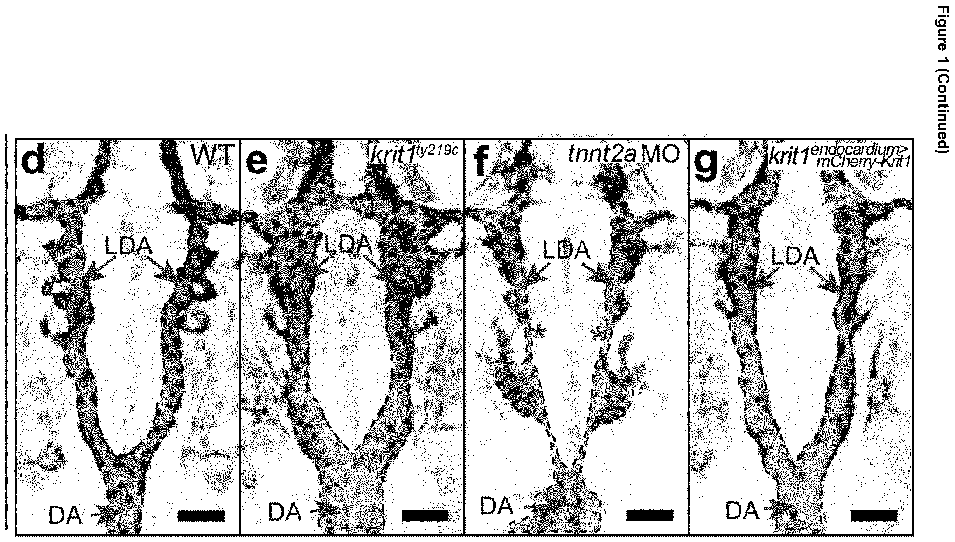

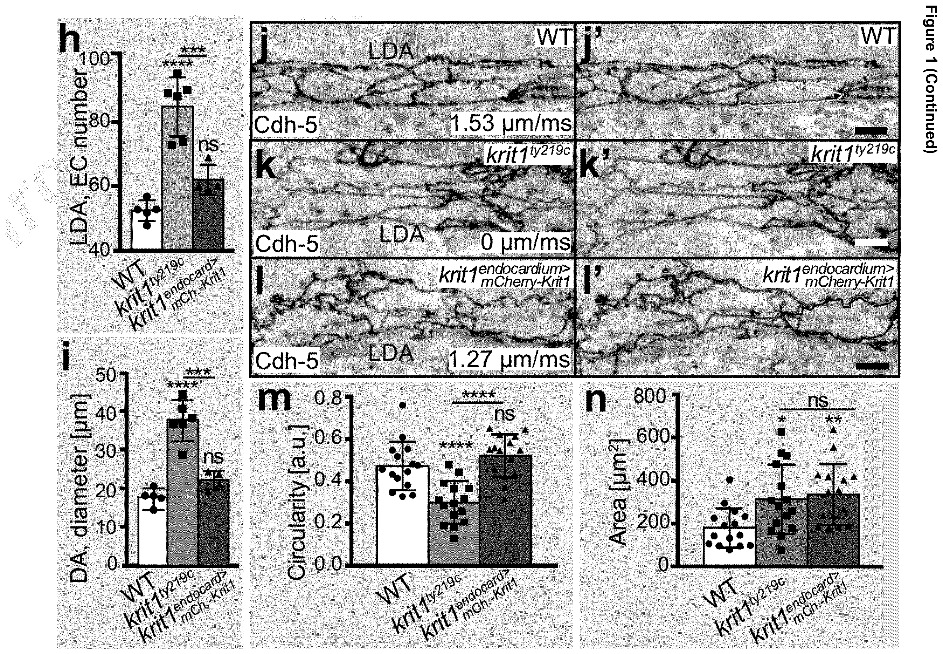

**Figure 1.** *Rescue of vascular anomalies in Krit1-deficient zebrafish upon restoration of blood flow.*

**a-g**, Endothelial cells marked by the expression of *Tg(kdrl:EGFP)$^{s843}$* at 56 hpf (shown as black/white inverted maximum projections of several confocal z-stack images). **a-c**, Lateral overview of different vascular beds show that wild-type (**a**) morphologies of the heart, lateral dorsal aorta (LDA), intersegmental vessels (ISVs), and caudal vein plexus (CVP) are abnormal in *krit1ty219c* mutants (**b**) and *tnnt2a* morphants (c). **d-g**, Phenotypes of LDA and DA (labelled in green) in different genetic conditions. In *krit1ty219c* mutants, the LDA and DA are massively dilated (**e**). *tnnt2a* morphants have a deformed LDA which is constricted in mid-section (**f**, red asterisks). **g**, *krit1endocardium>mCherry-Krit1* embryo with restored morphology of the LDA and DA. **h**, Quantifications of EC numbers of LDA in wild-type (n=5 embryos), *krit1ty219c* mutants (n=6 embryos) and *krit1endocardium>mCherry-Krit1* embryos (n=4 embryos), **i**, Quantification of the proximal DA diameter, in wild-type (n=5 embryos), *krit1ty219c* mutants (n=6 embryos) and *krit1endocardium>mCherry-Krit1* embryos (n=4 embryos), **j-l**, Maximum intensity projection of confocal z-stack of endothelial cells of the lateral dorsal aorta are marked by immuno-labelling of VE-Cadherin (Cdh-5, black labelling). The red blood cell (RBC) velocities are indicated in the lower right corners. **j**, While wild-type endothelial cells are elongated in direction of blood flow (RBC velocity 1.53 $\mu$m/ms), **k**, *krit1ty219c* mutant endothelial cells are strongly elongated (lack of blood flow, RBC velocity 0 mm/ms). In comparison, *krit1endocardium>mCherry-Krit1* endothelial cells, exposed to moderately normal RBC velocity (1.27 $\mu$m/ms), are less elongated and apparently have more of a wild-type morphology. **j'-l'**, individual endothelial cell membranes have manually been redrawn with unique colors. **m, n**, Quantification of LDA endothelial cell shapes, circularity and surface area in wild-type, *krit1ty219c* mutants, and *krit1endocardium>mCherry-Krit1* embryos, respectively (n=15 cells from 3 embryos for each condition). One-way ANOVA, Tukey's multiple comparison tests, ****$P\leqq0.0001$; ***$P=0.005$, **$P=0.007$; *$P=0.027$; ns, not significant. Scale bars: **a-c**, 200 $\mu$m; **d-g**, 50 $\mu$m; **j-l**, 10 $\mu$m.

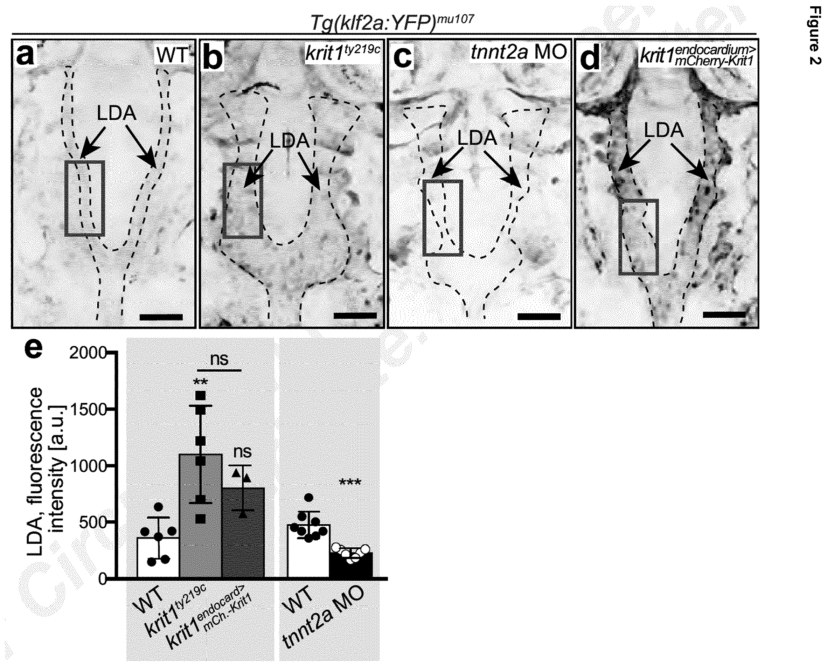

**Figure 2.** Loss *of zebrafish CCM proteins results in klf2a expression within the lateral dorsal aorta even in the absence of blood.*

**a-c**, Shown are reconstructions based on several confocal z-stack images of transgenic *Tg(klf2a:YFP)m107* reporter expression in the LDA (outlined by dashed line) at 54 hpf. YFP expression is present in the LDA of wild-type (**a**) and *krit1ty219c* mutant (**b**) which lacks blood flow. **c**, In contrast, the reporter *Tg(klf2a:YFP)m107* expression is completely absent in *tnnt2a* morphants *(tnnt2a* MO), which is another no-flow condition. **d**, Restoration of blood flow in *krit1endocardium>mCherry-Krit1* embryo does not alter high levels of *klf2a* expression. **e**, Quantification of *klf2a*:YFP expression measured in the LDA (red rectangle in **a-d**). The fluorescent YFP intensity is significantly higher in *krit1ty219c* mutant embryos (**b**) as compared to wild-type (**a**). Unlike in *tnnt2a* morphants, where *klf2a* reporter levels are significantly reduced (**c**), in *krit1endocardium>mCherry-Krit1* embryos, the *klf2a* reporter remains upregulated (**d**) Left panel, one-way ANOVA, Tukey's multiple comparison test; **$P=0038$, ns, not significant. Right panel, unpaired t-test ***$P=0.0003$. a.u., arbitrary unit. Scale bars: 50 $\mu$m.

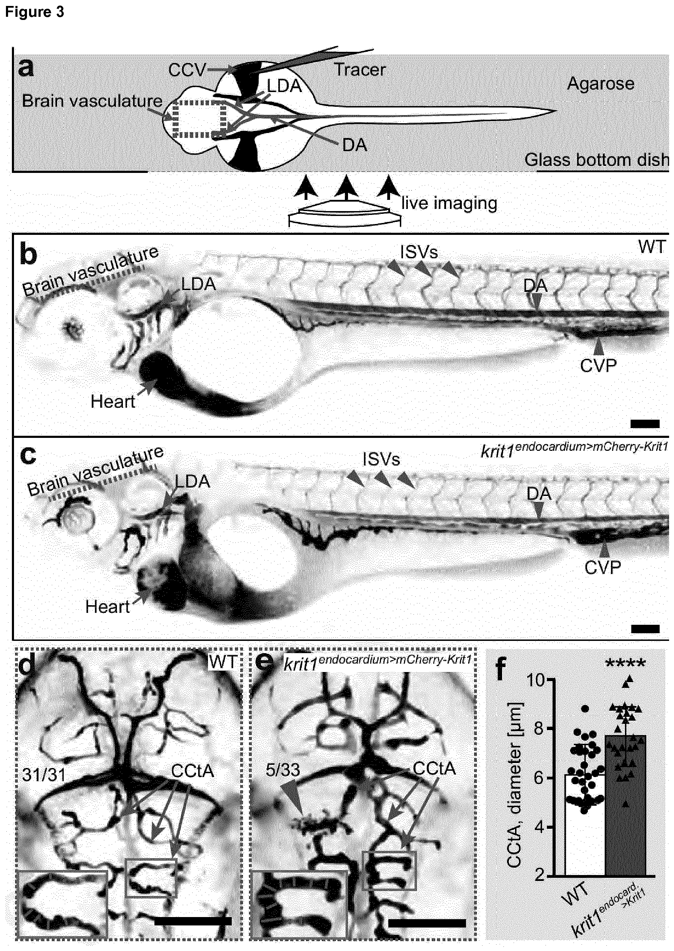

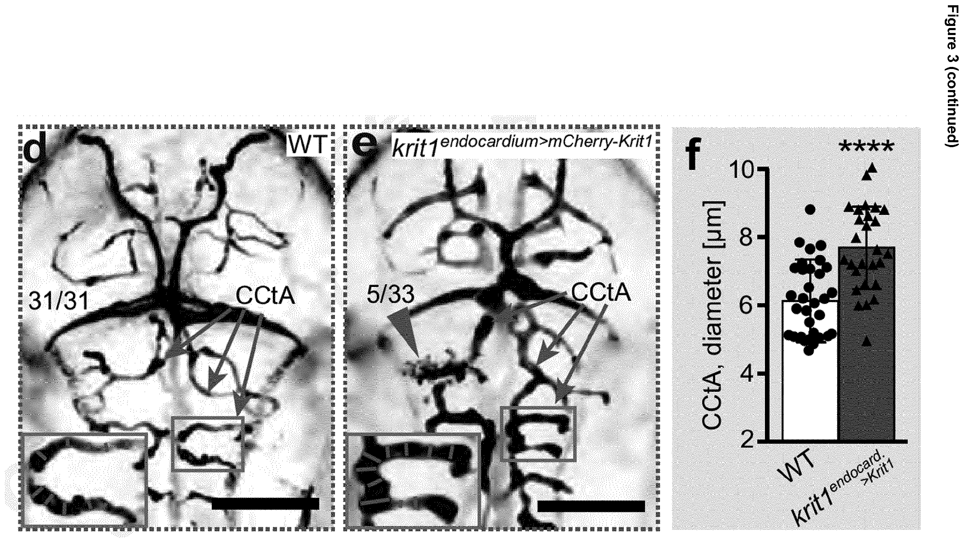

**Figure 3.** *Microangiography reveals that low-flow vascular beds in the cerebrovasculature of Krit1-deficient zebrafish are dilated and leaky.*

**a,** Schematic representation of the microangiography experiment in 2 days post fertilization embryos, **b-e,** Immediately after injection, 70 kDa Texas-Red-conjugated dextran spreads rapidly throughout the entire vasculature, outlining all vessels. Lateral views of projection images are based on inverted black/white confocal z-stack images of the tracer dye in wild-type (**b**) and *krit1endocardium>mCherry-Krit1* (**c**) embryos at 60 hpf. **b, c,** Red-dotted lines indicate area of the cerebrovasculature shown from dorsal views in **d** and **e. d,** In wild-type, the cerebral central arteries (CCtA) are perfused and do not leak (n=31/31 embryos), whereas in *krit1endocardium>mCherry-Krit1* embryos (**e**), CCtAs are dilated (compare insets in **d** and **e,** diameters indicated by red lines) and tracer dye immediately leaks into the periphery within 10-15 minutes upon injection (n=5/33 embryos; arrowhead in **e**). **f**, Quantification of CCtA diameters after three independent experiments reveals increased diameter in *krit1endocardium>mCherry-Krit1* embryos as compared to wild-type. Unpaired, two-tailed t-test with Welch's correction; ****$P<0.0001$. Scale bars: 100 $\mu$m.

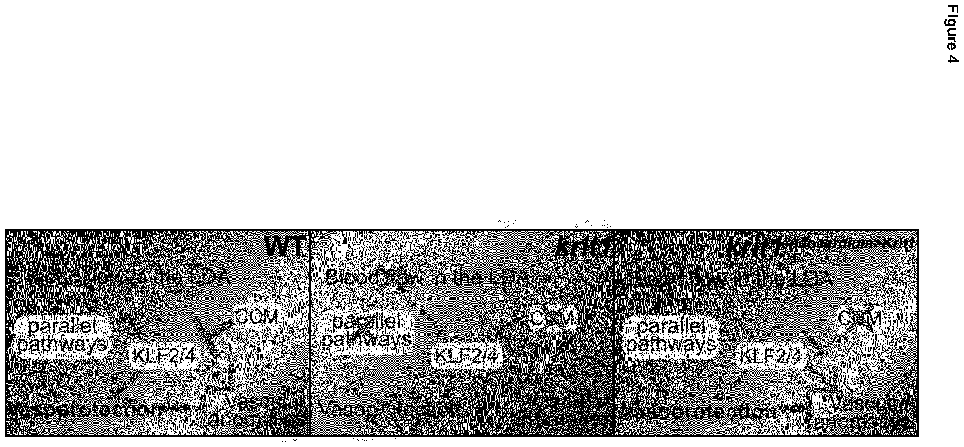

**Figure 4.** *Model figure.*

Our work suggests that in the dorsal aorta blood flow is a physiological modifier with a vasoprotective effect on Krit1-deficient endothelial cells (left panel). Upon loss of CCM proteins, pathological KLF2/4 signaling causes vascular anomalies (middle panel). These effects may be masked when high levels of fluid shear stress activate additional vasoprotective biomechanical signaling pathways or funnel KLF2/4 signal transduction towards vasoprotective target genes (right panel).

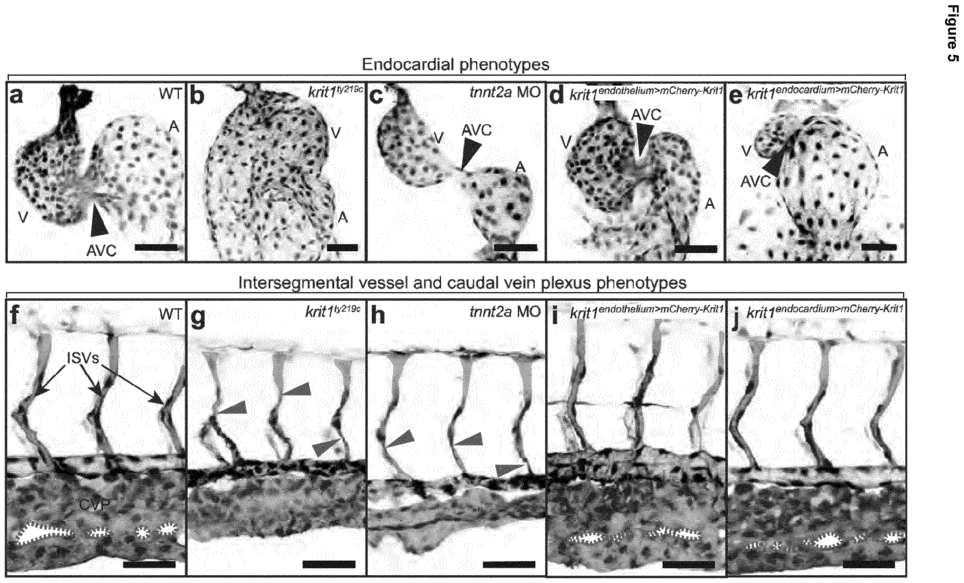

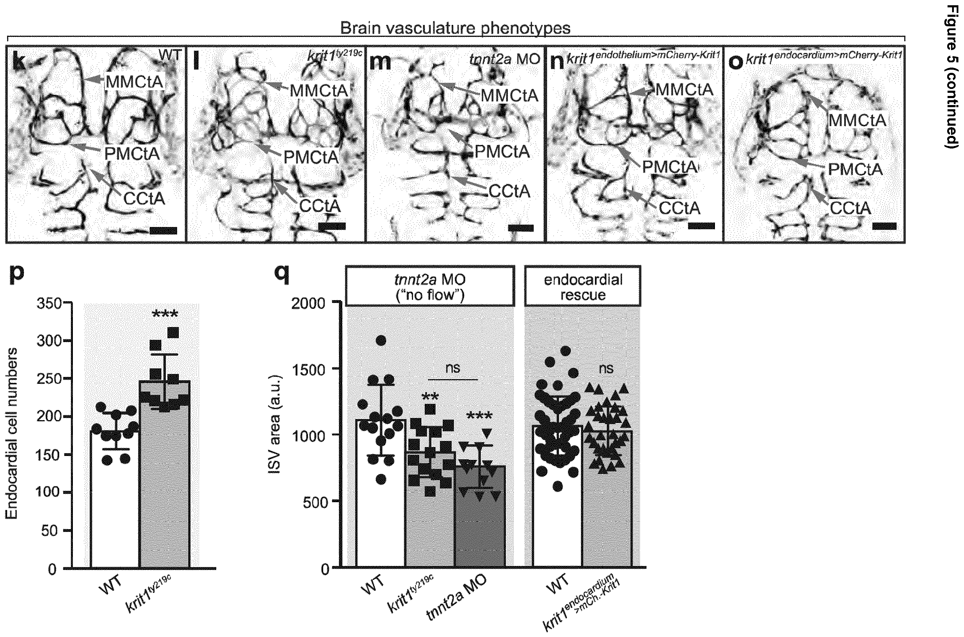

**Figure 5.** *Endothelium- and endocardium-specific expression of mCherry-Krit1 restores cardiac morphology in krit1ty219c mutants.*

**a-o**, Shown are different vascular beds visualised by endocardial and endothelial expression of the *Tg(kdrl:EGFP)s843* reporter at 54 hpf. **a-e**, Endocardial phenotypes. **a**, Wild-type hearts form two distinct cardiac chambers in which ventricle (V) and atrium (A) are separated by the atrioventricular canal (AVC). **b**, *krit1ty219c* mutant hearts are massively enlarged without a constriction at the AVC. **c**, The endocardium in *tnnt2a* morphants is smaller with two cardiac chambers separated by a constriction at the AVC. **d**, In *krit1endothelium>mCherry-Krit1* embryos, the cardiac morphology of the two chambers, atrium and ventricle, is completely restored. **e**, Similarly, in *krit1endocardium>mCherry-Krit1* embryos cardiac morphology is restored. **f-j**, Shown are morphologies of intersegmental vessels (marked in magenta) and the caudal vein plexus (marked in blue) in the different genetic conditions tested. **f**, In wild-type embryos, the intersegmental vessels form patent tubes and the caudal vein plexus forms a network of vessels characterized by fenestra (outlined with red-dotted line). *krit1*<sup>ty219c</sup> mutants (**g**) and *tnnt2a* morphants (**h**) have similar vascular anomalies of collapsed intersegmental vessels (red arrowheads) and a fused caudal vein plexus. In *krit1*<sup>endothelium>mCherry-Krit1</sup> embryos (**i**) and in *krit1*<sup>endocardium>mCherry-Krit1</sup> embryos (**j**), the intersegmental vessel morphology and caudal vein plexus morphologies are restored. **k-o**, Shown are morphologies of the cerebrovasculature with the middle mesencephalic central artery (MMCtA), posterior mesencephalic central artery (PMCtA), and cerebral central arteries (CCtA) in wild-type (**k**) and different genetic conditions tested (**l-o**). The cerebrovasculature is oversprouting between the MMCtA and PMCtA (labeled in magenta) in *krit1*<sup>ty219c</sup> mutant embryos (**l**) and to a lesser extend in *tnnt2a* morphants (**h**). In *krit1*<sup>endothelium>mCherry-Krit1</sup> embryos (**n**) and in *krit1*<sup>endocardium>mCherry-Krit1</sup> embryos (**o**) this oversprouting phenotype is reduced, **p**, Quantification of endocardial cell numbers at 48 hpf show a significant increase in *krit1*<sup>ty219c</sup> mutant at compared to wild-type. Unpaired, two-tailed t-test with Welch's correction; ***$P$=0.0005 **q**, Quantification of intersegmental vessel size. *krit1*<sup>ty219c</sup> mutant and *tnnt2a* morphant intersegmental vessels are significantly smaller than wild-type vessels (left panel), whereas the intersegmental vessels of *krit1*<sup>endocardium>mCherry-Krit1</sup> embryos are restored to wild-type dimensions (right panel). Left panel, one-way ANOVA, Tukey's multiple comparison test; **$P$=0.0077; ***$P$=0.0001; ns, not significant. Right panel, unpaired, two-tailed t-test with Welch's correction; $P$=0.4235; ns, not significant. a.u., arbitrary unit. Scale bars: 50 $\mu$m.

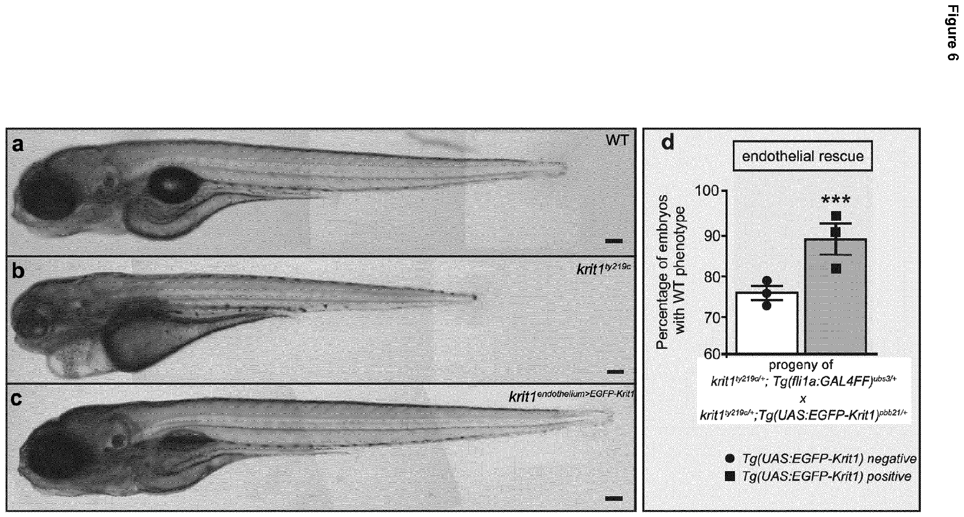

**Figure 6.** krit1<sup>ty219c</sup> mutant embryos are rescued by endothelial transgenic expression of EGFP-Krit1.

**a-c**, Lateral views of embryos at 5 dpf. Compared with wild-type (**a**), *krit1*<sup>ty219c</sup> mutants (**b**) develop pericardial edema due to cardiovascular anomalies and have a shortened body length. **c**, *krit1*<sup>ty219c</sup> mutants expressing *Tg(fli1a:GAL4FF)*<sup>ubs3;</sup> *Tg(UAS:EGFP-Krit1)*<sup>pbb21</sup> (referred to as *krit1*<sup>endothelium>EGFP-Krit1</sup>) develop normally until 5 dpf. **d**, Quantification of the occurrence of a wild-type phenotype among the progeny of different genetic crosses at 5 dpf. In a heterozygous incross of *krit1*<sup>ty219c/+</sup>; *Tg(fli1a:GAL4FF)*<sup>ubs3/+</sup>; *Tg(UAS:EGFP-Krit1)*<sup>pbb21/+</sup> the percentage of wild-type phenotypes is increased as compared to an incross of *krit1*<sup>ty219c/+</sup>fish. Student's t-test with s.d.; ***$P$=0.0341. Scale bars: 100 $\mu$m.

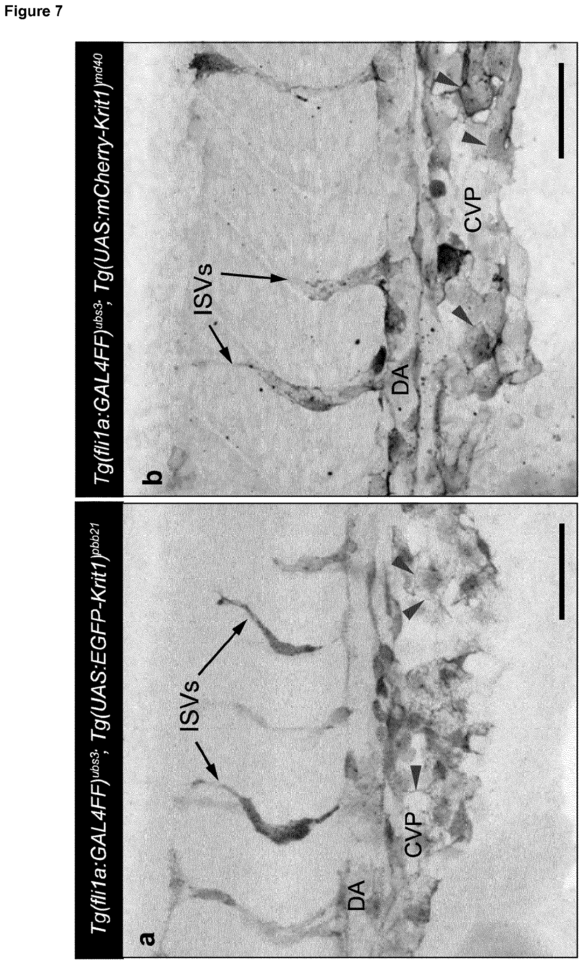

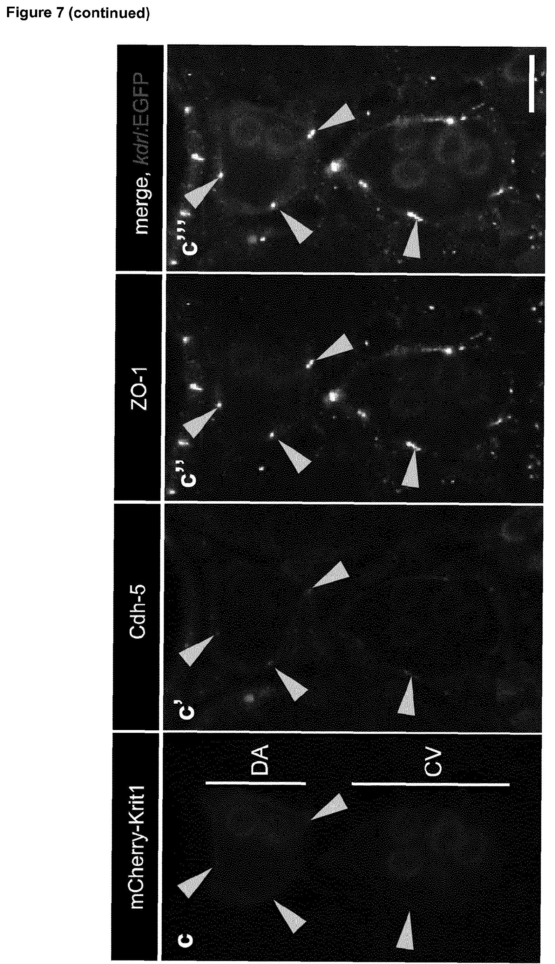

**Figure 7.** *Expression of EGFP-Krit1 and mCherry-Krit1 in wild-type embryos in different vascular beds.*

**a, b**, Shown are projection images based on several confocal microscopy z-stack images of different vascular beds including intersegmental vessels (ISVs), dorsal aorta (DA), and caudal vein plexus (CVP) at 24 hpf. EGFP-Krit1 (**a**, *Tg(fli1a:GAL4FF)*<sup>ubs3;</sup> *Tg(UAS:EGFP-Krit1)*<sup>pbb21</sup>) and mCherry-Krit1 (**b**, *Tg(fli1a:GAL4FF)*<sup>ubs3;</sup> *Tg(UAS:mCherry-Krit1)*<sup>md40</sup>) are expressed in endothelial cells where both fusion proteins also localize to cell junctions (red arrowheads). **c-c'''**, Single confocal z-planes of immunohistochemically stained transversal section in trunk region. **c**, Transgenically expressed mCherry-Krit1 protein localizes to endothelial cell junctions that are marked by junctional proteins Cdh-5 (**c'**) and ZO-1 (**c''**) (yellow arrowheads). **c'''**, Merge image of mCherry-Krit1 (red), Cdh-5 (green), ZO-1 (white), and endothelial cells. The endothelial cells are marked by reporter line *Tg(kdrl:EGFP)*<sup>s843</sup> (blue). Scale bars: **a, b**, 50 $\mu$m; **c-c'''**, 10 $\mu$m.

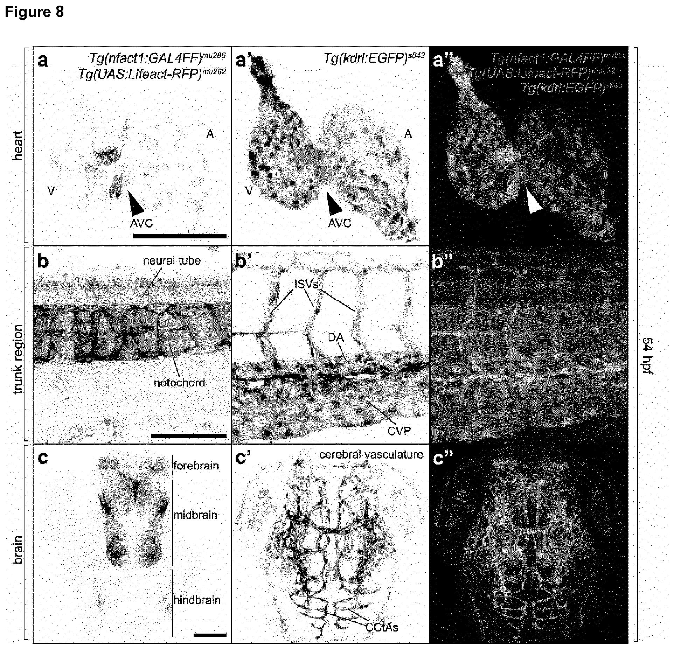

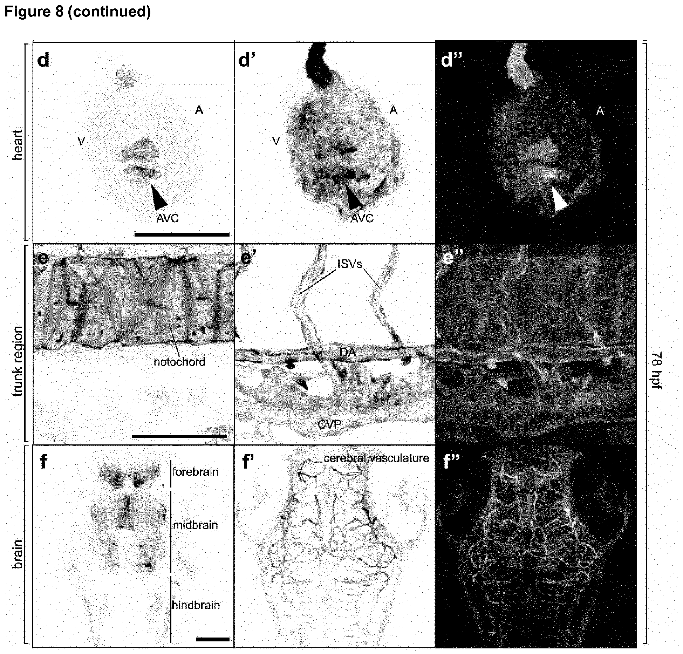

**Figure 8.** *Endothelial expression of the TgBAC(nfatc1:GAL4FF)*<sup>mu286</sup> *driver line is restricted to the endocardium.*

Shown are projection images based on several confocal microscopy z-stack images. All endothelial cells are marked by expression of *Tg(kdrl:EGFP)*<sup>s843</sup> (**a'-f'**; **a''-f''**, false-colored in cyan). **a-f**, Shown is the *Tg(UAS:Lifeact-RFP)*<sup>mu262</sup> expression, driven by *TgBAC(nfatc1:GAL4FF)*<sup>mu286</sup> (**a''-f''**, false-colored in magenta) in different embryonic tissues at 54 hpf (**a-c**) and 78 hpf (**d-f**). At 54 hpf (**a, a''**) and 78 hpf (**d, d''**) expression of *TgBAC(nfatc1:GAL4FF)*<sup>mu286</sup>; *Tg(UAS:Lifeact-RFP)*<sup>mu262</sup> is restricted to endocardial cells of the AVC (arrowheads) in the heart, whereas expression is undetectable in other endothelial tissues (**b, c, e, f,**). Scale bars: 50 $\mu$m.

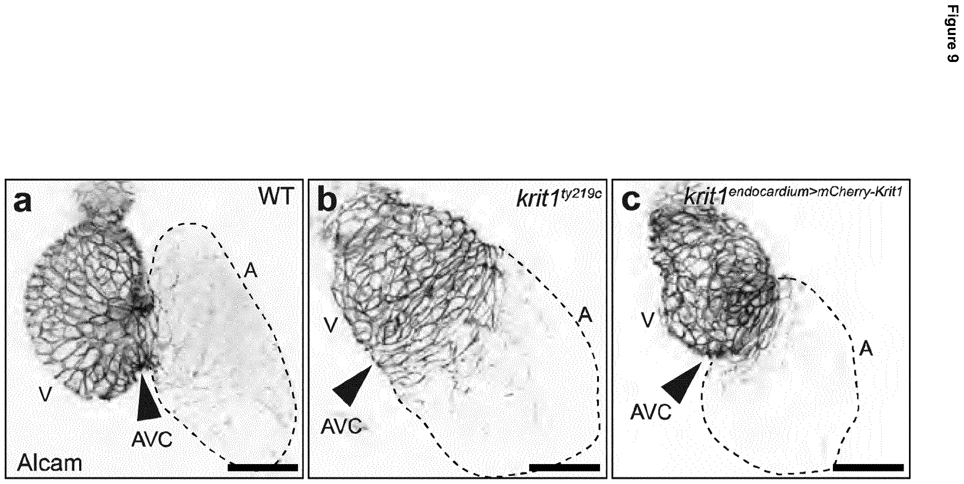

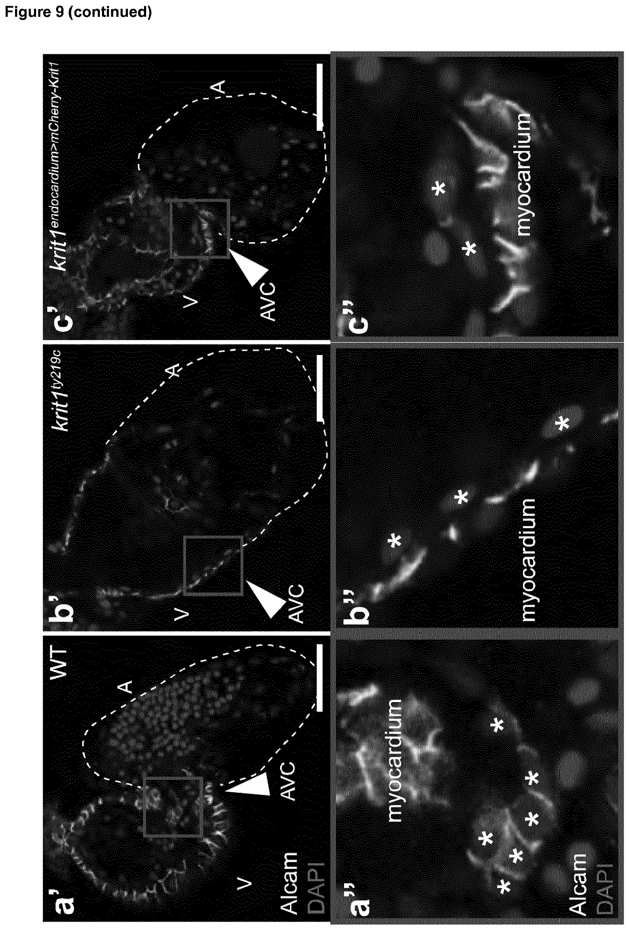

**Figure 9.** *TgBAC(nfatc1:GAL4FF)*<sup>mu286</sup>-*driven endocardial expression of Tg(UAS:mCherry-Krit1)*<sup>md40</sup> *restores endocardial morphology at the atrioventricular canal.*

**a-c**, Embryonic hearts of different genotypes at 54 hpf; shown are projection images based of several confocal microscopy z-stack images. The myocardial cells are outlined by junctional marker Alcam (a-c, black; a'-c', white). The narrowing of the atrioventricular canal (AVC, arrowhead), which is separating the cardiac chambers ventricle (V) and atrium (A) as seen in wild-type (**a**) is lost in *krit1*<sup>ty219c</sup> mutants (**b**) and restored in *krit1*<sup>endocardium>mCherry-Krit1</sup> embryos (**c**). **a'-c''**, Single confocal plane sections reveal that endocardial AVC cells (asterisks) express Alcam in wild-type (**a', a''**), whereas in *krit1*<sup>ty219c</sup> this Alcam expression is lost (**b', b''**). **c', c''**, In *krit1*<sup>endocardium>mCherry-Krit1</sup> embryos, endocardial cell morphology and Alcam expression at the endocardial AVC cells are restored (asterisks). Scale bars: 50 $\mu$m.

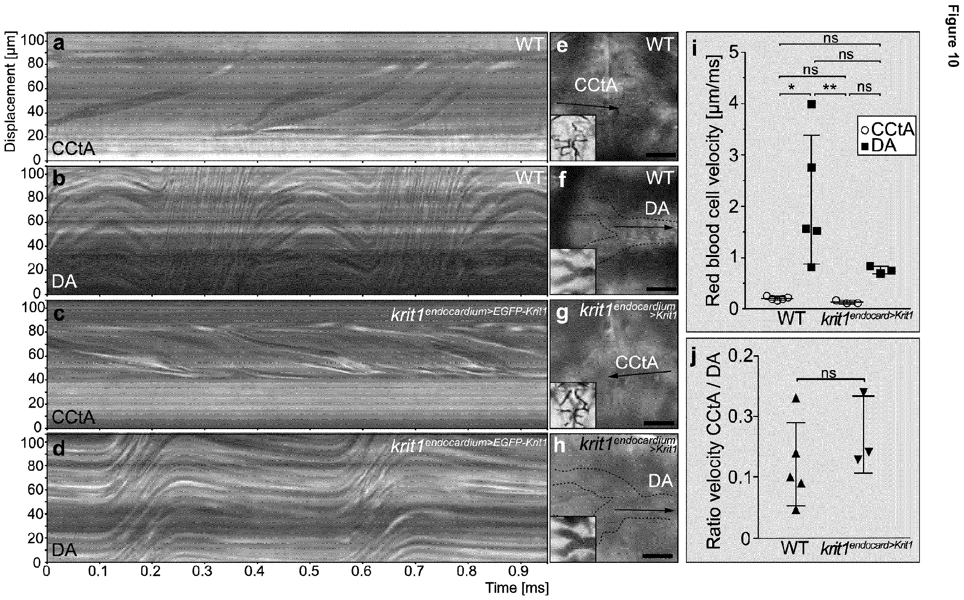

**Figure 10.** *Blood flow patterns in krit1*<sup>endocardium>mCherry-Krit1</sup> *are restored.*

**a-d**, Kymographs depicting the displacement (y-axis, $\mu$m) of red blood cells over time (x-axis, milli seconds, ms). **e-h**, Single confocal sections with differential interference contrast of blood vessels used for kymographs. The arrows indicate the length and position of the line-scan and the direction of blood flow. Insets show projections that are based on inverted black/white confocal z-stack images of 70 kDa Texas-Red-conjugated dextran tracer dye. **i**, Quantification of red blood cell velocities in the CCtA and DA in wild-type and *krit1*<sup>endocardium>mCherry-Krit1</sup> embryos at 56-60 hpf. Kruskal-Wallis test with s.d.; *$P$=0.0468; **$P$=0.0057; ns, not significant. **j**, Quantitative relation of the red blood cell velocity in the CCtA compared with the DA within each embryo shows no significant difference between wild-type and *krit1*<sup>endocardium>mCherry-Krit1</sup> embryos. Unpaired t-test with s.d.; ns, not significant ($P$=0.9739). Scale bar: **e-h**, 50 $\mu$m.

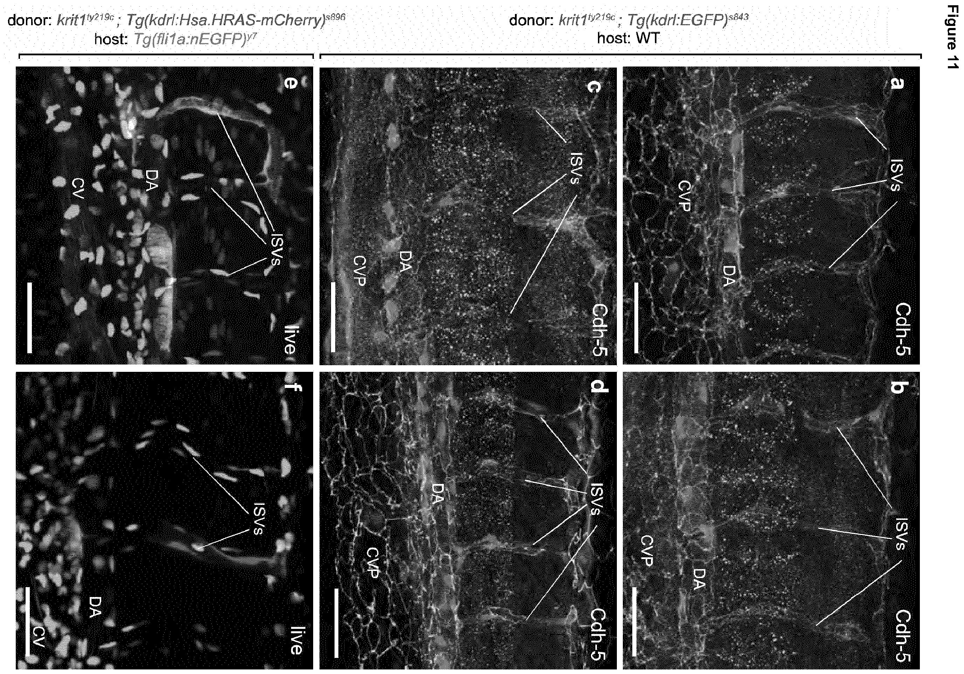

**Figure 11.** *krit1*<sup>ty219c</sup> *endothelial cells contribute to the formation of patent blood vessels.*

**a-d**, Projection images based on several confocal microscopy z-stack images of vessel beds with transplanted, Krit1-deficient (*krit1ty219c*) endothelial cells expressing *Tg(kdrl:EGFP)s843*, false-colored in magenta, which integrate normally within different vessel beds of the wild-type host. Endothelial cells are marked by immuno-labelling of VE-Cadherin (Cdh-5, white labelling). **g**, **h**, Live-imaging of Krit1-deficient endothelial cells. Intersegmental vessels that harbor Krit1-deficient cells do not collapse, but sustain normal ISV morphology in the presence of blood flow. ISVs, intersegmental vessels; DA, dorsal aorta; CV, caudal vein; CVP, caudal vein plexus. Scale bar: 50 μm.

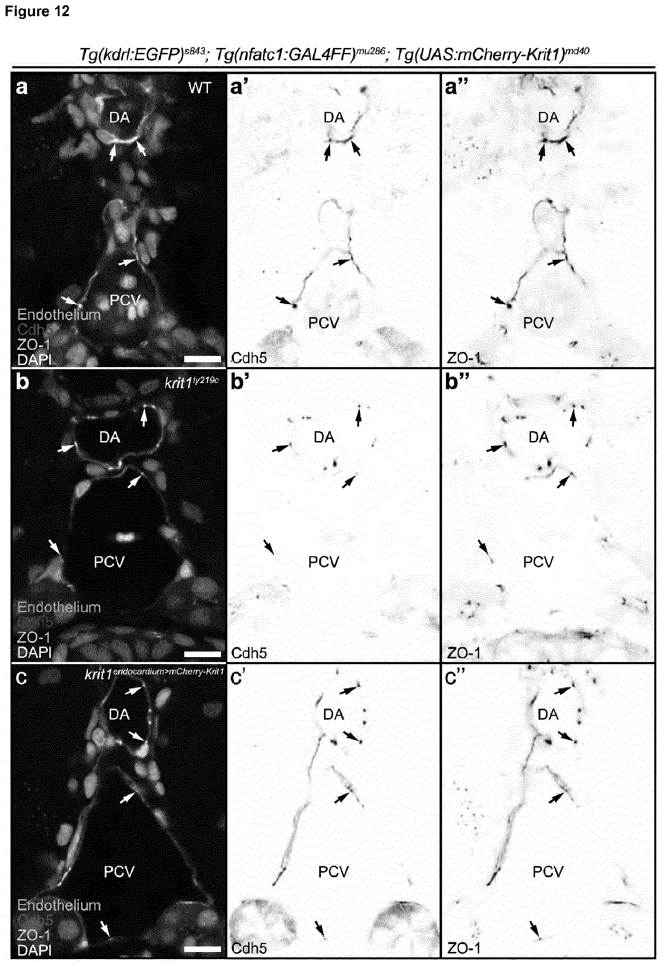

**Figure 12.** *krit1endocardium>mCherry-Krit1 embryos show normal localization of adherens and tight junction markers, Cdh-5 and ZO-1, respectively.*

**a-c**, Single confocal z-planes transversal section in trunk region, immunohistochemically stained for cell-cell junctions, which are marked by Cdh-5 (magenta) and ZO-1 (yellow). Nuclei are stained with DAPI (white) and endothelial cells are marked by transgenic expression of *Tg(kdrl:EGFP)s843* (false-colored in cyan). At the cell-cell junctions, Cdh-5 (**a'-c'**) and ZO-1 (**a"-c"**) labelling appears unchanged in *krit1ty219c* (**b'**, **b"**) and *krit1endocardium>mCherry-Krit1* embryos (**c'**, **c"**) as compared to wildtype (**a'-a"**).

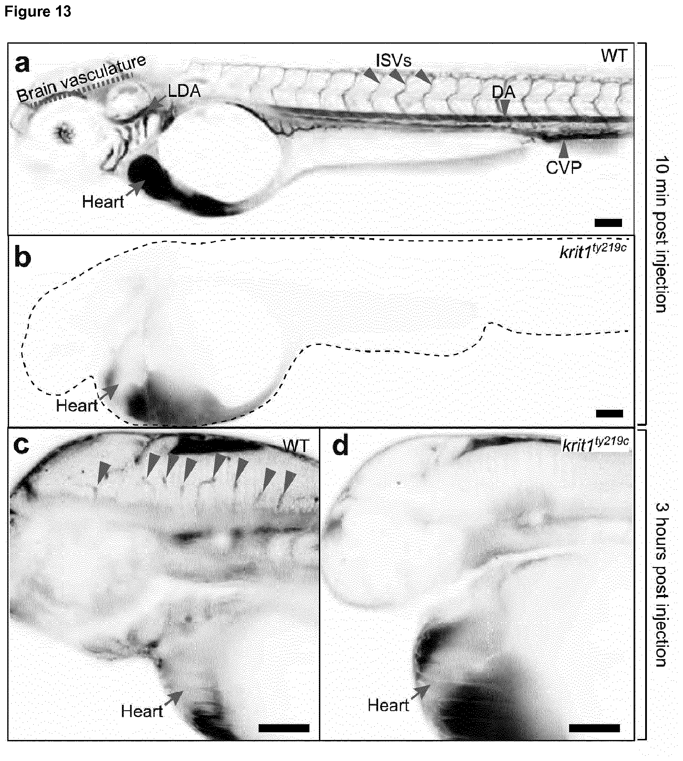

**Figure 13.** *Microangiography in krit1ty219c mutants.*

**a-d**, Lateral views of projection images based on inverted black/white confocal z-stack images of the tracer dye in wild-type (**a**) and *krit1ty219c* (**b**) embryos at 56 hpf. **a**, Immediately after injection, 70 kDa Texas-Red-conjugated dextran spreads rapidly throughout the entire vasculature, outlining all vessels, whereas in *krit1ty219c* embryos (**b**) the tracer dye distributes only to the dilated heart where it remains. **c**, 3 hours after injection, most of the tracer dye has diffused to surround tissues, also filling intercellular spaces such as brain ventricles. However tracer dye circulating is still visible in the vasculature such as the cerebral vessels (red arrowheads), **d**, In contrast, in *krit1ty219c* embryos, the tracer remains located in the heart and diffusion occurs to a lesser degree due to the lack of active circulation.

## EXAMPLES

The invention is further described by the following examples. These are not intended to limit the scope of the invention, but represent preferred embodiments of aspects of the invention provided for greater illustration of the invention described herein.

## Introduction

Hemodynamic forces play an important role in shaping the cardiovascular system[1-2]. The biomechanical forces induced by blood flow are dependent on factors such as vessel diameter and geometry, or blood viscosity and differ in regions closer to the heart than in more peripheral regions[3,4]. These differences trigger gradations in levels of biomechanical signaling in endothelial cells of distinct vascular beds and have consequences on the cells' orientation, migration, and proliferation[1]. Pathological changes in biomechanical signaling pathways or abnormal patterns of blood flow have been implicated in the etiology of various vascular diseases[2] including cerebral cavernous malformations (CCMs)[5-9]. Familial forms of CCM occur with a prevalence of ~0.5 % within the general population[10] and are mainly caused by a two-hit mechanism[11] whereby heterozygous carriers with a germline mutation in one allele of either *CCM1/KRIT1*(Kev Interaction Trapped Protein 1)[12], *CCM2/MGC4607*, or *CCM3/PDCD10* (Programmed Cell Death 10)[13] acquire a second somatic mutation in the other allele[14]. The CCM defects occur mainly within the cerebrovasculature and patients have a continuous risk of neurological deficits and hemorrhagic stroke[10]. Several lines of evidence point at a role of blood flow in the etiology of CCM lesions: (1) CCM lesions are mostly restricted to venous blood vessels that are perfused at low levels in patients[15]; (2) a loss of CCM protein functions causes a strong upregulation of KLF2/4, which are blood-flow sensitive mediators of biomechanical signaling within endothelial cell[5,10,11,217,16-18]; (3) a knockdown of Klf2a/b suppresses cardiovascular malformations in zebrafish CCM mutants[7]; and (4) the formation of lesions in the cerebrovasculature in conditional endothelial-specific *CCM1* or *CCM2* mouse knock-outs is suppressed by knock-outs of *KLF2/4*[16-18]. Although all of these data suggest that blood flow plays an important role in the CCM pathology, how blood flow modulates the formation of vascular anomalies in CCM mutant tissues has remained unclear. Here we use the zebrafish model of CCM1 to explore the role of blood flow within high shear stress vascular bed, the lateral dorsal aorta.

## Methods of the Examples

### Zebrafish genetics and maintenance.

Handling of zebrafish was performed in compliance with German and Brandenburg state law, carefully monitored by the local authority for animal protection (LUVG, Brandenburg, Germany; Animal protocol #2347-18-2015). The following strains of zebrafish were maintained under standard conditions as previously described[19]: *krit1ty219c* [20,21], *Tg(kdrl:EGFP)s843* [22], *Tg(fli1a:nEGFP)y723*, *Tg(kdrl:Hsa.HRAS-mCherry)s896* [24], *Tg(YFP)mu107* [25],

*Tg(fli1a:GAL4FF)[ubs3][26]*, *TgBAC(nfatc1 :GAL4ff)[mu286][27]*, *Tg(UAS:EGFP-Kritl, cryaa:EGFP)[pbb2l]* [referred to as *Tg(UAS:EGFP-Kritl)[pbb21]*][28], *Tg(UAS:Lifeact-RFP)[mu262]* (gift from W. Herzog).

*Molecular cloning and generation of transgenic zebrafish.*

We used an existing Gateway pDONR221 vector containing the open reading frame of zebrafish *krit1* (referred to as pME-Krit1)[29] to generate an N-terminal mCherry fusion construct pME-mCherry-Krit1. This middle-entry construct was used in combination with the Gateway plasmids pDestTol2 and p5E-UAS[29,30] and p3E-cryaa:EGFPpA[28] to generate the final construct pDestTol2pA-UAS:mCherry-Krit1,cryaa:EGFP by standard Gateway cloning recombination reactions. pDestTol2pA-UAS:mCherry-Krit1,cryaa:EGFP (25 pg/embryo) was co-injected with mRNA encoding Tol2 transposase (50 pg/embryo) into one-cell-stage zebrafish embryos and F0 fish were screened for germline transmission. The transgenic line *Tg(UAS:mCherry-Krit1,cryaa:EGFP)[md40]* [referred to as *Tg(UAS:mCherry-Krit1)[md40]*] was selected for further analyses.

*Endothelial and endocardial rescue experiments.*

For endothelial rescue experiments based on restoration of blood flow in *krit1[ty219c]* mutants, expression from the stable *Tg(UAS:EGFP-Krit1)[pbb21]* or *Tg(UAS:mCherry-Krit1)[md40]*transgenes was driven using the pan-endothelial Gal4 driver line *Tg(fli1a:GAL4FF)[ubs3]* in *krit1[ty219c]* mutant embryos (referred to as *krit1[endothelium>EGFP-Krit1]* and *krit1[endothelium>mcherry-Krit1]*). The rescued embryos were genotyped for presence of the transgenic Gal4 driver, the UAS responder constructs and the *krit1[ty219c]* mutant allele. For endocardial rescue experiments, we used a comparable strategy using the *TgBAC(nfatc1 :GAL4ff)[mu286]* driver line to express the stable *Tg(UAS:mCherry-Krit1)[md40]* transgene in *krit1[ty219c]* mutant embryos (referred to as *krit1[endocardium>mCherry-Krit1]*). Rescued *krit1[ty219c]* embryos usually presented with mild blood pooling at the inflow tract. Those embryos were pre-selected at 48 hours post fertilization (hpf) and directly processed for further experiments. Subsequently, the presence of transgenic Gal4 driver, UAS responder constructs, and *krit1[ty219c]* allele was tested by molecular genotyping.

*Antisense oligonucleotide morpholino treatment.*

All antisense oligonucleotide morpholinos were obtained from Gene Tools and injections were performed at the one-cell-stage as previously described[31]. The following amounts were used: *tnnt2a* (5'-CATGTTTGCTCTGATCT-GACACGCA-3[1])[31] 2 ng/embryo.

*Microangiography and live imaging.*

Embryos were treated with 1-phenyl-2-thiourea (PTU) (Sigma Aldrich) to inhibit the appearance of pigmentation. Embryos were manually dechorionated and embedded laterally or dorsally in 1% low melting agarose (Lonza 50081) containing 0.16 mg/ml Tricaine in glass-bottom dishes (MatTek). Microangiography was performed as previously described[32]. 5 $\mu$g/$\mu$l Dextran TexasRed (70 kDa, D1864, Invitrogen) was injected into the common cardinal vein (CCV) at 54-60 hpf and immediately processed for optical sectioning with a LSM 710 confocal microscope (Zeiss) using a 10x objective for lateral overviews and 20x objective for dorsal views.

*Statistical analysis of genetic test crosses for krit1[endothelium>EGFP-Krit1].*

The *krit1[ty219c/+]* allele harbors a recessive mutation leading to embryonic lethality by 5 day post fertilization (dpf). For the statistical analysis, three independent single pair crosses using adult zebrafish of the genotype *krit1[ty219c/+]; Tg(fli1a:GAL4FF)[ubs3/+]* and *krit1[ty219c/+]; Tg(UAS:EGFP-Krit1)[pbb21/+]* were performed. The progeny of those crosses was screened for endothelial expression of EGFP-Krit1 at 24 hpf and raised separately from embryos without EGFP-Krit1 expression. At 5 dpf, embryos were assessed by morphological inspection and considered wild-type when they presented normal blood flow, a defined atrioventricular canal, and two separated and non-ballooned cardiac chambers, all phenotypes characteristically lost in *krit1* mutants. Figure 6d shows the means of the percentages of the occurrence of phenotypically wild-type embryos at 5 dpf within each group among the progeny of three independent crosses with s.d.. Prism 7 (GraphPad) was used to perform an unpaired, two-tailed t test (*P*=0.0341). Means are statistically significantly different when *P* < 0.05.

*Statistical analysis of lateral dorsal aorta cell numbers.*

For the statistical analysis of the endothelial cell numbers in Figure 1h, lateral dorsal aorta cell nuclei were visualized based on 3D reconstructions of confocal microscopy z-stack images using Imaris V7.7.2 software (Bitplane, UK). The *Tg(kdri.-GFP)$^{s843}$* expression was used to count endothelial cell numbers in an area defined between the branching point of the dorsal aorta and the branching point of the first aortic arch. Cell numbers are shown as means with s.d.. Prism 7 (GraphPad) was used to perform Shapiro-Wilk normality test and one-way ANOVA tests followed by Tukey's multiple comparisons tests. Means are statistically significantly different when $P < 0.05$.

*Statistical analysis of the proximal dorsal aorta diameter.*

The statistical analysis of the diameter of the dorsal aorta in Figure 1i was assessed based on maximum projections of confocal microscopy z-stack images using Fiji software to quantify one measure point (diameter in $\mu$m) per proximal dorsal aorta and embryo. Figure 1i shows means with s.d.. Prism 7 (GraphPad) was used to perform Shapiro-Wilk normality test and one-way ANOVA tests followed by Tukey's multiple comparisons tests. Means are statistically significantly different when $P < 0.05$.

*Statistical analysis of the cerebral central arteries diameter*

The statistical analysis of the diameter of the cerebral central arteries (CCtAs) in Figure 3f was assessed based on maximum projections of confocal microscopy z-stack images using Fiji software. Shown the average values of 8-10 measurements (diameter in $\mu$m) of a single CCtA per embryo and means with s.d. from a total of three independent experiments (9-12 embryos per condition). Prism 7 (GraphPad) was used to perform D'Agostino and Pearson normality test and unpaired, two-tailed t-test with Welch's correction. Means are statistically significantly different when $P < 0.05$.

*Statistical analysis of endothelial cell shapes within the lateral dorsal aorta.*

The statistical analysis of endothelial cell morphologies within the lateral dorsal aorta in Figure 2m, n was assessed based on maximum projections of confocal microscopy z-stack images of immunohistochemically stained endothelial cells marked with an anti-VE-Cadherin (Cdh-5) antibody. Using Imaris and the Freehand tool of Fiji Software, the outlines of five endothelial cells of each lateral dorsal aorta were taken and measured for surface area and perimeter values. The circularity of endothelial cells was determined according to the following equation

$$Circularity = 4\pi(area/perimeter^2)$$

(with a perfect circle equals 1). Prism 7 (GraphPad) was used to perform D'Agostino and Pearson normality test and one-way ANOVA test followed by Tukey's multiple comparisons test.. Means are statistically significantly different when $P < 0.05$.

*Statistical analysis of the fluorescent intensity of klf2a: YFP expression in the lateral dorsal aorta.*

For the statistical analysis of the fluorescence intensity of the transgenic expression of *Tg(klf2a:YFP)$^{mu107}$*in Figure 2e, a region within the lateral dorsal aorta was outlined using the transgenic expression of *Tg(kdri:Hsa.HRAS-mCherry)$^{s896}$* based on maximum projections of 5 confocal microscopy z-stack images. Using Fiji Measure Tool, the mean *Tg(klf2a:YFP)$^{mu107}$* signal intensity was assessed. Figure 2e shows means with s.d. and Prism 7 (GraphPad) was used to perform statistical analyses and normal distribution was tested using Shapiro-Wilk normality test. For the analysis between wild-type, *krit1$^{ty219c}$* mutants and *krit1$^{endocardium>mCherry-Krit1}$*, a one-way ANOVA tests followed by Tukey's multiple comparisons test was performed. For the analysis between wild-type and *tnnt2a* morphants, an unpaired, two-tailed t-test with Welch's correction was used. Means are statistically significantly different when $P < 0.05$.

*Statistical analysis of endocardial cell numbers.*

For the statistical analysis of the endocardial cell numbers in Figure 5p, endocardial cell nuclei were visualized based on 3D reconstructions of confocal microscopy z-stack images using Imaris V7.7.2 software (Bitplane, UK). Expression of *Tg(kdri.-GFP)$^{s843}$* was used to count endocardial cell numbers, shown as means with s.d.. Prism 7 (GraphPad) was used to perform D'Agostino and Pearson normality test and unpaired, two-tailed t-test with Welch's correction. Means are statistically significantly different when $P < 0.05$.

*Statistical analysis of intersegmental vessel area.*

For the statistical analysis of intersegmental vessel (ISV) areas as shown in Figure 5q, ISVs were visualized based on maximum projections of several confocal microscopy images using Fiji software. The *Tg(kdri.-GFP)*[s843] expression was used to outline and measure the area of at least three ISVs per embryo (n=5-6 per condition) using Fiji software tools and are shown as means with s.d. in Figure 5q.

Prism 7 (GraphPad) was used to perform D'Agostino and Pearson normality test and one-way ANOVA test followed by Tukey's multiple comparisons (Figure 5q, left panel) and unpaired, two-tailed t-test with Welch's correction (Figure 5q, right panel). Means are statistically significantly different when $P < 0.05$.

*Kymographs and statistical analysis.*

Embryos were mounted as described for microangiography. Kymographs were generated using differential interference contrast (DIC) and by selecting a line-scan of 106.26 $\mu$m (0.415 $\mu$m per pixel on the y-axis), positioned parallel to the direction of blood flow, and with 1000 cycles covering a time span of 945.45 ms (0.945 ms per pixel on the x-axis). The slope generated by the displacement of the DIC signal translates into the velocity of blood cells as in m = $[\tan(\alpha)](0.415 \mu m/0.945 ms)$. At least three kymographs per vessel (CCtA and DA, respectively) and embryo were generated (wild-type, n=5; *krit1*[endocardium>mCherry-Krit1], n=3). Figure 10i shows blood flow velocity means with s.d.. Prism 7 (GraphPad) was used to perform D'Agostino and Pearson normality test, showing normal distribution for samples CCtA-WT and *CCtA-krit1*[endocardium>mCherry-Krit1]. Statistical analysis was performed using Kruskal-Wallis test and Dunn's multiple comparison test; means are statistically significantly different when $P < 0.05$. Figure 10j shows the ratio of the blood flow velocity in the CCtA over the blood flow velocity in the DA within each embryo with s.d.. Prism 7 (GraphPad) was used to perform Shapiro-Wilk's normality test and an unpaired, two-tailed t test (P=0.9739).

*Cell transplantations.*

Cell transplantations were performed essentially as described[33]. Approximately 10-20 cells from *kritl*[^190] mutants marked by transgenic expression of *Tg(kdrl:EGFP)*[s843] were removed at sphere stage (4-5 hpf) and transplanted into age-matched wild-type host embryos (Figure 11a-d) or *krit1*[ty219c] mutants marked by transgenic expression of *Tg(kdril:Hsa.HRAS-mCherry)*[s896] were transplanted into wild-type embryos marked with *Tg(fli1a:nEGFP)*[y7] (*Figure* 11e, f). Donor genotypes were determined based phenotypes at 48 hpf or by molecular genotyping. The visual inspection of cell shapes of transplanted cells was performed based on identifying the transgenic expression of *Tg(kdri.-EGFP)*[s843] or *Tg(kdri:Hsa.HRAS-mCherry)*[s896] combined with immuno-labelling against the cell-junctional marker Cdh-5.

*Sample preparation and mass spectrometry.*

Pooled protein extracts (~25pg/sample) from 2 days post fertilization (dpf) zebrafish embryonic tails were mixed with SDS sample buffer and boiled at 95 °C for 10 min. Each sample was derived from approximately 30 embryos and experiments were performed in biological triplicates. Within each replicate, wild-type and *krit1*[ty219c] mutant embryos were siblings derived from one clutch that were treated in parallel. Subsequently, the samples were separated side-by-side by SDS-PAGE (Tris-glycine gradient gel, 4-20%, Invitrogen). Whole gel lanes of corresponding samples were cut into 28 bands and in-gel digestion was performed using $^{16}O/^{18}O$-labeling as previously described[34,35]. In brief, samples were incubated over night at 37 °C with 50 ng trypsin (sequencing grade modified, Promega) in 25 $\mu$L of 50 mM ammonium bicarbonate. Wild-type samples were digested in the presence of heavy water (Campro Scientific GmbH, 97% $^{18}O$), whereas *krit1*[ty219c] and *tnnt2a* morphant samples were digested in the presence of regular $^{16}O$-water. To prevent oxygen back-exchange by residual trypsin activity, samples were heated at 95 °C for 20 min. After cooling down, 50 $\mu$L of 0.5% TFA in acetonitrile was added to decrease the pH of the sample from ~8 to ~2. Afterwards, corresponding heavy- and light-isotope samples were combined and peptides were dried under vacuum. Peptides were reconstituted in 10 $\mu$L of 0.1% (v/v) TFA, 5% (v/v) acetonitrile and 6.5 $\mu$L were analyzed by a reversed-phase capillary nano liquid chromatography system (Ultimate 3000, Thermo Scientific) connected to an Orbitrap Velos mass spectrometer (Thermo Scientific). LC separations were performed on a capillary column (Acclaim PepMap100 C18, 2 $\mu$m, 100 Å, 75 $\mu$m i.d. $\times$ 25 cm, Thermo Scientific) at an eluent flow rate of 300 nL/min. The column was pre-equilibrated with 3% mobile phase B followed by a linear increase of 3-50% mobile phase B in 50 min. Mobile phase A contained 0.1% formic acid in water, and mobile phase B contained 0.1% formic acid in acetonitrile. Mass spectra were acquired in a data-dependent mode utilizing a single MS survey scan (m/z 350-1500) with a resolution of 60,000 in the Orbitrap and MS/MS scans of the 20 most intense precursor ions in the linear trap quadrupole.

*Proteomic data analysis.*

Identification and quantification of $^{16}O/^{18}O$-labeled samples was performed using the Mascot Distiller Quantitation Toolbox (version 2.6.3.0) and Mascot Server (version 2.5.0, Matrix Science). Experimental data was searched against the SwissProt *Danio rerio* protein database (April 15, 2017, 43.539 sequences). A maximum of two missed cleavages was allowed and the mass tolerance of precursor and sequence ions was set to 10 ppm and 0.35 Da, respectively. Methionine oxidation, acetylation (protein N-terminus), propionamide (C), and C-terminal $^{18}O_1$- and $^{18}O_2$-isotope labelling were used as variable modifications. A significance threshold of 0.05 was used based on decoy database searches. For quantification at protein level, a minimum of two quantified peptides and a maximum standard deviation of 3 were set as thresholds. Relative protein ratios were calculated from the intensity-weighted average of all peptide ratios. Known contaminants (e.g. keratins) were removed from the protein output tables. Normalization of protein ratios was performed using the median ratio of all proteins. Only proteins that were identified and quantified in at least 2 out of 3 biological replicates are reported in table S2. Proteins with a consistent minimum fold change of >1.5 (in each replicate) were considered as affected. Proteins with conflicting ratios between biological replicates were considered as unchanged.

*Immunohistochemistry and imaging*

Zebrafish whole-mount immunohistochemistry was performed as previously described[28]. The following antibodies were used: rabbit anti-VE-Cadherin (Cdh-5) (1 :200, gift from M. Affolter[36]); mouse anti-Alcam (1:200, DSHB AB _531904); mouse anti-ZO-1 (1:200, Life Technologies 33-9100). Secondary antibodies were Alexa Fluor 633-conjugated goat anti-rabbit (1 :200, Invitrogen A21070); FITC-conjugated donkey anti-mouse (1:200, Jackson ImmunoResearch 715-095-150); goat anti-rabbit DyLight 405 (1 :200, Jackson ImmunoResearch 123-475-021); goat anti-mouse Dylight 649 (1:200, Jackson ImmunoResearch 205-492-176). Embryos were fixed with 2% PFA overnight and permeabilized with PBST with 0.5% Triton X-100 for 1 hour and subsequently incubated with primary antibody diluted in PBST with 0.2% Triton X-100, 1% BSA, and 5% NGS. All specimens were mounted in SlowFade Gold (Invitrogen S36936). Images were recorded on LSM 710 or LSM 780 confocal microscopes (Zeiss) and processed with Imaris V7.7.2 (Bitplane, UK) and Fiji software.

**Results of the examples**

In animal models, a complete loss of CCM proteins disrupts cardiac function[7,16,20,21,37] and vascular anomalies develop not only in small venous vascular beds, as in human patients, but also within the endocardium, aorta, and other major blood vessels[,7,16,17,37,38]. The role of blood flow as an essential stimulus for correct cardiovascular development is poorly understood in the context of CCM vascular defects[5-7]. Hence we wondered whether the vascular anomalies in high shear stress vascular beds such as the zebrafish aorta[3,4] only developed because CCM mutant endothelial cells were lacking blood flow as a biomechanical stimulus.

Zebrafish mutants lacking blood flow, such as *troponin t2a (tnnt2a)* mutants that are defective in an essential regulatory sarcomeric protein required for cardiac contractility, can survive for several days; this has permitted longitudinal studies into the developmental consequences of lacking blood flow[31]. To analyze the effects of a lack of hemodynamic forces on vascular patterning, we compared the vessels of *tnnt2a* morphants in which blood flow is absent with those in *krit1ty219c* mutants[20,21] (Fig. 1a-c). The zebrafish *krit1ty219c* mutants form massively dilated hearts that fail to produce blood flow and are marked by increased endocardial cell numbers as compared to wild-type (Fig. 5a, b, p). The *krit1ty219c mutant* vasculature is also characterized by collapsed intersegmental vessels, dilated and fused caudal venous plexus (Fig. 1a, b; Fig. 5f, g, q), and oversprouting cerebrovasculature (Fig. 5k, l). The *tnnt2a* morphant animals had anomalies comparable to *krit1ty219c* mutants in some smaller endothelial beds including intersegmental vessels, the caudal venous plexus, and the brain vasculature (Fig. 5f-h, k-m). However, in striking contrast to the massive hyperplasia of both endocardium and lateral dorsal aorta in *krit1ty219c* mutants (Fig. 1d, e, h; Fig. 5a, b)[7], *tnnt2a* morphants developed a smaller endocardium[39] (Fig. 5c) and lateral dorsal aorta (Fig. 1f). This indicates that the vascular anomaly of the lateral dorsal aorta in *krit1ty219c* mutants does not simply result from a loss of blood flow, but rather from a combined effect of loss of Krit1 and absence of blood flow. One way of testing this hypothesis would be to restore blood flow in *krit1ty219c* mutants and to observe whether this suppressed the vascular malformation of the lateral dorsal aorta.

To restore blood flow in *krit1ty219c* mutants, we first generated transgenic lines of zebrafish for the tissue-specific rescue with functional EGFP-Krit1[28] or mCherry-Krit1 fusion proteins. Expression from the stable *Tg(UAS:EGFP-Kritl)pbb21* or *Tg(UAS:mCherry-Krit1)md40* transgenes was driven using the pan-endothelial Gal4 driver line *Tg(fli1a:GAL4FF)ubs3* in *krit1ty219c* mutant embryos (referred to as *krit1endothelium>EGFP-Krit1* and *krit1endothelium>mCherry-Krit1*) (see Materials and Methods for a more detailed explanation of the experimental procedure). This restored early cardiovascular development including the morphology of the endocardial chambers and atrioventricular canal (AVC), lumenization of intersegmental vessels, network formation of caudal venous plexus, and normal angiogenesis of cerebral vasculature (Fig. 5d, i, n). Accordingly, blood flow was re-established, and *krit1*[endothelium>EGFP-Krit1] or *krit1*[endothelium>mCherry-Krit1] embryos were phenotypically normal until 5 days post-fertilization (dpf) (Fig. 6). In line with previous observations from other cellular model systems, the EGFP-Krit1 and mCherry-Krit1 fusion proteins localized also to cell junctions within zebrafish endothelial cells (Fig. 7)[40-42]. Hence, pan-endothelial expression of EGFP-Krit1 or mCherry-Krit1 in *krit1*[ty219c] mutants rescues heart morphology and function and also permits a proper vascular development throughout embryogenesis.

To address whether a restoration of blood flow would affect Krit1-deficient endothelial cells, we used a Gal4 driver line *TgBAC(nfatc1:GAL4FF*[mu286]) which is not expressed within endothelial cells outside of the heart. Within the cardiovascular system, this transgenic driver line expresses Gal4 only within endocardial cells, an expression domain which is restricted mostly to the atrioventricular canal (AVC) between 54-78 hpf (Fig. 8a, d), but which is not present within any other endothelial tissue (Fig. 8b, c, e, f). This transgenic driver line was used to express mCherry-Krit1 within the *krit1*[ty219c] mutant endocardium (referred to as *krit1*[endocardium>mCherry-Krit1]). This resulted in embryos whose hearts were functionally rescued including AVC morphology (Fig. 5e; Fig. 9) and that produced blood flow until 3-4 dpf within an endothelium that was otherwise depleted of Krit1. To further characterize blood flow conditions in *krit1*[endocardium>mCherry-Krit1] embryos, we quantified the pattern and the velocity of blood flow in cerebral arteries versus the dorsal aorta. While the blood flow pattern in the dorsal aorta was generally more rapid and highly pulsatile, within smaller vessels of the cerebral central arteries (CCtAs) in *krit1*[endocardium>mCherry-Krit1] and wild-type it was laminar and slower (Fig. 10a-i). Importantly, the quantitative differences in blood flow velocities between these two vascular beds within *krit1*[endocardium>mCherry-krit1] embryos were similar to those in wild-type embryos (Fig. 10j.).

Strikingly, the morphology of the lateral dorsal aorta and its numbers of endothelial cells as well as the diameter of the adjacent dorsal aorta were completely restored in *krit1*[endocardium>mCherry-Krit1] embryos (Fig. 1g-i; n=4). We next set out to investigate whether the restoration of the lateral dorsal aorta morphology in *krit1*[endocardium>mCherry-Krit1] embryos was due to changes of endothelial cell morphologies compared to *krit1*[ty219c] mutants at 54-60 hpf. To quantify endothelial cell morphologies, we counterstained embryos with an antibody against Cdh-5. This revealed that wild-type endothelial cells of the lateral dorsal aorta were aligned with the direction of blood flow and had moderate surface area sizes (Fig. 1j, m, n; n= 3) while *krit1*[fy219c] mutant endothelial cells had enlarged surface area sizes and were highly elongated (less circularity) (Fig. 1k, m, n; n=3). Upon restoration of blood flow in *krit1*[endocardium>mCherry-Krit1] embryos, endothelial cell elongation was normalized whereas cell surface areas were not reduced (Fig. 1l, m, n; n=3). We conclude that blood flow suppresses several aspects of vascular anomalies that are characteristic of the lateral dorsal aorta and the adjacent dorsal aorta in *krit1*[ty219c] mutants. For instance, blood flow reverts the striking elongation phenotype of lateral dorsal aorta endothelial cells in *krit1*[ty219c] mutants.

Previous studies showed that vascular defects due to the loss of CCM proteins involve pathological KLF2/4 signaling[7,16-18]. This triggers an endothelial-to-mesenchymal transition (endMT) within lowly-perfused blood vessels[18,43,44], a process common to many vascular pathologies[45]. To assess whether Klf2a expression was altered also within the lateral dorsal aorta, we used a transgenic *klf2a* reporter line[25]. Quantifications using this reporter line revealed that in comparison to wild-type, *krit1*[ty219c] mutants strongly expressed *klf2a* within the lateral dorsal aorta, although these embryos lack blood flow (Fig. 2a, b, e). This was different from *tnnt2a* morphants which also lack blood and have a strongly reduced *klf2a* reporter expression[46] (Fig. 2c, e). Restoration of blood flow in *krit1*[endocardium>mCherry-Krit1] embryos did not alter the high levels of *klf2a* expression (Fig. 2d, e). We conclude that since *krit1*[endocardium>mCherry-Krit1] embryos lack a hyperplastic lateral dorsal aorta, high expression levels of Klf2a are apparently not sufficient to cause this vascular anomaly when blood flow is present.

Other blood vessels that are normally affected in *krit1*[ty219c] mutants were also rescued in *krit1*[endocardium>mCherry-Krit1] embryos as assessed by morphological inspection; this included intersegmental vessels, which were lumenized and the caudal venous plexus that shaped into a network-like system (Fig. 5j, q). In a complementary approach, *krit1*[ty219c] mutant endothelial cells that were transplanted into wild-type embryos contributed to intersegmental blood vessels which were also correctly lumenized (Fig. 11; n=6 host embryos with transplanted cells). Furthermore, the oversprouting brain vasculature phenotype in *krit1*[ty219c] mutants was markedly reduced in *krit1*[endocardium>mCherry-Krit1] embryos (Fig. 5l, o).

Immunohistological stainings against adherens and tight junction markers Cdh-5 and ZO-1 in the dorsal aorta and the caudal vein plexus in *krit1*[endocardium>mCherry-Krit1] embryos were unchanged as compared to *krit1*[ty219c] or wild-type embryos (Fig. 12). These data indicated that the barrier function of the endothelial vasculature was intact within most blood vessels and preventing the tracer dye from leakage into the surrounding tissue. To further corroborate this finding, we performed mass spectrometric analyses of protein samples derived from tail regions of wild-type and *krit1*[ty219c] mutant embryos at 2 dpf. In tune with the observation that most blood vessels were tight against leakiness, detectable cell junctional proteins in *krit1*[ty219c] mutants did not significantly differ in their protein levels relative to wild-type (*Table 3*).

*Table 3. List of known adherens and tight junctional proteins and their relative changes in krit1<sup>ty219c</sup> mutant tissues compared to wild-type at 2 dpf determined using quantitative mass spectrometry.*

| Accession | Gene Name | Replicate | wild-type / mutant protein levels | SD (geo) | number quantified of peptides |
|---|---|---|---|---|---|
| Q9PVF8_DANRE | ctnna1 | A | 1.088 | 1.087 | 20 |
| | | B | 1.026 | 1.045 | 25 |
| | | C | 1.319 | 1.036 | 18 |
| CTNA2_DANRE | ctnna2 | A | 1.052supp | 1.115 | 3 |
| | | B | 1.053 | 1.095 | 8 |
| | | C | 1.124 | 1.095 | 4 |
| A0A0N4SUA5_DANRE | ctnnb1 | A | 0.668 | 1.153 | 12 |
| | | B | 0.981 | 1.046 | 14 |
| | | C | 1.175 | 1.205 | 8 |
| E7FC99_DANRE | ctnnb2 | C | 1.079 | 1.296 | 6 |
| E7F2W2_DANRE | tjp1a | A | 0.978 | 1.163 | 2 |
| | | B | 1.166 | 1.176 | 3 |
| | | C | 0.750 | 1.086 | 4 |
| A0A0R4IHR7_DANRE | tjp1b | B | 1.171 | 1.239 | 3 |
| | | C | 0.730 | 1.04 | 5 |
| F6PEN0_DANRE | tjp2a | A | 0.727 | 1.088 | 4 |
| | | B | 1.160 | 1.089 | 10 |
| | | C | 1.023 | 1.083 | 8 |
| B0UXH8_DANRE | tjp2b | B | 0.795 | 1.158 | 5 |
| E9QGL7_DANRE | tjp3 | B | 0.899 | 1.378 | 3 |
| | | C | 0.749 | 1.028 | 2 |

The apparent phenotypic restoration of vascular morphology in *krit1<sup>endocardium>mCherry-Krit1</sup>* embryos prompted us to use microangiography to investigate whether blood vessels were also tight against leakage of a tracer dye anywhere within the vasculature. In particular, we wondered whether the loss of Krit1 would result in defects within lowly-perfused blood vessels[3,4] since those were not protected by high levels of shear stress as seen in the dorsal aorta. We injected 70kDa Texas-Red-conjugated dextran into the lateral common cardinal vein, which spread rapidly throughout all vascular beds in *krit1<sup>endocardium>mCherry-Krit1</sup>* and wild-type embryos and investigated the distribution of the tracer dye immediately within 10-15 minutes upon injection (Fig. 3a-e). Of note, microangiography was not possible in *krit1<sup>ty219c</sup>* embryos because the tracer dye remained at the site of injection close to and within the heart (Fig. 13). In tune with the finding by mass spectrometry and immunohistochemical sections of blood vessels that there were no obvious endothelial cell barrier dysfunctions, inspection through microangiography revealed that at 54-60 hpf nearly the entire vasculature was tightly sealed against a release of the dextran in wild-type and *krit1<sup>endocardium>mCherry-Krit1</sup>* embryos (Fig. 3c). The microangiography survey of the entire vasculature revealed an exception only in small anterior cerebral central arteries, which were normally perfused with the tracer dye but were all dilated and showed a leakage of the tracer dye in some *krit1<sup>endocardium>mCherry-Krit1</sup>* embryos immediately upon injection (n=5/33 embryos injected; Fig. 3e, f). Taken together, the data suggest that in contrast to the restoration of the hyperplastic lateral dorsal aorta in *krit1<sup>endocardium>mCherry-Krit1</sup>* embryos, 1) lesions can occur within lowly-perfused blood vessels and 2) vessel dilation is not restored in all small Krit1-deficient cerebral blood vessels.

## Discussion of the examples

Cerebral cavernous malformations are vascular pathologies that occur mainly within a subset of lowly-perfused blood vessels of the brain. Our study provides functional evidence for a modifying effect exerted by blood flow. Our results show that in *krit1*[endocardium>mCherry-Krit1] embryos, the lateral dorsal aorta is protected from vascular anomalies, while the embryonic cerebrovasculature recapitulates pathological phenotypes observed in mouse models that are based on conditional endothelial-specific knockouts of *CCM* genes[17,38,45]. In mouse, the conditional pan-endothelial depletion of CCM proteins leads to lesion formation mainly within vascular beds of the cerebral region of the brain which are related to the cerebral central arteries affected in zebrafish. Clearly, the presence of blood flow is not sufficiently protective in these small cerebral blood vessels. Apparently, other factors may modify the occurrence of vascular anomalies. For instance, the unique molecular identities of different cerebral vascular beds[47], unique cellular environments of the brain[38], autophagy[9], the innate immune system[48], differences in redox signalling within particular vascular beds[9], different developmental states of endothelial cells[49], or chromatin modifications/epigenetic states[50] may all contribute to the etiology of the CCM pathology.

Our current work implies that blood flow is a physiological modifier with a vasoprotective effect on Krit1-deficient endothelial cells. This suggests that the pathological effects of KLF2/4 signaling upon loss of CCM proteins may be masked when high levels of blood flow activate additional vasoprotective biomechanical signaling pathways or funnel KLF2/4 signal transduction towards vasoprotective target genes (Fig. 4). In support of this model, we found that upon restoration of blood flow in *krit1*[endocardium>mCherry-Krit1] embryos, high expression levels of *klf2a* are not sufficient to trigger the pathological hyperplastic phenotype of the lateral dorsal aorta. This finding may provide an explanation why CCM patients never suffer from vascular anomalies within highly-perfused blood vessels, since these are protected by high levels of blood flow. Uncovering those fluid shear stress-dependent vasoprotective molecular pathways that shield large blood vessels from pathophysiological KLF2/4 signaling may be relevant not only for a better understanding of cerebral cavernous malformations but also of other vascular pathologies. It will be intriguing to revisit the possibility that KLF2/4 may be culprits in other vascular pathologies as well, when protective biomechanical signaling pathways become derailed.

## REFERENCES

1. Baeyens N, Schwartz MA. Biomechanics of vascular mechanosensation and remodeling. Mol Biol Cell. 2016;27:7-11.

2. Baeyens N, Bandyopadhyay C, Coon BG, Yun S, Schwartz MA. Endothelial fluid shear stress sensing in vascular health and disease. J Clin Invest. 2016;126:821-828.

3. Anton H et al. Pulse propagation by a capacitive mechanism drives embryonic blood flow. Development. 2013;140:4426-4434.

4. Boselli F, Freund JB, Vermot J, Blood flow mechanics in cardiovascular development. Cell. Mol. Life Sci. 2015;72;2545-2559.

5. Macek Jilkova Z, Lisowska J, Manet S, Verdier C, Deplano V, Geindreau C, Faurobert E, Albighs-Rizo C, Duperray A. CCM proteins control endothelial β1 integrin dependent response to shear stress. Biol Open. 2014;3:1228-1235.

6. Mleynek TM, Chan AC, Redd M, Gibson CC, Davis CT, Shi DS, Chen T, Carter KL, Ling J, Blanco R, Gerhardt H, Whitehead K, Li DY. Lack of CCM1 induces hypersprouting and impairs response to flow. Hum Mol Genet. 2014;23:6223-6234.

7. Renz M, Otten C, Faurobert E, Rudolph F, Zhu Y, Boulday G, Duchene J, Mickoleit M, Dietrich A-C, Ramspacher C, Steed E, Manet-Dup6 S, Benz A, Hassel D, Vermot J, Huisken J, Tournier-Lasserve E, Felbor U, Sure U, Albiges-Rizo C, Abdelilah-Seyfried S. Regulation of β1 integrin-Klf2-mediated angiogenesis by CCM proteins. Dev Cell. 2015;32:181-190.

8. Liu H, Rigamonti D, BadrA, Zhang J, Ccm1 Regulates Microvascular Morphogenesis during Angiogenesis. Journal of Vascular Research. 2011 ;48:130-140.

9. Retta SF, Glading AJ, Oxidative stress and inflammation in cerebral cavernous malformation disease pathogenesis: Two sides of the same coin. The International Journal of Biochemistry & Cell Biology. 2016;81:254-270.

10. Spiegler S, Rath M, Paperlein C, Felbor U. Cerebral Cavernous Malformations: An Update on Prevalence, Molecular Genetic Analyses, and Genetic Counselling. Mol Syndromol. 2018;9:60-69.

11. Knudson AG. Mutation and cancer: statistical study of retinoblastoma. Proc Natl Acad Sci USA. 1971;68:820-823.

12. Dubovsky J, Zabramski JM, Kurth J, Spetzler RF, Rich SS, Orr HT, Weber JL. A gene responsible for cavernous malformations of the brain maps to chromosome 7q. Hum Mol Genet. 1995;4:453-458.

13. Craig HD, Günel M, Cepeda O, Johnson EW, Ptacek L, Steinberg GK, Ogilvy CS, Berg MJ, Crawford SC, Scott RM, Steichen-Gersdorf E, Sabroe R, Kennedy CT, Mettler G, Beis MJ, Fryer A, Awad IA, Lifton RP. Multilocus linkage identifies two new loci for a mendelian form of stroke, cerebral cavernous malformation, at 7p15-13 and 3q25.2-27. Hum Mol Genet. 1998;7:1851-1858.

14. Akers AL, Johnson E, Steinberg GK, Zabramski JM, Marchuk DA. Biallelic somatic and germline mutations in cerebral cavernous malformations (CCMs): evidence for a two-hit mechanism of CCM pathogenesis. Hum Mol Genet. 2009;18:919-930.

15. Chohan MO, Marchiò S, Morrison LA, Sidman RL, Cavenee WK, Dejana E, Yonas H, Pasqualini R, Arap W. Emerging Pharmacologic Targets in Cerebral Cavernous Malformation and Potential Strategies to Alter the Natural History of a Difficult Disease: A Review. JAMA Neurol. 2018.

16. Zhou Z, Rawnsley DR, Goddard LM, Pan W, Cao X-J, Jakus Z, Zheng H, Yang J, Arthur JSC, Whitehead KJ, Li D, Zhou B, Garcia BA, Zheng X, Kahn ML. The cerebral cavernous malformation pathway controls cardiac development via regulation of endocardial MEKK3 signaling and KLF expression. Dev Cell. 2015;32:168-180.

17. Zhou Z, Tang AT, Wong W-Y, Bamezai S, Goddard LM, Shenkar R, Zhou S, Yang J, Wright AC, Foley M, Arthur JSC, Whitehead KJ, Awad IA, Li DY, Zheng X, Kahn ML. Cerebral cavernous malformations arise from endothelial gain of MEKK3-KLF2/4 signalling. Nature. 2016;532:122-126.

18. Cuttano R, Rudini N, Bravi L, Corada M, Giampietro C, Papa E, Morini MF, Maddaluno L, Baeyens N, Adams RH, Jain MK, Owens GK, Schwartz M, Lampugnani MG, Dejana E. KLF4 is a key determinant in the development and progression of cerebral cavernous malformations. EMBO Mol Med. 2016;8:6-24.

19. Westerfield M. The zebrafish book. A guide for the laboratory use of zebrafish (Danio rerio). 2000. 4th ed., Univ. of Oregon Press, Eugene.

20. Chen JN et al, Mutations affecting the cardiovascular system and other internal organs in zebrafish. Development 1996; 123:293-302.

21. Mably JD, Chuang LP, Serluca FC, Mohideen M-APK, Chen J-N, Fishman MC. santa and valentine pattern concentric growth of cardiac myocardium in the zebrafish. Development. 2006;133:3139-3146.

22. Jin S-W, Beis D, Mitchell T, Chen J-N, Stainier DYR. Cellular and molecular analyses of vascular tube and lumen formation in zebrafish. Development. 2005;132:5199-5209.

23. Roman BL, Phan VN, Lawson ND, Kulik M, Childs S, Lekven AC, Garrity DM, Moon RT, Fishman MC, Lechleider RJ, Weinstein BM. Disruption of acvrl1 increases endothelial cell number in zebrafish cranial vessels. Development. 2002. 129;3009-3019.

24. Chi NC, Shaw RM, De Val S, Kang G, Jan LY, Black BL, Stainier DYR. Foxn4 directly regulates tbx2b expression and atrioventricular canal formation. Genes Dev. 2008;22:734-739.

25. Sugden WW, Meissner R, Aegerter-Wilmsen T, Tsaryk R, Leonard EV, Bussmann J, Hamm MJ, Herzog W, Jin Y, Jakobsson L, Denz C, Siekmann AF. Endoglin controls blood vessel diameter through endothelial cell shape changes in response to haemodynamic cues. Nat Cell Biol. 2017;19:653-665.

26. Herwig L et al. Distinct Cellular Mechanisms of Blood Vessel Fusion in the Zebrafish Embryo. Current Biology. 2011;21:1942-1948.

27. Pestel J, Ramadass R, Gauvrit S, Helker C, Herzog W, Stainier DYR. Real-time 3D visualization of cellular rearrangements during cardiac valve formation. Development. 2016;143:2217-2227.

28. Donat S, Lourenço M, Paolini A, Otten C, Renz M, Abdelilah-Seyfried S. Heg1 and Ccm1/2 proteins control endocardial mechanosensitivity during zebrafish valvulogenesis. Elife. 2018;7.

29. Kwan KM, Fujimoto E, Grabher C, Mangum BD, Hardy ME, Campbell DS, Parant JM, Yost HJ, Kanki JP, Chien C-B. The Tol2kit: a multisite gateway-based construction kit for Tol2 transposon transgenesis constructs. Dev Dyn. 2007;236:3088-3099.

30. Villefranc JA, Amigo J, Lawson ND. Gateway compatible vectors for analysis of gene function in the zebrafish. Dev Dyn. 2007;236:3077-3087.

31. Sehnert AJ, Huq A, Weinstein BM, Walker C, Fishman M, Stainier DYR. Cardiac troponin T is essential in sarcomere assembly and cardiac contractility. Nat Genet. 2002;31:106-110.

32. Weinstein BM, Stemple DL, DrieverW, Fishman MC. Gridlock, a localized heritable vascular patterning defect in the zebrafish. Nat Med. 1995;1:1143-1147.

33. Li P, White RM, Zon Ll. Transplantation in zebrafish. Methods Cell Biol. 2011;105:403-417.

34. Lange S, Sylvester M, Schumann M, Freund C, Krause E. Identification of phosphorylation-dependent interaction partners of the adapter protein ADAP using quantitative mass spectrometry: SILAC vs (18)O-labeling. J Proteome Res. 2010;9:4113-4122.

35. Kristiansen TZ, Harsha HC, Grønborg M, Maitra A, Pandey A. Differential membrane proteomics using 180-labeling to identify biomarkers for cholangiocarcinoma. J Proteome Res. 2008;7:4670-4677.

36. Blum Y, Belting H-G, Ellertsdottir E, Herwig L, Lüders F, Affolter M. Complex cell rearrangements during intersegmental vessel sprouting and vessel fusion in the zebrafish embryo. Dev Biol. 2008;316:312-322.

37. Kleaveland B, Zheng X, Liu JJ, Blum Y, Tung JJ, Zou Z, Sweeney SM, Chen M, Guo L, Lu M, Zhou D, Kitajewski J, Affolter M, Ginsberg MH, Kahn ML. Regulation of cardiovascular development and integrity by the heart of glass-cerebral cavernous malformation protein pathway. Nat Med. 2009;15:169-176.

38. Boulday G, Blécon A, Petit N, Chareyre F, Garcia LA, Niwa-Kawakita M, Giovannini M, Tournier-Lasserve E. Tissue-specific conditional CCM2 knockout mice establish the essential role of endothelial CCM2 in angiogenesis: implications for human cerebral cavernous malformations. Dis Model Mech. 2009;2:168-177.

39. Dietrich A-C, Lombardo VA, Veerkamp J, Priller F, Abdelilah-Seyfried S. Blood flow and Bmp signaling control endocardial chamber morphogenesis. Dev Cell. 2014;30:367-377.

40. Fisher OS, Boggon TJ. Signaling pathways and the cerebral cavernous malformations proteins: lessons from structural biology. Cell MolLife Sci. 2014;71:1881-1892.

41. Glading AJ, Ginsberg MH. Rap1 and its effector KRIT1/CCM1 regulate beta-catenin signaling. Dis Model Mech. 2010;3:73-83.

42. Günel M, Laurans MSH, Shin D, DiLuna ML, Voorhees J, Choate K, Nelson-Williams C, Lifton RP. KRIT1, a gene mutated in cerebral cavernous malformation, encodes a microtubuleassociated protein. Proc Natl Acad Sci USA. 2002;99:10677-10682.

43. Bravi L, Malinverno M, Pisati F, Rudini N, Cuttano R, Pallini R, Martini M, Larocca LM, Locatelli M, Levi V, Bertani GA, Dejana E, Lampugnani MG. Endothelial Cells Lining Sporadic Cerebral Cavernous Malformation Cavernomas Undergo Endothelial-to-Mesenchymal Transition. Stroke. 2016;47:886-890.

44. Maddaluno L, Rudini N, Cuttano R, Bravi L, Giampietro C, Corada M, Ferrarini L, Orsenigo F, Papa E, Boulday G, Tournier-Lasserve E, Chapon F, Richichi C, Retta SF, Lampugnani MG, Dejana E. EndMT contributes to the onset and progression of cerebral cavernous malformations. Nature. 2013;498:492-496.

45. Dejana E, Lampugnani MG. Endothelial cell transitions. Science. 2018;362:746-747.

46. Vermot J, Forouhar AS, Liebling M, Wu D, Plummer D, Gharib M, Fraser SE. Reversing blood flows act through klf2a to ensure normal valvulogenesis in the developing heart. PLoS Biol. 2009;7:e1000246.

47. Vanlandewijck M, He L, Mae MA, Andrae J, Ando K, Del Gaudio F, et al. A molecular atlas of cell types and zonation in the brain vasculature. Nature. 2018;554:475.

48. Tang AT, et al. Endothelial TLR4 and the microbiome drive cerebral cavernous malformations. Nature. 2017;545:305-310.

49. Dejana E, Hirschi KK, Simons M. The molecular basis of endothelial cell plasticity. Nature Communications. 2017;8:14361

50. Morini MF, Giampietro C, Corada M, Pisati F, Lavarone E, Cunha Si, et al. VE-Cadherin-Mediated Epigenetic Regulation of Endothelial Gene Expression. Circulation Research. 2018;122(2):231-45

51. Often, C., Knox, J., Boulday, G., Eymery, M., Haniszewski, M., Neuenschwander, M., Radetzki, S., Vogt, I., Hahn, K., De Luca, C., Cardoso, C., Hamad, S., Igual Gil, C., Roy, P., Albiges-Rizo, C., Faurobert, E., von Kries, J.P., Campillos, M., Tournier-Lasserve, E., Derry, W.B., Abdelilah-Seyfried, S., 2018. Systematic pharmacological screens uncover novel pathways involved in cerebral cavernous malformations. EMBO Mol Med 10.

52. Reinhard, M., Schuchardt, F., Meckel, S., Heinz, J., Felbor, U., Sure, U., Geisen, U., 2016. Propranolol stops progressive multiple cerebral cavernoma in an adult patient. J. Neurol. Sci. 367, 15-17.

53. Zabramski, J.M., Kalani, M.Y.S., Filippidis, A.S., Spetzler, R.F., 2016. Propranolol Treatment of Cavernous Malformations with Symptomatic Hemorrhage. World Neurosurgery 88, 631-639.

## Claims

1. A transgenic zebrafish lacking at least one endogenous functional protein required for a functional CCM complex, comprising an exogenous nucleic acid encoding a functional version of said at least one protein under the transcriptional control of an endocardium-specific genetic system,

   wherein said transgenic zebrafish comprises a cardiovascular system with a heart that produces blood flow, and wherein said zebrafish develops vascular lesions in cerebral blood vessels, in particular in lowly perfused cerebral blood vessels, and
   wherein the at least one protein required for a functional CCM complex is selected from the group comprising CCM1 (KRIT1), CCM2, CCM3, and Heg1.

2. Transgenic zebrafish according to claim 1, wherein the at least one protein is CCM1 and the zebrafish lacking at least one endogenous functional protein required for a functional CCM complex is a zebrafish homozygously carrying a loss-of-function allele of krit1, such as a krit1-ty219c allele.

3. Transgenic zebrafish according to any one of the preceding claims, wherein the endocardium-specific genetic system comprises a Gal4-UAS system, wherein the Gal4-UAS system comprises a Gal4 gene, preferably Gal4ff, under the control of an endocardium-specific transcriptional regulator, such as an endocardium-specific promoter, and a nucleic acid sequence encoding said functional protein required for a functional CCM complex under the control of a UAS region.

4. Transgenic zebrafish according to claim 3, wherein the endocardium-specific expression of the Gal4 gene is driven by an nfatc1 promoter.

5. Transgenic zebrafish according to any one of the preceding claims, wherein the functional version of said at least one protein is a fusion protein of the at least one functional protein and a reporter protein, such as a fluorescent reporter protein.

6. Transgenic zebrafish according to claim 5, wherein the vascular lesions can be visualized by

   - a traceable reagent injected into the cardiovascular system of said transgenic zebrafish, such as a traceable dye or labeled nanobeads, or
   - by using a transgenic zebrafish additionally expressing fluorescently-labelled blood proteins or blood cells.

7. Transgenic zebrafish according to any one of the preceding claims, wherein the zebrafish is at a stage of development selected from the group consisting of an embryo stage, a larva stage, and an adult stage.

8. Method for measuring a pharmacological effect of a drug, comprising

   - contacting a transgenic zebrafish according to any one of claims 1 to 7 with a drug, and
   - measuring a pharmacological effect of the drug.

9. Method for measuring a pharmacological effect of a drug according to claim 8, wherein the pharmacological effect of the drug is an effect on the formation of vascular lesions, the development of cerebral cavernous malformation and/or the development of stroke.

10. Method for measuring a pharmacological effect of a drug according to claim 9, wherein the pharmacological effect of the drug is measured by comparing the formation of vascular lesions, the development of cerebral cavernous malformation and/or the development of stroke in the transgenic zebrafish contacted with the drug to the formation of vascular lesions, the development of cerebral cavernous malformation and/or the development of stroke in a transgenic zebrafish according to any one of claims 1 to 7 not contacted with said drug.

11. Use of a transgenic zebrafish according to any one of claims 1 to 7 as a model for the treatment and/or prevention of vascular lesions, cerebral cavernous malformation and/or stroke.

12. Use of a transgenic zebrafish according to any one of claims 1 to 7 in a method of screening a compound for an effect on the formation of vascular lesions, the development of cerebral cavernous malformation and/or the development of stroke, the method comprising

   - contacting said transgenic zebrafish to a compound and
   - measuring the formation of vascular lesions, the development of cerebral cavernous malformation and/or the development of stroke.

**Patentansprüche**

1. Transgener Zebrafisch, dem mindestens ein endogenes funktionelles Protein fehlt, das für einen funktionellen CCM-Komplex erforderlich ist, umfassend eine exogene Nukleinsäure, die eine funktionelle Version des mindestens einen Proteins unter der Transkriptionskontrolle eines endokardspezifischen genetischen Systems kodiert,

   wobei der transgene Zebrafisch ein Herz-Kreislauf-System mit einem Herzen umfasst, das den Blutfluss erzeugt, und wobei der Zebrafisch Gefäßläsionen in zerebralen Blutgefäßen entwickelt, insbesondere in schwach durchbluteten zerebralen Blutgefäßen, und
   wobei das mindestens eine Protein, das für einen funktionellen CCM-Komplex erforderlich ist, aus der Gruppe ausgewählt ist, die CCM1 (KRIT1), CCM2, CCM3 und Heg1 umfasst.

2. Transgener Zebrafisch nach Anspruch 1, wobei das mindestens eine Protein CCM1 ist und der Zebrafisch, dem mindestens ein endogenes funktionelles Protein fehlt, das für einen funktionellen CCM-Komplex erforderlich ist, ein Zebrafisch ist, der homozygot ein Funktionsverlust-Allel von Krit1 trägt, wie etwa ein Krit1-ty219c-Allel.

3. Transgener Zebrafisch nach einem der vorhergehenden Ansprüche, wobei das endokardspezifische genetische System ein Ga14-UAS-System umfasst, wobei das Gal4-UAS-System ein Gal4-Gen, vorzugsweise Gal4ff, unter der Kontrolle eines endokardspezifischen Transkriptionsregulators, wie etwa eines endokardspezifischen Promotors, und eine Nukleinsäuresequenz, die das funktionelle Protein, das für einen funktionellen CCM-Komplex erforderlich ist, unter der Kontrolle einer UAS-Region kodiert, umfasst.

4. Transgener Zebrafisch nach Anspruch 3, wobei die endokardspezifische Expression des Ga14-Gens durch einen nfatc1-Promotor gesteuert wird.

5. Transgener Zebrafisch nach einem der vorhergehenden Ansprüche, wobei die funktionelle Version des mindestens einen Proteins ein Fusionsprotein des mindestens einen funktionellen Proteins und eines Reporterproteins, wie etwa eines fluoreszierenden Reporterproteins, ist.

6. Transgener Zebrafisch nach Anspruch 5, wobei die Gefäßläsionen durch Folgendes sichtbar gemacht werden können:

   - ein rückverfolgbares Reagenz, das in das Herz-Kreislauf-System des transgenen Zebrafisches injiziert wird, wie etwa einen rückverfolgbaren Farbstoff oder markierte Nanokügelchen, oder
   - durch Verwendung eines transgenen Zebrafisches, der zusätzlich fluoreszenzmarkierte Blutproteine oder Blutzellen exprimiert.

7. Transgener Zebrafisch nach einem der vorhergehenden Ansprüche, wobei sich der Zebrafisch in einem Entwicklungsstadium befindet, das aus der Gruppe ausgewählt ist, die aus einem Embryostadium, einem Larvenstadium und einem Erwachsenenstadium besteht.

8. Verfahren zum Messen einer pharmakologischen Wirkung eines Arzneimittels, umfassend

   - Inkontaktbringen eines transgenen Zebrafisches nach einem der Ansprüche 1 bis 7 mit einem Arzneimittel und
   - Messen einer pharmakologischen Wirkung des Arzneimittels.

9. Verfahren zum Messen einer pharmakologischen Wirkung eines Arzneimittels nach Anspruch 8, wobei die pharmakologische Wirkung des Arzneimittels eine Wirkung auf die Bildung von Gefäßläsionen, die Entwicklung einer zerebralen kavernösen Fehlbildung und/oder die Entwicklung eines Schlaganfalls ist.

10. Verfahren zum Messen einer pharmakologischen Wirkung eines Arzneimittels nach Anspruch 9, wobei die pharmakologische Wirkung des Arzneimittels durch Vergleichen der Bildung von Gefäßläsionen, der Entwicklung einer zerebralen kavernösen Fehlbildung und/oder der Entwicklung eines Schlaganfalls bei dem transgenen Zebrafisch, der mit dem Arzneimittel in Kontakt gebracht wurde, mit der Bildung von Gefäßläsionen, der Entwicklung einer zerebralen kavernösen Fehlbildung und/oder der Entwicklung eines Schlaganfalls bei einem transgenen Zebrafisch nach einem der Ansprüche 1 bis 7, der nicht mit dem Arzneimittel in Kontakt gebracht wurde, gemessen wird.

11. Verwendung eines transgenen Zebrafisches nach einem der Ansprüche 1 bis 7 als Modell für die Behandlung und/oder Prävention von Gefäßläsionen, einer zerebralen kavernösen Fehlbildung und/oder eines Schlaganfalls.

12. Verwendung eines transgenen Zebrafisches nach einem der Ansprüche 1 bis 7 in einem Verfahren zum Screenen einer Verbindung auf eine Wirkung auf die Bildung von Gefäßläsionen, die Entwicklung einer zerebralen kavernösen Fehlbildung und/oder die Entwicklung eines Schlaganfalls, wobei das Verfahren Folgendes umfasst:

   - Inkontaktbringen des transgenen Zebrafisches mit einer Verbindung und
   - Messung der Bildung von Gefäßläsionen, der Entwicklung einer zerebralen kavernösen Fehlbildung und/oder der Entwicklung eines Schlaganfalls.

**Revendications**

1. Poisson zèbre transgénique dépourvu d'au moins une protéine fonctionnelle endogène requise pour un complexe CCM fonctionnel, comprenant un acide nucléique exogène codant pour une version fonctionnelle de ladite au moins une protéine sous le contrôle transcriptionnel d'un système génétique spécifique de l'endocarde,

   dans lequel ledit poisson zèbre transgénique comprend un système cardiovasculaire avec un cœur qui produit un flux sanguin, et dans lequel ledit poisson zèbre développe des lésions vasculaires dans les vaisseaux sanguins cérébraux, en particulier dans les vaisseaux sanguins cérébraux faiblement perfusés, et

dans lequel l'au moins une protéine requise pour un complexe CCM fonctionnel est choisie dans le groupe comprenant CCM1 (KRIT1), CCM2, CCM3 et Heg1.

2. Poisson zèbre transgénique selon la revendication 1, dans lequel l'au moins une protéine est CCM1 et le poisson zèbre dépourvu d'au moins une protéine fonctionnelle endogène requise pour un complexe CCM fonctionnel est un poisson zèbre portant de manière homozygote un allèle de perte de fonction de krit1, tel qu'un allèle krit1-ty219c.

3. Poisson zèbre transgénique selon l'une quelconque des revendications précédentes, dans lequel le système génétique spécifique de l'endocarde comprend un système Ga14-UAS, dans lequel le système Ga14-UAS comprend un gène Gal4, de préférence Gal4ff, sous le contrôle d'un régulateur transcriptionnel spécifique de l'endocarde, tel qu'un promoteur spécifique de l'endocarde, et une séquence d'acide nucléique codant pour ladite protéine fonctionnelle requise pour un complexe CCM fonctionnel sous le contrôle d'une région UAS.

4. Poisson zèbre transgénique selon la revendication 3, dans lequel l'expression spécifique de l'endocarde du gène Gal4 est pilotée par un promoteur nfatc1.

5. Poisson zèbre transgénique selon l'une quelconque des revendications précédentes, dans lequel la version fonctionnelle de ladite au moins une protéine est une protéine de fusion de l'au moins une protéine fonctionnelle et d'une protéine rapporteur, telle qu'une protéine rapporteur fluorescente.

6. Poisson zèbre transgénique selon la revendication 5, dans lequel les lésions vasculaires peuvent être visualisées par

   - un réactif traçable injecté dans le système cardiovasculaire dudit poisson zèbre transgénique, tel qu'un colorant traçable ou des nanobilles marquées, ou
   - en utilisant un poisson zèbre transgénique exprimant en outre des protéines sanguines ou des cellules sanguines marquées par fluorescence.

7. Poisson zèbre transgénique selon l'une quelconque des revendications précédentes, dans lequel le poisson zèbre est à un stade de développement choisi dans le groupe constitué d'un stade embryonnaire, d'un stade larvaire et d'un stade adulte.

8. Procédé de mesure d'un effet pharmacologique d'un médicament, comprenant

   - la mise en contact d'un poisson zèbre transgénique selon l'une quelconque des revendications 1 à 7 avec un médicament, et
   - la mesure d'un effet pharmacologique du médicament.

9. Procédé de mesure d'un effet pharmacologique d'un médicament selon la revendication 8, dans lequel l'effet pharmacologique du médicament est un effet sur la formation de lésions vasculaires, le développement de malformation caverneuse cérébrale et/ou le développement d'accident vasculaire cérébral.

10. Procédé de mesure d'un effet pharmacologique d'un médicament selon la revendication 9, dans lequel l'effet pharmacologique du médicament est mesuré en comparant la formation de lésions vasculaires, le développement d'une malformation caverneuse cérébrale et/ou le développement d'un accident vasculaire cérébral chez le poisson zèbre transgénique mis en contact avec le médicament à la formation de lésions vasculaires, au développement d'une malformation caverneuse cérébrale et/ou au développement d'un accident vasculaire cérébral chez un poisson zèbre transgénique selon l'une quelconque des revendications 1 à 7 non mis en contact avec ledit médicament.

11. Utilisation d'un poisson zèbre transgénique selon l'une quelconque des revendications 1 à 7 comme modèle pour le traitement et/ou la prévention de lésions vasculaires, de malformations caverneuses cérébrales et/ou d'accidents vasculaires cérébraux.

12. Utilisation d'un poisson zèbre transgénique selon l'une quelconque des revendications 1 à 7 dans un procédé de criblage d'un composé pour un effet sur la formation de lésions vasculaires, le développement d'une malformation caverneuse cérébrale et/ou le développement d'un accident vasculaire cérébral, le procédé comprenant

   - la mise en contact dudit poisson zèbre transgénique avec un composé et
   - la mesure de la formation de lésions vasculaires, le développement d'une malformation caverneuse cérébrale et/ou le développement d'un accident vasculaire cérébral.

**Figure 1**

**Figure 1 (Continued)**

**Figure 1 (Continued)**

**Figure 2**

**Figure 3**

**Figure 3 (continued)**

**Figure 4**

**Figure 5**

**Figure 5 (continued)**

**Figure 6**

Figure 7

**Figure 7 (continued)**

Figure 8

**Figure 8 (continued)**

**Figure 9**

**Figure 9 (continued)**

Figure 10

**Figure 11**

**Figure 12**

*Tg(kdrl:EGFP)$^{s843}$; Tg(nfatc1:GAL4FF)$^{mu286}$; Tg(UAS:mCherry-Krit1)$^{md40}$*

**Figure 13**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7034141 B
- US 7037645 B
- US 6087476 B
- US 6730822 B
- US 6380458 B
- US 5932780 A
- US 6949242 B
- US 6897045 B
- US 6784289 B
- US 6395549 B
- US 6229070 B
- US 6100448 A
- US 5610053 A
- US 6239328 B
- US 5773695 A
- US 5773689 A

**Non-patent literature cited in the description**

- **KLEAVELAND et al.** *Nat Med,* February 2009, vol. 15 (2), 169-76
- **KIMMEL.** *Trends Genet,* 1989, vol. 5, 283-8
- **DRIEVER et al.** *Development,* 1996, vol. 123, 37-46
- **HAFFTER et al.** *Development,* 1996, vol. 123, 1-36
- **STREISINGER.** *Natl. Cancer Inst. Monogr,* 1984, vol. 65, 53-58
- **CULP et al.** *Proc Natl Acad Sci USA,* 1991, vol. 88, 7953-7957
- **INOUE et al.** *Cell. Differ. Develop.,* 1990, vol. 29, 123-128
- **MULLER et al.** *FEBS Lett,* 1993, vol. 324, 27-32
- **MURAKAMI et al.** *J. Biotechnol,* 1994, vol. 34, 35-42
- **MULLER et al.** *Mol. Mar. Biol. Biotechnol,* 1992, vol. 1, 276-281
- **SYMONDS et al.** *Aquaculture,* 1994, vol. 119, 313-327
- **ZELENIN et al.** *FEBS Lett,* 1991, vol. 287, 118-120
- **LU et al.** *Mol Mar Biol Biotechnol,* 1997, vol. 6, 289-95
- **SZELEI et al.** *Transgenic Res,* 1994, vol. 3, 116-119
- **KAWAKAMI, K.** *Genome Biol,* 2007, vol. 8 (1), S7
- **KLEAVELAND et al.** *Nature Medicine,* February 2009, vol. 15 (2), 169-176
- **SPIEGELER et al.** *Mol Syndromol,* 2018, vol. 9, 60-69
- **JENNIFER LIPPINCOTT-SCHWARTZ ; GEORGE H. PATTERSON.** Development and Use of Fluorescent Protein Markers in Living Cells. *300(5616) Science,* 2003, 87-91
- **JIN ZHANG et al.** *Nat. Rev. Mol. Cell. Biol,* 2002, vol. 3 (12), 906-918
- **J. SAMBROOK ; E. F. FRITSCH ; T. MANIATIS.** Molecular Cloning: A Laboratory Manual. Cold Spring Harbor Laboratory Press, 1989, 1-3
- **AUSUBEL, F. M et al.** Current Protocols in Molecular Biology. John Wiley & Sons, 1995, vol. 9,13,16
- **B. ROE ; J. CRABTREE ; A. KAHN.** DNA Isolation and Sequencing: Essential Techniques. John Wiley & Sons
- **J. M. POLAK ; JAMES O'D. MCGEE.** In Situ Hybridization: Principles and Practice. Oxford University Press, 1990
- Oligonucleotide Synthesis: A Practical Approach. Irl Press, 1984
- **D. M. J. LILLEY ; J. E. DAHLBERG.** Methods of Enzymology: DNA Structure Part A: Synthesis and Physical Analysis of DNA Methods in Enzymology. Academic Press, 1992
- **EDWARD HARLOW ; DAVID LANE ; ED HARLOW.** Using Antibodies: A Laboratory Manual: Portable Protocol. Cold Spring Harbor Laboratory Press, 1999
- Antibodies: A Laboratory Manual. Cold Spring Harbor Laboratory Press, 1988
- **RAMAKRISHNA SEETHALA ; PRABHAVATHI ; B. FERNANDES.** Handbook of Drug Screening. Marcel Dekker, 2001
- **JANE ROSKAMS ; LINDA RODGERS.** Lab Ref: A Handbook of Recipes, Reagents, and Other Reference Tools for Use at the Bench. Cold Spring Harbor Laboratory, 2002

- **BAEYENS N ; SCHWARTZ MA.** Biomechanics of vascular mechanosensation and remodeling. *Mol Biol Cell,* 2016, vol. 27, 7-11
- **BAEYENS N ; BANDYOPADHYAY C ; COON BG ; YUN S ; SCHWARTZ MA.** Endothelial fluid shear stress sensing in vascular health and disease. *J Clin Invest,* 2016, vol. 126, 821-828
- **ANTON H et al.** Pulse propagation by a capacitive mechanism drives embryonic blood flow. *Development,* 2013, vol. 140, 4426-4434
- **BOSELLI F ; FREUND JB ; VERMOT J.** Blood flow mechanics in cardiovascular development. *Cell. Mol. Life Sci,* 2015, vol. 72, 2545-2559
- **MACEK JILKOVA Z ; LISOWSKA J ; MANET S ; VERDIER C ; DEPLANO V ; GEINDREAU C ; FAUROBERT E ; ALBIGHS-RIZO C ; DUPERRAY A.** CCM proteins control endothelial β1 integrin dependent response to shear stress. *Biol Open,* 2014, vol. 3, 1228-1235
- **MLEYNEK TM ; CHAN AC ; REDD M ; GIBSON CC ; DAVIS CT ; SHI DS ; CHEN T ; CARTER KL ; LING J ; BLANCO R.** Lack of CCM1 induces hypersprouting and impairs response to flow. *Hum Mol Genet,* 2014, vol. 23, 6223-6234
- **RENZ M ; OTTEN C ; FAUROBERT E ; RUDOLPH F ; ZHU Y ; BOULDAY G ; DUCHENE J ; MICKOLEIT M ; DIETRICH A-C ; RAMSPACHER C.** Regulation of β1 integrin-Klf2-mediated angiogenesis by CCM proteins. *Dev Cell,* 2015, vol. 32, 181-190
- **LIU H ; RIGAMONTI D ; BADRA, ZHANG J.** Ccm1 Regulates Microvascular Morphogenesis during Angiogenesis. *Journal of Vascular Research,* 2011, vol. 48, 130-140
- **RETTA SF ; GLADING AJ.** Oxidative stress and inflammation in cerebral cavernous malformation disease pathogenesis: Two sides of the same coin. *The International Journal of Biochemistry & Cell Biology,* 2016, vol. 81, 254-270
- **SPIEGLER S ; RATH M ; PAPERLEIN C ; FELBOR U.** Cerebral Cavernous Malformations: An Update on Prevalence, Molecular Genetic Analyses, and Genetic Counselling. *Mol Syndromol,* 2018, vol. 9, 60-69
- **KNUDSON AG.** Mutation and cancer: statistical study of retinoblastoma. *Proc Natl Acad Sci USA.,* 1971, vol. 68, 820-823
- **DUBOVSKY J ; ZABRAMSKI JM ; KURTH J ; SPETZLER RF ; RICH SS ; ORR HT ; WEBER JL.** A gene responsible for cavernous malformations of the brain maps to chromosome 7q. *Hum Mol Genet,* 1995, vol. 4, 453-458
- **CRAIG HD ; GÜNEL M ; CEPEDA O ; JOHNSON EW ; PTACEK L ; STEINBERG GK ; OGILVY CS ; BERG MJ ; CRAWFORD SC ; SCOTT RM.** *Multilocus linkage identifies two new loci for a mendelian form of stroke, cerebral cavernous malformation,* vol. 7, 15-13
- *Hum Mol Genet,* 1998, vol. 7, 1851-1858
- **AKERS AL ; JOHNSON E ; STEINBERG GK ; ZABRAMSKI JM ; MARCHUK DA.** Biallelic somatic and germline mutations in cerebral cavernous malformations (CCMs): evidence for a two-hit mechanism of CCM pathogenesis. *Hum Mol Genet,* 2009, vol. 18, 919-930
- **CHOHAN MO ; MARCHIÒ S ; MORRISON LA ; SIDMAN RL ; CAVENEE WK ; DEJANA E ; YONAS H ; PASQUALINI R ; ARAP W.** Emerging Pharmacologic Targets in Cerebral Cavernous Malformation and Potential Strategies to Alter the Natural History of a Difficult Disease: A Review. *JAMA Neurol,* 2018
- **ZHOU Z ; RAWNSLEY DR ; GODDARD LM ; PAN W ; CAO X-J ; JAKUS Z ; ZHENG H ; YANG J ; ARTHUR JSC ; WHITEHEAD KJ.** The cerebral cavernous malformation pathway controls cardiac development via regulation of endocardial MEKK3 signaling and KLF expression. *Dev Cell,* 2015, vol. 32, 168-180
- **ZHOU Z ; TANG AT ; WONG W-Y ; BAMEZAI S ; GODDARD LM ; SHENKAR R ; ZHOU S ; YANG J ; WRIGHT AC ; FOLEY M.** Cerebral cavernous malformations arise from endothelial gain of MEKK3-KLF2/4 signalling. *Nature,* 2016, vol. 532, 122-126
- **CUTTANO R ; RUDINI N ; BRAVI L ; CORADA M ; GIAMPIETRO C ; PAPA E ; MORINI MF ; MADDALUNO L ; BAEYENS N ; ADAMS RH.** KLF4 is a key determinant in the development and progression of cerebral cavernous malformations. *EMBO Mol Med,* 2016, vol. 8, 6-24
- **WESTERFIELD M.** The zebrafish book. A guide for the laboratory use of zebrafish (Danio rerio). Univ. of Oregon Press, 2000
- **CHEN JN et al.** Mutations affecting the cardiovascular system and other internal organs in zebrafish. *Development,* 1996, vol. 123, 293-302
- **MABLY JD ; CHUANG LP ; SERLUCA FC ; MOHIDEEN M-APK ; CHEN J-N ; FISHMAN MC.** santa and valentine pattern concentric growth of cardiac myocardium in the zebrafish. *Development,* 2006, vol. 133, 3139-3146
- **JIN S-W ; BEIS D ; MITCHELL T ; CHEN J-N ; STAINIER DYR.** Cellular and molecular analyses of vascular tube and lumen formation in zebrafish. *Development,* 2005, vol. 132, 5199-5209
- **ROMAN BL ; PHAN VN ; LAWSON ND ; KULIK M ; CHILDS S ; LEKVEN AC ; GARRITY DM ; MOON RT ; FISHMAN MC ; LECHLEIDER RJ.** Disruption of acvrl1 increases endothelial cell number in zebrafish cranial vessels. *Development,* 2002, vol. 129, 3009-3019
- **CHI NC ; SHAW RM ; DE VAL S ; KANG G ; JAN LY ; BLACK BL ; STAINIER DYR.** Foxn4 directly regulates tbx2b expression and atrioventricular canal formation. *Genes Dev,* 2008, vol. 22, 734-739

- **SUGDEN WW ; MEISSNER R ; AEGERTER-WILMSEN T ; TSARYK R ; LEONARD EV ; BUSSMANN J ; HAMM MJ ; HERZOG W ; JIN Y ; JAKOBSSON L.** Endoglin controls blood vessel diameter through endothelial cell shape changes in response to haemodynamic cues. *Nat Cell Biol,* 2017, vol. 19, 653-665
- **HERWIG L et al.** Distinct Cellular Mechanisms of Blood Vessel Fusion in the Zebrafish Embryo. *Current Biology,* 2011, vol. 21, 1942-1948
- **PESTEL J ; RAMADASS R ; GAUVRIT S ; HELKER C ; HERZOG W ; STAINIER DYR.** Real-time 3D visualization of cellular rearrangements during cardiac valve formation. *Development,* 2016, vol. 143, 2217-2227
- **DONAT S ; LOURENÇO M ; PAOLINI A ; OTTEN C ; RENZ M ; ABDELILAH-SEYFRIED S.** Heg1 and Ccm1/2 proteins control endocardial mechanosensitivity during zebrafish valvulogenesis. *Elife,* 2018, vol. 7
- **KWAN KM ; FUJIMOTO E ; GRABHER C ; MANGUM BD ; HARDY ME ; CAMPBELL DS ; PARANT JM ; YOST HJ ; KANKI JP ; CHIEN C-B.** The Tol2kit: a multisite gateway-based construction kit for Tol2 transposon transgenesis constructs. *Dev Dyn,* 2007, vol. 236, 3088-3099
- **VILLEFRANC JA ; AMIGO J ; LAWSON ND.** Gateway compatible vectors for analysis of gene function in the zebrafish. *Dev Dyn,* 2007, vol. 236, 3077-3087
- **SEHNERT AJ ; HUQ A ; WEINSTEIN BM ; WALKER C ; FISHMAN M ; STAINIER DYR.** Cardiac troponin T is essential in sarcomere assembly and cardiac contractility. *Nat Genet,* 2002, vol. 31, 106-110
- **WEINSTEIN BM ; STEMPLE DL ; DRIEVER W ; FISHMAN MC. GRIDLOCK.** localized heritable vascular patterning defect in the zebrafish. *Nat Med,* 1995, vol. 1, 1143-1147
- **LI P ; WHITE RM ; ZON LL.** Transplantation in zebrafish. *Methods Cell Biol,* 2011, vol. 105, 403-417
- **LANGE S ; SYLVESTER M ; SCHUMANN M ; FREUND C ; KRAUSE E.** Identification of phosphorylation-dependent interaction partners of the adapter protein ADAP using quantitative mass spectrometry: SILAC vs (18)O-labeling. *J Proteome Res,* 2010, vol. 9, 4113-4122
- **KRISTIANSEN TZ ; HARSHA HC ; GRØNBORG M ; MAITRA A ; PANDEY A.** Differential membrane proteomics using 180-labeling to identify biomarkers for cholangiocarcinoma. *J Proteome Res,* 2008, vol. 7, 4670-4677
- **BLUM Y ; BELTING H-G ; ELLERTSDOTTIR E ; HERWIG L ; LÜDERS F ; AFFOLTER M.** Complex cell rearrangements during intersegmental vessel sprouting and vessel fusion in the zebrafish embryo. *Dev Biol,* 2008, vol. 316, 312-322
- **KLEAVELAND B ; ZHENG X ; LIU JJ ; BLUM Y ; TUNG JJ ; ZOU Z ; SWEENEY SM ; CHEN M ; GUO L ; LU M.** Regulation of cardiovascular development and integrity by the heart of glass-cerebral cavernous malformation protein pathway. *Nat Med,* 2009, vol. 15, 169-176
- **BOULDAY G ; BLÉCON A ; PETIT N ; CHAREYRE F ; GARCIA LA ; NIWA-KAWAKITA M ; GIOVANNINI M ; TOURNIER-LASSERVE E.** Tissue-specific conditional CCM2 knockout mice establish the essential role of endothelial CCM2 in angiogenesis: implications for human cerebral cavernous malformations. *Dis Model Mech,* 2009, vol. 2, 168-177
- **DIETRICH A-C ; LOMBARDO VA ; VEERKAMP J ; PRILLER F ; ABDELILAH-SEYFRIED S.** Blood flow and Bmp signaling control endocardial chamber morphogenesis. *Dev Cell,* 2014, vol. 30, 367-377
- **FISHER OS ; BOGGON TJ.** Signaling pathways and the cerebral cavernous malformations proteins: lessons from structural biology. *Cell MolLife Sci,* 2014, vol. 71, 1881-1892
- **GLADING AJ ; GINSBERG MH.** Rap1 and its effector KRIT1/CCM1 regulate beta-catenin signaling. *Dis Model Mech,* 2010, vol. 3, 73-83
- **GÜNEL M ; LAURANS MSH ; SHIN D ; DILUNA ML ; VOORHEES J ; CHOATE K ; NELSON-WILLIAMS C ; LIFTON RP.** KRIT1, a gene mutated in cerebral cavernous malformation, encodes a microtubuleassociated protein. *Proc Natl Acad Sci USA,* 2002, vol. 99, 10677-10682
- **BRAVI L ; MALINVERNO M ; PISATI F ; RUDINI N ; CUTTANO R ; PALLINI R ; MARTINI M ; LAROCCA LM ; LOCATELLI M ; LEVI V.** Endothelial Cells Lining Sporadic Cerebral Cavernous Malformation Cavernomas Undergo Endothelial-to-Mesenchymal Transition. *Stroke,* 2016, vol. 47, 886-890
- **MADDALUNO L ; RUDINI N ; CUTTANO R ; BRAVI L ; GIAMPIETRO C ; CORADA M ; FERRARINI L ; ORSENIGO F ; PAPA E ; BOULDAY G.** EndMT contributes to the onset and progression of cerebral cavernous malformations. *Nature,* 2013, vol. 498, 492-496
- **DEJANA E ; LAMPUGNANI MG.** Endothelial cell transitions. *Science,* 2018, vol. 362, 746-747
- **VERMOT J ; FOROUHAR AS ; LIEBLING M ; WU D ; PLUMMER D ; GHARIB M ; FRASER SE.** Reversing blood flows act through klf2a to ensure normal valvulogenesis in the developing heart. *PLoS Biol,* 2009, vol. 7, e1000246
- **VANLANDEWIJCK M ; HE L ; MAE MA ; ANDRAE J ; ANDO K ; DEL GAUDIO F et al.** A molecular atlas of cell types and zonation in the brain vasculature. *Nature,* 2018, vol. 554, 475
- **TANG AT et al.** Endothelial TLR4 and the microbiome drive cerebral cavernous malformations. *Nature,* 2017, vol. 545, 305-310

- **DEJANA E ; HIRSCHI KK ; SIMONS M.** The molecular basis of endothelial cell plasticity. *Nature Communications,* 2017, vol. 8, 14361
- **MORINI MF ; GIAMPIETRO C ; CORADA M ; PISATI F ; LAVARONE E ; CUNHA SI et al.** VE-Cadherin-Mediated Epigenetic Regulation of Endothelial Gene Expression. *Circulation Research,* 2018, vol. 122 (2), 231-45
- **OFTEN, C. ; KNOX, J. ; BOULDAY, G. ; EYMERY, M. ; HANISZEWSKI, M. ; NEUENSCHWANDER, M. ; RADETZKI, S. ; VOGT, I. ; HAHN, K. ; DE LUCA, C.** Systematic pharmacological screens uncover novel pathways involved in cerebral cavernous malformations. *EMBO Mol Med,* 2018, vol. 10
- **REINHARD, M. ; SCHUCHARDT, F. ; MECKEL, S. ; HEINZ, J. ; FELBOR, U. ; SURE, U. ; GEISEN, U.** 2016. Propranolol stops progressive multiple cerebral cavernoma in an adult patient. *J. Neurol. Sci,* vol. 367, 15-17
- **ZABRAMSKI, J.M. ; KALANI, M.Y.S. ; FILIPPIDIS, A.S. ; SPETZLER, R.F.** Propranolol Treatment of Cavernous Malformations with Symptomatic Hemorrhage. *World Neurosurgery,* 2016, vol. 88, 631-639